# EUROPEAN PATENT APPLICATION

(11) **EP 3 594 358 A1**
(43) Date of publication of application: **15.01.2020**
(21) Application number: 18305958.3
(22) Date of filing: 13.07.2018
(51) Int. Cl.: C12Q 1/37

(54) **IN VITRO SCREENING ASSAY OF TCPASE MODULATORS**

(71) Applicant: Centre National de la Recherche Scientifique - CNRS, 75016 Paris (FR); UNIVERSITE DE MONTPELLIER, 34090 Montpellier (FR)
(72) Inventor: ROGOWSKI, Krzysztof, 34070 MONTPELLIER (FR); VAN DER LAAN, Siem, 34190 CAZILHAC (FR)
(74) Representative: Regimbeau

(57) **Abstract**

The present invention concerns an *in vitro* screening assay for identification of modulators of tubulin carboxypeptidase (TCPase) activity comprising the steps of:
(i) Contacting (a) a substrate of TCPase enzyme comprising an amino acids sequence having at least the last 4 amino acids residues of the C-terminal sequence of an α-tubulin and/or a Microtubule Associated Protein (MAP) and, as ultimate C-terminal amino acid residue, an aromatic amino acid residue, preferably a tyrosine (Y), and b) an isolated or recombinant TCPase enzyme; in the presence or absence (control) of the modulator compound to be tested, and under conditions for substrate cleavage, preferably detyrosination, and/or liberation of a C-terminal free aromatic amino acid residue, preferably a C-terminal free tyrosine;
(ii) Using reagents for detecting and measuring the signal related to substrate cleavage, preferably detyrosination, and/or liberation of the C-terminal free aromatic amino acid residue, preferably the C-terminal free tyrosine ;
(iii) Measuring and comparing the level of substrate cleavage, preferably detyrosination and/or the level of the C-terminal free aromatic amino acid residue, preferably the C-terminal free tyrosine in presence and in absence (control) of the compound to be tested, and
(iv) Selecting the modulators of TCPase for which the level of substrate cleavage, preferably detyrosination or liberation of the C-terminal aromatic amino acid residue, preferably C-terminal free tyrosine is increased in the presence of the compound to be tested (activators of TCPase) or decreased in the presence of the compound to be tested (inhibitors of TCPase).

The present invention also concerns kits for performing such *in vitro* screening assay.

SEQUENCE LISTING

<110>  CENTRE NATIONAL DE LA RECHERCHE SCIENTIFIQUE (CNRS)
       UNIVERSITE DE MONTPELLIER

<120>  In vitro screening assay of TCPase modulators

<130>  B74240D38071

<160>  40

<170>  PatentIn version 3.5

<210>  1
<211>  494
<212>  PRT
<213>  Homo sapiens (Human)


<220>
<221>  SITE
<222>  (1)..(494)
<223>  Ubiquitin carboxyl-terminal hydrolase 14

<400>  1

Met Pro Leu Tyr Ser Val Thr Val Lys Trp Gly Lys Glu Lys Phe Glu
1               5                   10                  15


Gly Val Glu Leu Asn Thr Asp Glu Pro Pro Met Val Phe Lys Ala Gln
            20                  25                  30


Leu Phe Ala Leu Thr Gly Val Gln Pro Ala Arg Gln Lys Val Met Val
        35                  40                  45


Lys Gly Gly Thr Leu Lys Asp Asp Asp Trp Gly Asn Ile Lys Ile Lys
    50                  55                  60


Asn Gly Met Thr Leu Leu Met Met Gly Ser Ala Asp Ala Leu Pro Glu
65                  70                  75                  80


Glu Pro Ser Ala Lys Thr Val Phe Val Glu Asp Met Thr Glu Glu Gln
                85                  90                  95


Leu Ala Ser Ala Met Glu Leu Pro Cys Gly Leu Thr Asn Leu Gly Asn
            100                 105                 110


Thr Cys Tyr Met Asn Ala Thr Val Gln Cys Ile Arg Ser Val Pro Glu
        115                 120                 125


Leu Lys Asp Ala Leu Lys Arg Tyr Ala Gly Ala Leu Arg Ala Ser Gly
    130                 135                 140


Glu Met Ala Ser Ala Gln Tyr Ile Thr Ala Ala Leu Arg Asp Leu Phe

145                 150                 155                 160

Asp Ser Met Asp Lys Thr Ser Ser Ser Ile Pro Pro Ile Ile Leu Leu
                165                 170                 175

Gln Phe Leu His Met Ala Phe Pro Gln Phe Ala Glu Lys Gly Glu Gln
            180                 185                 190

Gly Gln Tyr Leu Gln Gln Asp Ala Asn Glu Cys Trp Ile Gln Met Met
        195                 200                 205

Arg Val Leu Gln Gln Lys Leu Glu Ala Ile Glu Asp Asp Ser Val Lys
    210                 215                 220

Glu Thr Asp Ser Ser Ser Ala Ser Ala Ala Thr Pro Ser Lys Lys Lys
225                 230                 235                 240

Ser Leu Ile Asp Gln Phe Phe Gly Val Glu Phe Glu Thr Thr Met Lys
                245                 250                 255

Cys Thr Glu Ser Glu Glu Glu Glu Val Thr Lys Gly Lys Glu Asn Gln
            260                 265                 270

Leu Gln Leu Ser Cys Phe Ile Asn Gln Glu Val Lys Tyr Leu Phe Thr
        275                 280                 285

Gly Leu Lys Leu Arg Leu Gln Glu Glu Ile Thr Lys Gln Ser Pro Thr
    290                 295                 300

Leu Gln Arg Asn Ala Leu Tyr Ile Lys Ser Ser Lys Ile Ser Arg Leu
305                 310                 315                 320

Pro Ala Tyr Leu Thr Ile Gln Met Val Arg Phe Phe Tyr Lys Glu Lys
                325                 330                 335

Glu Ser Val Asn Ala Lys Val Leu Lys Asp Val Lys Phe Pro Leu Met
            340                 345                 350

Leu Asp Met Tyr Glu Leu Cys Thr Pro Glu Leu Gln Glu Lys Met Val
        355                 360                 365

Ser Phe Arg Ser Lys Phe Lys Asp Leu Glu Asp Lys Lys Val Asn Gln
    370                 375                 380

Gln Pro Asn Thr Ser Asp Lys Lys Ser Ser Pro Gln Lys Glu Val Lys
385                 390                 395                 400

Tyr Glu Pro Phe Ser Phe Ala Asp Asp Ile Gly Ser Asn Asn Cys Gly
                405                 410                 415

Tyr Tyr Asp Leu Gln Ala Val Leu Thr His Gln Gly Arg Ser Ser Ser
            420                 425                 430

Ser Gly His Tyr Val Ser Trp Val Lys Arg Lys Gln Asp Glu Trp Ile
        435                 440                 445

Lys Phe Asp Asp Asp Lys Val Ser Ile Val Thr Pro Glu Asp Ile Leu
    450                 455                 460

Arg Leu Ser Gly Gly Gly Asp Trp His Ile Ala Tyr Val Leu Leu Tyr
465                 470                 475                 480

Gly Pro Arg Arg Val Glu Ile Met Glu Glu Glu Ser Glu Gln
                485                 490

<210>  2
<211>  858
<212>  PRT
<213>  Homo sapiens (Human)

<220>
<221>  SITE
<222>  (1)..(858)
<223>  Ubiquitin carboxyl-terminal hydrolase 5

<400>  2

Met Ala Glu Leu Ser Glu Glu Ala Leu Leu Ser Val Leu Pro Thr Ile
1               5                   10                  15

Arg Val Pro Lys Ala Gly Asp Arg Val His Lys Asp Glu Cys Ala Phe
            20                  25                  30

Ser Phe Asp Thr Pro Glu Ser Glu Gly Gly Leu Tyr Ile Cys Met Asn
        35                  40                  45

Thr Phe Leu Gly Phe Gly Lys Gln Tyr Val Glu Arg His Phe Asn Lys
    50                  55                  60

Thr Gly Gln Arg Val Tyr Leu His Leu Arg Arg Thr Arg Arg Pro Lys
65                  70                  75                  80

Glu Glu Asp Pro Ala Thr Gly Thr Gly Asp Pro Pro Arg Lys Lys Pro
                85                  90                  95

Thr Arg Leu Ala Ile Gly Val Glu Gly Gly Phe Asp Leu Ser Glu Glu
            100                 105                 110

Lys Phe Glu Leu Asp Glu Asp Val Lys Ile Val Ile Leu Pro Asp Tyr
        115                 120                 125

Leu Glu Ile Ala Arg Asp Gly Leu Gly Gly Leu Pro Asp Ile Val Arg
    130                 135                 140

Asp Arg Val Thr Ser Ala Val Glu Ala Leu Leu Ser Ala Asp Ser Ala
145                 150                 155                 160

Ser Arg Lys Gln Glu Val Gln Ala Trp Asp Gly Glu Val Arg Gln Val
                165                 170                 175

Ser Lys His Ala Phe Ser Leu Lys Gln Leu Asp Asn Pro Ala Arg Ile
            180                 185                 190

Pro Pro Cys Gly Trp Lys Cys Ser Lys Cys Asp Met Arg Glu Asn Leu
        195                 200                 205

Trp Leu Asn Leu Thr Asp Gly Ser Ile Leu Cys Gly Arg Arg Tyr Phe
    210                 215                 220

Asp Gly Ser Gly Gly Asn Asn His Ala Val Glu His Tyr Arg Glu Thr
225                 230                 235                 240

Gly Tyr Pro Leu Ala Val Lys Leu Gly Thr Ile Thr Pro Asp Gly Ala
                245                 250                 255

Asp Val Tyr Ser Tyr Asp Glu Asp Asp Met Val Leu Asp Pro Ser Leu
            260                 265                 270

Ala Glu His Leu Ser His Phe Gly Ile Asp Met Leu Lys Met Gln Lys
        275                 280                 285

Thr Asp Lys Thr Met Thr Glu Leu Glu Ile Asp Met Asn Gln Arg Ile
    290                 295                 300

Gly Glu Trp Glu Leu Ile Gln Glu Ser Gly Val Pro Leu Lys Pro Leu
305                 310                 315                 320

Phe Gly Pro Gly Tyr Thr Gly Ile Arg Asn Leu Gly Asn Ser Cys Tyr
                325                 330                 335

Leu Asn Ser Val Val Gln Val Leu Phe Ser Ile Pro Asp Phe Gln Arg
            340                 345                 350

Lys Tyr Val Asp Lys Leu Glu Lys Ile Phe Gln Asn Ala Pro Thr Asp

355                 360                 365

Pro Thr Gln Asp Phe Ser Thr Gln Val Ala Lys Leu Gly His Gly Leu
    370                 375                 380

Leu Ser Gly Glu Tyr Ser Lys Pro Val Pro Glu Ser Gly Asp Gly Glu
385                 390                 395                 400

Arg Val Pro Glu Gln Lys Glu Val Gln Asp Gly Ile Ala Pro Arg Met
                405                 410                 415

Phe Lys Ala Leu Ile Gly Lys Gly His Pro Glu Phe Ser Thr Asn Arg
            420                 425                 430

Gln Gln Asp Ala Gln Glu Phe Phe Leu His Leu Ile Asn Met Val Glu
        435                 440                 445

Arg Asn Cys Arg Ser Ser Glu Asn Pro Asn Glu Val Phe Arg Phe Leu
    450                 455                 460

Val Glu Glu Lys Ile Lys Cys Leu Ala Thr Glu Lys Val Lys Tyr Thr
465                 470                 475                 480

Gln Arg Val Asp Tyr Ile Met Gln Leu Pro Val Pro Met Asp Ala Ala
                485                 490                 495

Leu Asn Lys Glu Glu Leu Leu Glu Tyr Glu Glu Lys Lys Arg Gln Ala
            500                 505                 510

Glu Glu Glu Lys Met Ala Leu Pro Glu Leu Val Arg Ala Gln Val Pro
        515                 520                 525

Phe Ser Ser Cys Leu Glu Ala Tyr Gly Ala Pro Glu Gln Val Asp Asp
    530                 535                 540

Phe Trp Ser Thr Ala Leu Gln Ala Lys Ser Val Ala Val Lys Thr Thr
545                 550                 555                 560

Arg Phe Ala Ser Phe Pro Asp Tyr Leu Val Ile Gln Ile Lys Lys Phe
                565                 570                 575

Thr Phe Gly Leu Asp Trp Val Pro Lys Lys Leu Asp Val Ser Ile Glu
            580                 585                 590

Met Pro Glu Glu Leu Asp Ile Ser Gln Leu Arg Gly Thr Gly Leu Gln
        595                 600                 605

Pro Gly Glu Glu Glu Leu Pro Asp Ile Ala Pro Pro Leu Val Thr Pro
    610                 615                 620

Asp Glu Pro Lys Gly Ser Leu Gly Phe Tyr Gly Asn Glu Asp Glu Asp
625                 630                 635                 640

Ser Phe Cys Ser Pro His Phe Ser Ser Pro Thr Ser Pro Met Leu Asp
                645                 650                 655

Glu Ser Val Ile Ile Gln Leu Val Glu Met Gly Phe Pro Met Asp Ala
            660                 665                 670

Cys Arg Lys Ala Val Tyr Tyr Thr Gly Asn Ser Gly Ala Glu Ala Ala
        675                 680                 685

Met Asn Trp Val Met Ser His Met Asp Asp Pro Asp Phe Ala Asn Pro
    690                 695                 700

Leu Ile Leu Pro Gly Ser Ser Gly Pro Gly Ser Thr Ser Ala Ala Ala
705                 710                 715                 720

Asp Pro Pro Pro Glu Asp Cys Val Thr Thr Ile Val Ser Met Gly Phe
                725                 730                 735

Ser Arg Asp Gln Ala Leu Lys Ala Leu Arg Ala Thr Asn Asn Ser Leu
            740                 745                 750

Glu Arg Ala Val Asp Trp Ile Phe Ser His Ile Asp Asp Leu Asp Ala
        755                 760                 765

Glu Ala Ala Met Asp Ile Ser Glu Gly Arg Ser Ala Ala Asp Ser Ile
    770                 775                 780

Ser Glu Ser Val Pro Val Gly Pro Lys Val Arg Asp Gly Pro Gly Lys
785                 790                 795                 800

Tyr Gln Leu Phe Ala Phe Ile Ser His Met Gly Thr Ser Thr Met Cys
                805                 810                 815

Gly His Tyr Val Cys His Ile Lys Lys Glu Gly Arg Trp Val Ile Tyr
            820                 825                 830

Asn Asp Gln Lys Val Cys Ala Ser Glu Lys Pro Pro Lys Asp Leu Gly
        835                 840                 845

Tyr Ile Tyr Phe Tyr Gln Arg Val Ala Ser
    850                 855

<210>  3
<211>  478
<212>  PRT
<213>  Homo sapiens (Human)


<220>
<221>  SITE
<222>  (1)..(478)
<223>  Methionine aminopeptidase 2

<400>  3

Met Ala Gly Val Glu Glu Val Ala Ala Ser Gly Ser His Leu Asn Gly
1               5                   10                  15

Asp Leu Asp Pro Asp Asp Arg Glu Glu Gly Ala Ala Ser Thr Ala Glu
            20                  25                  30

Glu Ala Ala Lys Lys Lys Arg Arg Lys Lys Lys Lys Ser Lys Gly Pro
        35                  40                  45

Ser Ala Ala Gly Glu Gln Glu Pro Asp Lys Glu Ser Gly Ala Ser Val
    50                  55                  60

Asp Glu Val Ala Arg Gln Leu Glu Arg Ser Ala Leu Glu Asp Lys Glu
65                  70                  75                  80

Arg Asp Glu Asp Asp Glu Asp Gly Asp Gly Asp Gly Asp Gly Ala Thr
                85                  90                  95

Gly Lys Lys Lys Lys Lys Lys Lys Lys Lys Arg Gly Pro Lys Val Gln
            100                 105                 110

Thr Asp Pro Pro Ser Val Pro Ile Cys Asp Leu Tyr Pro Asn Gly Val
        115                 120                 125

Phe Pro Lys Gly Gln Glu Cys Glu Tyr Pro Pro Thr Gln Asp Gly Arg
    130                 135                 140

Thr Ala Ala Trp Arg Thr Thr Ser Glu Glu Lys Lys Ala Leu Asp Gln
145                 150                 155                 160

Ala Ser Glu Glu Ile Trp Asn Asp Phe Arg Glu Ala Ala Glu Ala His
                165                 170                 175

Arg Gln Val Arg Lys Tyr Val Met Ser Trp Ile Lys Pro Gly Met Thr
            180                 185                 190

Met Ile Glu Ile Cys Glu Lys Leu Glu Asp Cys Ser Arg Lys Leu Ile

195                 200                 205

Lys Glu Asn Gly Leu Asn Ala Gly Leu Ala Phe Pro Thr Gly Cys Ser
    210                 215                 220

Leu Asn Asn Cys Ala Ala His Tyr Thr Pro Asn Ala Gly Asp Thr Thr
225                 230                 235                 240

Val Leu Gln Tyr Asp Asp Ile Cys Lys Ile Asp Phe Gly Thr His Ile
                245                 250                 255

Ser Gly Arg Ile Ile Asp Cys Ala Phe Thr Val Thr Phe Asn Pro Lys
            260                 265                 270

Tyr Asp Thr Leu Leu Lys Ala Val Lys Asp Ala Thr Asn Thr Gly Ile
        275                 280                 285

Lys Cys Ala Gly Ile Asp Val Arg Leu Cys Asp Val Gly Glu Ala Ile
    290                 295                 300

Gln Glu Val Met Glu Ser Tyr Glu Val Glu Ile Asp Gly Lys Thr Tyr
305                 310                 315                 320

Gln Val Lys Pro Ile Arg Asn Leu Asn Gly His Ser Ile Gly Gln Tyr
                325                 330                 335

Arg Ile His Ala Gly Lys Thr Val Pro Ile Val Lys Gly Gly Glu Ala
            340                 345                 350

Thr Arg Met Glu Glu Gly Glu Val Tyr Ala Ile Glu Thr Phe Gly Ser
        355                 360                 365

Thr Gly Lys Gly Val Val His Asp Asp Met Glu Cys Ser His Tyr Met
    370                 375                 380

Lys Asn Phe Asp Val Gly His Val Pro Ile Arg Leu Pro Arg Thr Lys
385                 390                 395                 400

His Leu Leu Asn Val Ile Asn Glu Asn Phe Gly Thr Leu Ala Phe Cys
                405                 410                 415

Arg Arg Trp Leu Asp Arg Leu Gly Glu Ser Lys Tyr Leu Met Ala Leu
            420                 425                 430

Lys Asn Leu Cys Asp Leu Gly Ile Val Asp Pro Tyr Pro Pro Leu Cys
        435                 440                 445

Asp Ile Lys Gly Ser Tyr Thr Ala Gln Phe Glu His Thr Ile Leu Leu
    450                 455                 460

Arg Pro Thr Cys Lys Glu Val Val Ser Arg Gly Asp Asp Tyr
465                 470                 475

<210>  4
<211>  623
<212>  PRT
<213>  Homo sapiens (Human)

<220>
<221>  SITE
<222>  (1)..(623)
<223>  Xaa-Pro aminopeptidase 1

<400>  4

Met Pro Pro Lys Val Thr Ser Glu Leu Leu Arg Gln Leu Arg Gln Ala
1               5                   10                  15

Met Arg Asn Ser Glu Tyr Val Thr Glu Pro Ile Gln Ala Tyr Ile Ile
            20                  25                  30

Pro Ser Gly Asp Ala His Gln Ser Glu Tyr Ile Ala Pro Cys Asp Cys
        35                  40                  45

Arg Arg Ala Phe Val Ser Gly Phe Asp Gly Ser Ala Gly Thr Ala Ile
    50                  55                  60

Ile Thr Glu Glu His Ala Ala Met Trp Thr Asp Gly Arg Tyr Phe Leu
65                  70                  75                  80

Gln Ala Ala Lys Gln Met Asp Ser Asn Trp Thr Leu Met Lys Met Gly
                85                  90                  95

Leu Lys Asp Thr Pro Thr Gln Glu Asp Trp Leu Val Ser Val Leu Pro
            100                 105                 110

Glu Gly Ser Arg Val Gly Val Asp Pro Leu Ile Ile Pro Thr Asp Tyr
        115                 120                 125

Trp Lys Lys Met Ala Lys Val Leu Arg Ser Ala Gly His His Leu Ile
    130                 135                 140

Pro Val Lys Glu Asn Leu Val Asp Lys Ile Trp Thr Asp Arg Pro Glu
145                 150                 155                 160

Arg Pro Cys Lys Pro Leu Leu Thr Leu Gly Leu Asp Tyr Thr Gly Ile
                165                 170                 175

Ser Trp Lys Asp Lys Val Ala Asp Leu Arg Leu Lys Met Ala Glu Arg
            180                 185                 190

Asn Val Met Trp Phe Val Val Thr Ala Leu Asp Glu Ile Ala Trp Leu
        195                 200                 205

Phe Asn Leu Arg Gly Ser Asp Val Glu His Asn Pro Val Phe Phe Ser
    210                 215                 220

Tyr Ala Ile Ile Gly Leu Glu Thr Ile Met Leu Phe Ile Asp Gly Asp
225                 230                 235                 240

Arg Ile Asp Ala Pro Ser Val Lys Glu His Leu Leu Leu Asp Leu Gly
                245                 250                 255

Leu Glu Ala Glu Tyr Arg Ile Gln Val His Pro Tyr Lys Ser Ile Leu
            260                 265                 270

Ser Glu Leu Lys Ala Leu Cys Ala Asp Leu Ser Pro Arg Glu Lys Val
        275                 280                 285

Trp Val Ser Asp Lys Ala Ser Tyr Ala Val Ser Glu Thr Ile Pro Lys
    290                 295                 300

Asp His Arg Cys Cys Met Pro Tyr Thr Pro Ile Cys Ile Ala Lys Ala
305                 310                 315                 320

Val Lys Asn Ser Ala Glu Ser Glu Gly Met Arg Arg Ala His Ile Lys
                325                 330                 335

Asp Ala Val Ala Leu Cys Glu Leu Phe Asn Trp Leu Glu Lys Glu Val
            340                 345                 350

Pro Lys Gly Gly Val Thr Glu Ile Ser Ala Ala Asp Lys Ala Glu Glu
        355                 360                 365

Phe Arg Arg Gln Gln Ala Asp Phe Val Asp Leu Ser Phe Pro Thr Ile
    370                 375                 380

Ser Ser Thr Gly Pro Asn Gly Ala Ile Ile His Tyr Ala Pro Val Pro
385                 390                 395                 400

Glu Thr Asn Arg Thr Leu Ser Leu Asp Glu Val Tyr Leu Ile Asp Ser
                405                 410                 415

Gly Ala Gln Tyr Lys Asp Gly Thr Thr Asp Val Thr Arg Thr Met His

420                 425                 430

Phe Gly Thr Pro Thr Ala Tyr Glu Lys Glu Cys Phe Thr Tyr Val Leu
        435                 440                 445

Lys Gly His Ile Ala Val Ser Ala Ala Val Phe Pro Thr Gly Thr Lys
    450                 455                 460

Gly His Leu Leu Asp Ser Phe Ala Arg Ser Ala Leu Trp Asp Ser Gly
465                 470                 475                 480

Leu Asp Tyr Leu His Gly Thr Gly His Gly Val Gly Ser Phe Leu Asn
                485                 490                 495

Val His Glu Gly Pro Cys Gly Ile Ser Tyr Lys Thr Phe Ser Asp Glu
            500                 505                 510

Pro Leu Glu Ala Gly Met Ile Val Thr Asp Glu Pro Gly Tyr Tyr Glu
        515                 520                 525

Asp Gly Ala Phe Gly Ile Arg Ile Glu Asn Val Val Leu Val Val Pro
    530                 535                 540

Val Lys Thr Lys Tyr Asn Phe Asn Asn Arg Gly Ser Leu Thr Phe Glu
545                 550                 555                 560

Pro Leu Thr Leu Val Pro Ile Gln Thr Lys Met Ile Asp Val Asp Ser
                565                 570                 575

Leu Thr Asp Lys Glu Cys Asp Trp Leu Asn Asn Tyr His Leu Thr Cys
            580                 585                 590

Arg Asp Val Ile Gly Lys Glu Leu Gln Lys Gln Gly Arg Gln Glu Ala
        595                 600                 605

Leu Glu Trp Leu Ile Arg Glu Thr Gln Pro Ile Ser Lys Gln His
    610                 615                 620

<210>  5
<211>  1249
<212>  PRT
<213>  Homo sapiens (Human)

<220>
<221>  SITE
<222>  (1)..(1249)
<223>  Tripeptidyl-peptidase 2

<400>  5

Met Ala Thr Ala Ala Thr Glu Glu Pro Phe Pro Phe His Gly Leu Leu
1               5                   10                  15

Pro Lys Lys Glu Thr Gly Ala Ala Ser Phe Leu Cys Arg Tyr Pro Glu
            20                  25                  30

Tyr Asp Gly Arg Gly Val Leu Ile Ala Val Leu Asp Thr Gly Val Asp
        35                  40                  45

Pro Gly Ala Pro Gly Met Gln Val Thr Thr Asp Gly Lys Pro Lys Ile
    50                  55                  60

Val Asp Ile Ile Asp Thr Thr Gly Ser Gly Asp Val Asn Thr Ala Thr
65                  70                  75                  80

Glu Val Glu Pro Lys Asp Gly Glu Ile Val Gly Leu Ser Gly Arg Val
                85                  90                  95

Leu Lys Ile Pro Ala Ser Trp Thr Asn Pro Ser Gly Lys Tyr His Ile
            100                 105                 110

Gly Ile Lys Asn Gly Tyr Asp Phe Tyr Pro Lys Ala Leu Lys Glu Arg
        115                 120                 125

Ile Gln Lys Glu Arg Lys Glu Lys Ile Trp Asp Pro Val His Arg Val
    130                 135                 140

Ala Leu Ala Glu Ala Cys Arg Lys Gln Glu Glu Phe Asp Val Ala Asn
145                 150                 155                 160

Asn Gly Ser Ser Gln Ala Asn Lys Leu Ile Lys Glu Glu Leu Gln Ser
                165                 170                 175

Gln Val Glu Leu Leu Asn Ser Phe Glu Lys Lys Tyr Ser Asp Pro Gly
            180                 185                 190

Pro Val Tyr Asp Cys Leu Val Trp His Asp Gly Glu Val Trp Arg Ala
        195                 200                 205

Cys Ile Asp Ser Asn Glu Asp Gly Asp Leu Ser Lys Ser Thr Val Leu
    210                 215                 220

Arg Asn Tyr Lys Glu Ala Gln Glu Tyr Gly Ser Phe Gly Thr Ala Glu
225                 230                 235                 240

Met Leu Asn Tyr Ser Val Asn Ile Tyr Asp Asp Gly Asn Leu Leu Ser
                245                 250                 255

Ile Val Thr Ser Gly Gly Ala His Gly Thr His Val Ala Ser Ile Ala
        260                 265                 270

Ala Gly His Phe Pro Glu Glu Pro Glu Arg Asn Gly Val Ala Pro Gly
    275                 280                 285

Ala Gln Ile Leu Ser Ile Lys Ile Gly Asp Thr Arg Leu Ser Thr Met
    290                 295                 300

Glu Thr Gly Thr Gly Leu Ile Arg Ala Met Ile Glu Val Ile Asn His
305                 310                 315                 320

Lys Cys Asp Leu Val Asn Tyr Ser Tyr Gly Glu Ala Thr His Trp Pro
                325                 330                 335

Asn Ser Gly Arg Ile Cys Glu Val Ile Asn Glu Ala Val Trp Lys His
        340                 345                 350

Asn Ile Ile Tyr Val Ser Ser Ala Gly Asn Asn Gly Pro Cys Leu Ser
    355                 360                 365

Thr Val Gly Cys Pro Gly Gly Thr Thr Ser Ser Val Ile Gly Val Gly
    370                 375                 380

Ala Tyr Val Ser Pro Asp Met Met Val Ala Glu Tyr Ser Leu Arg Glu
385                 390                 395                 400

Lys Leu Pro Ala Asn Gln Tyr Thr Trp Ser Ser Arg Gly Pro Ser Ala
                405                 410                 415

Asp Gly Ala Leu Gly Val Ser Ile Ser Ala Pro Gly Gly Ala Ile Ala
        420                 425                 430

Ser Val Pro Asn Trp Thr Leu Arg Gly Thr Gln Leu Met Asn Gly Thr
    435                 440                 445

Ser Met Ser Ser Pro Asn Ala Cys Gly Gly Ile Ala Leu Ile Leu Ser
    450                 455                 460

Gly Leu Lys Ala Asn Asn Ile Asp Tyr Thr Val His Ser Val Arg Arg
465                 470                 475                 480

Ala Leu Glu Asn Thr Ala Val Lys Ala Asp Asn Ile Glu Val Phe Ala
                485                 490                 495

Gln Gly His Gly Ile Ile Gln Val Asp Lys Ala Tyr Asp Tyr Leu Val

            500                 505                 510

Gln Asn Thr Ser Phe Ala Asn Lys Leu Gly Phe Thr Val Thr Val Gly
        515                 520                 525

Asn Asn Arg Gly Ile Tyr Leu Arg Asp Pro Val Gln Val Ala Ala Pro
    530                 535                 540

Ser Asp His Gly Val Gly Ile Glu Pro Val Phe Pro Glu Asn Thr Glu
545                 550                 555                 560

Asn Ser Glu Lys Ile Ser Leu Gln Leu His Leu Ala Leu Thr Ser Asn
                565                 570                 575

Ser Ser Trp Val Gln Cys Pro Ser His Leu Glu Leu Met Asn Gln Cys
            580                 585                 590

Arg His Ile Asn Ile Arg Val Asp Pro Arg Gly Leu Arg Glu Gly Leu
        595                 600                 605

His Tyr Thr Glu Val Cys Gly Tyr Asp Ile Ala Ser Pro Asn Ala Gly
    610                 615                 620

Pro Leu Phe Arg Val Pro Ile Thr Ala Val Ile Ala Ala Lys Val Asn
625                 630                 635                 640

Glu Ser Ser His Tyr Asp Leu Ala Phe Thr Asp Val His Phe Lys Pro
                645                 650                 655

Gly Gln Ile Arg Arg His Phe Ile Glu Val Pro Glu Gly Ala Thr Trp
            660                 665                 670

Ala Glu Val Thr Val Cys Ser Cys Ser Ser Glu Val Ser Ala Lys Phe
        675                 680                 685

Val Leu His Ala Val Gln Leu Val Lys Gln Arg Ala Tyr Arg Ser His
    690                 695                 700

Glu Phe Tyr Lys Phe Cys Ser Leu Pro Glu Lys Gly Thr Leu Thr Glu
705                 710                 715                 720

Ala Phe Pro Val Leu Gly Gly Lys Ala Ile Glu Phe Cys Ile Ala Arg
                725                 730                 735

Trp Trp Ala Ser Leu Ser Asp Val Asn Ile Asp Tyr Thr Ile Ser Phe
            740                 745                 750

His Gly Ile Val Cys Thr Ala Pro Gln Leu Asn Ile His Ala Ser Glu
        755                 760                 765

Gly Ile Asn Arg Phe Asp Val Gln Ser Ser Leu Lys Tyr Glu Asp Leu
    770                 775                 780

Ala Pro Cys Ile Thr Leu Lys Asn Trp Val Gln Thr Leu Arg Pro Val
785                 790                 795                 800

Ser Ala Lys Thr Lys Pro Leu Gly Ser Arg Asp Val Leu Pro Asn Asn
                805                 810                 815

Arg Gln Leu Tyr Glu Met Val Leu Thr Tyr Asn Phe His Gln Pro Lys
            820                 825                 830

Ser Gly Glu Val Thr Pro Ser Cys Pro Leu Leu Cys Glu Leu Leu Tyr
        835                 840                 845

Glu Ser Glu Phe Asp Ser Gln Leu Trp Ile Ile Phe Asp Gln Asn Lys
    850                 855                 860

Arg Gln Met Gly Ser Gly Asp Ala Tyr Pro His Gln Tyr Ser Leu Lys
865                 870                 875                 880

Leu Glu Lys Gly Asp Tyr Thr Ile Arg Leu Gln Ile Arg His Glu Gln
                885                 890                 895

Ile Ser Asp Leu Glu Arg Leu Lys Asp Leu Pro Phe Ile Val Ser His
            900                 905                 910

Arg Leu Ser Asn Thr Leu Ser Leu Asp Ile His Glu Asn His Ser Phe
        915                 920                 925

Ala Leu Leu Gly Lys Lys Lys Ser Ser Asn Leu Thr Leu Pro Pro Lys
    930                 935                 940

Tyr Asn Gln Pro Phe Phe Val Thr Ser Leu Pro Asp Asp Lys Ile Pro
945                 950                 955                 960

Lys Gly Ala Gly Pro Gly Cys Tyr Leu Ala Gly Ser Leu Thr Leu Ser
                965                 970                 975

Lys Thr Glu Leu Gly Lys Lys Ala Asp Val Ile Pro Val His Tyr Tyr
            980                 985                 990

Leu Ile Pro Pro Pro Thr Lys Thr  Lys Asn Gly Ser Lys  Asp Lys Glu
        995                 1000                1005

Lys Asp Ser Glu Lys Glu Lys Asp Leu Lys Glu Glu Phe Thr Glu
    1010                1015                1020

Ala Leu Arg Asp Leu Lys Ile Gln Trp Met Thr Lys Leu Asp Ser
    1025                1030                1035

Ser Asp Ile Tyr Asn Glu Leu Lys Glu Thr Tyr Pro Asn Tyr Leu
    1040                1045                1050

Pro Leu Tyr Val Ala Arg Leu His Gln Leu Asp Ala Glu Lys Glu
    1055                1060                1065

Arg Met Lys Arg Leu Asn Glu Ile Val Asp Ala Ala Asn Ala Val
    1070                1075                1080

Ile Ser His Ile Asp Gln Thr Ala Leu Ala Val Tyr Ile Ala Met
    1085                1090                1095

Lys Thr Asp Pro Arg Pro Asp Ala Ala Thr Ile Lys Asn Asp Met
    1100                1105                1110

Asp Lys Gln Lys Ser Thr Leu Val Asp Ala Leu Cys Arg Lys Gly
    1115                1120                1125

Cys Ala Leu Ala Asp His Leu Leu His Thr Gln Ala Gln Asp Gly
    1130                1135                1140

Ala Ile Ser Thr Asp Ala Glu Gly Lys Glu Glu Glu Gly Glu Ser
    1145                1150                1155

Pro Leu Asp Ser Leu Ala Glu Thr Phe Trp Glu Thr Thr Lys Trp
    1160                1165                1170

Thr Asp Leu Phe Asp Asn Lys Val Leu Thr Phe Ala Tyr Lys His
    1175                1180                1185

Ala Leu Val Asn Lys Met Tyr Gly Arg Gly Leu Lys Phe Ala Thr
    1190                1195                1200

Lys Leu Val Glu Glu Lys Pro Thr Lys Glu Asn Trp Lys Asn Cys
    1205                1210                1215

Ile Gln Leu Met Lys Leu Leu Gly Trp Thr His Cys Ala Ser Phe
    1220                1225                1230

Thr Glu Asn Trp Leu Pro Ile Met Tyr Pro Pro Asp Tyr Cys Val
    1235                1240                1245

Phe

<210>  6
<211>  365
<212>  PRT
<213>  Homo sapiens (Human)

<220>
<221>  SITE
<222>  (1)..(365)
<223>  Vasohibin-1

<400>  6

Met Pro Gly Gly Lys Lys Val Ala Gly Gly Gly Ser Ser Gly Ala Thr
1               5                   10                  15

Pro Thr Ser Ala Ala Ala Thr Ala Pro Ser Gly Val Arg Arg Leu Glu
            20                  25                  30

Thr Ser Glu Gly Thr Ser Ala Gln Arg Asp Glu Glu Pro Glu Glu Glu
        35                  40                  45

Gly Glu Glu Asp Leu Arg Asp Gly Gly Val Pro Phe Phe Val Asn Arg
    50                  55                  60

Gly Gly Leu Pro Val Asp Glu Ala Thr Trp Glu Arg Met Trp Lys His
65                  70                  75                  80

Val Ala Lys Ile His Pro Asp Gly Glu Lys Val Ala Gln Arg Ile Arg
                85                  90                  95

Gly Ala Thr Asp Leu Pro Lys Ile Pro Ile Pro Ser Val Pro Thr Phe
            100                 105                 110

Gln Pro Ser Thr Pro Val Pro Glu Arg Leu Glu Ala Val Gln Arg Tyr
        115                 120                 125

Ile Arg Glu Leu Gln Tyr Asn His Thr Gly Thr Gln Phe Phe Glu Ile
    130                 135                 140

Lys Lys Ser Arg Pro Leu Thr Gly Leu Met Asp Leu Ala Lys Glu Met
145                 150                 155                 160

Thr Lys Glu Ala Leu Pro Ile Lys Cys Leu Glu Ala Val Ile Leu Gly
                165                 170                 175

Ile Tyr Leu Thr Asn Ser Met Pro Thr Leu Glu Arg Phe Pro Ile Ser
            180                 185                 190

Phe Lys Thr Tyr Phe Ser Gly Asn Tyr Phe Arg His Ile Val Leu Gly
        195                 200                 205

Val Asn Phe Ala Gly Arg Tyr Gly Ala Leu Gly Met Ser Arg Arg Glu
    210                 215                 220

Asp Leu Met Tyr Lys Pro Pro Ala Phe Arg Thr Leu Ser Glu Leu Val
225                 230                 235                 240

Leu Asp Phe Glu Ala Ala Tyr Gly Arg Cys Trp His Val Leu Lys Lys
                245                 250                 255

Val Lys Leu Gly Gln Ser Val Ser His Asp Pro His Ser Val Glu Gln
            260                 265                 270

Ile Glu Trp Lys His Ser Val Leu Asp Val Glu Arg Leu Gly Arg Asp
        275                 280                 285

Asp Phe Arg Lys Glu Leu Glu Arg His Ala Arg Asp Met Arg Leu Lys
    290                 295                 300

Ile Gly Lys Gly Thr Gly Pro Pro Ser Pro Thr Lys Asp Arg Lys Lys
305                 310                 315                 320

Asp Val Ser Ser Pro Gln Arg Ala Gln Ser Ser Pro His Arg Arg Asn
                325                 330                 335

Ser Arg Ser Glu Arg Arg Pro Ser Gly Asp Lys Lys Thr Ser Glu Pro
            340                 345                 350

Lys Ala Met Pro Asp Leu Asn Gly Tyr Gln Ile Arg Val
        355                 360                 365

<210>  7
<211>  572
<212>  PRT
<213>  Homo sapiens (Human)

<220>
<221>  SITE
<222>  (1)..(572)
<223>  Dihydropyrimidinase-related protein 1

<220>
<221>  SITE
<222>  (1)..(572)
<223>  CRMP1

<400>  7

Met Ser Tyr Gln Gly Lys Lys Ser Ile Pro His Ile Thr Ser Asp Arg
1               5                   10                  15

Leu Leu Ile Lys Gly Gly Arg Ile Ile Asn Asp Asp Gln Ser Leu Tyr
            20                  25                  30

Ala Asp Val Tyr Leu Glu Asp Gly Leu Ile Lys Gln Ile Gly Glu Asn
        35                  40                  45

Leu Ile Val Pro Gly Gly Val Lys Thr Ile Glu Ala Asn Gly Arg Met
    50                  55                  60

Val Ile Pro Gly Gly Ile Asp Val Asn Thr Tyr Leu Gln Lys Pro Ser
65                  70                  75                  80

Gln Gly Met Thr Ala Ala Asp Asp Phe Phe Gln Gly Thr Arg Ala Ala
                85                  90                  95

Leu Val Gly Gly Thr Thr Met Ile Ile Asp His Val Val Pro Glu Pro
            100                 105                 110

Gly Ser Ser Leu Leu Thr Ser Phe Glu Lys Trp His Glu Ala Ala Asp
        115                 120                 125

Thr Lys Ser Cys Cys Asp Tyr Ser Leu His Val Asp Ile Thr Ser Trp
    130                 135                 140

Tyr Asp Gly Val Arg Glu Glu Leu Glu Val Leu Val Gln Asp Lys Gly
145                 150                 155                 160

Val Asn Ser Phe Gln Val Tyr Met Ala Tyr Lys Asp Val Tyr Gln Met
                165                 170                 175

Ser Asp Ser Gln Leu Tyr Glu Ala Phe Thr Phe Leu Lys Gly Leu Gly
            180                 185                 190

Ala Val Ile Leu Val His Ala Glu Asn Gly Asp Leu Ile Ala Gln Glu
        195                 200                 205

Gln Lys Arg Ile Leu Glu Met Gly Ile Thr Gly Pro Glu Gly His Ala
    210                 215                 220

Leu Ser Arg Pro Glu Glu Leu Glu Ala Glu Ala Val Phe Arg Ala Ile
225                 230                 235                 240

Thr Ile Ala Gly Arg Ile Asn Cys Pro Val Tyr Ile Thr Lys Val Met
                245                 250                 255

Ser Lys Ser Ala Ala Asp Ile Ile Ala Leu Ala Arg Lys Lys Gly Pro
            260                 265                 270

Leu Val Phe Gly Glu Pro Ile Ala Ala Ser Leu Gly Thr Asp Gly Thr
        275                 280                 285

His Tyr Trp Ser Lys Asn Trp Ala Lys Ala Ala Ala Phe Val Thr Ser
    290                 295                 300

Pro Pro Leu Ser Pro Asp Pro Thr Thr Pro Asp Tyr Leu Thr Ser Leu
305                 310                 315                 320

Leu Ala Cys Gly Asp Leu Gln Val Thr Gly Ser Gly His Cys Pro Tyr
                325                 330                 335

Ser Thr Ala Gln Lys Ala Val Gly Lys Asp Asn Phe Thr Leu Ile Pro
            340                 345                 350

Glu Gly Val Asn Gly Ile Glu Glu Arg Met Thr Val Val Trp Asp Lys
        355                 360                 365

Ala Val Ala Thr Gly Lys Met Asp Glu Asn Gln Phe Val Ala Val Thr
    370                 375                 380

Ser Thr Asn Ala Ala Lys Ile Phe Asn Leu Tyr Pro Arg Lys Gly Arg
385                 390                 395                 400

Ile Ala Val Gly Ser Asp Ala Asp Val Val Ile Trp Asp Pro Asp Lys
                405                 410                 415

Leu Lys Thr Ile Thr Ala Lys Ser His Lys Ser Ala Val Glu Tyr Asn
            420                 425                 430

Ile Phe Glu Gly Met Glu Cys His Gly Ser Pro Leu Val Val Ile Ser
        435                 440                 445

Gln Gly Lys Ile Val Phe Glu Asp Gly Asn Ile Asn Val Asn Lys Gly
    450                 455                 460

Met Gly Arg Phe Ile Pro Arg Lys Ala Phe Pro Glu His Leu Tyr Gln
465                 470                 475                 480

Arg Val Lys Ile Arg Asn Lys Val Phe Gly Leu Gln Gly Val Ser Arg
                485                 490                 495

Gly Met Tyr Asp Gly Pro Val Tyr Glu Val Pro Ala Thr Pro Lys Tyr
            500                 505                 510

Ala Thr Pro Ala Pro Ser Ala Lys Ser Ser Pro Ser Lys His Gln Pro
        515                 520                 525

Pro Pro Ile Arg Asn Leu His Gln Ser Asn Phe Ser Leu Ser Gly Ala
    530                 535                 540

Gln Ile Asp Asp Asn Asn Pro Arg Arg Thr Gly His Arg Ile Val Ala
545                 550                 555                 560

Pro Pro Gly Gly Arg Ser Asn Ile Thr Ser Leu Gly
                565                 570

<210>  8
<211>  572
<212>  PRT
<213>  Homo sapiens (Human)

<220>
<221>  SITE
<222>  (1)..(572)
<223>  Dihydropyrimidinase-related protein 2

<400>  8

<210>  9
<211>  570
<212>  PRT
<213>  Homo sapiens (Human)

<220>
<221>  SITE
<222>  (1)..(570)
<223>  Dihydropyrimidinase-related protein 3

<400>  9

Met Ser Tyr Gln Gly Lys Lys Asn Ile Pro Arg Ile Thr Ser Asp Arg
1               5                   10                  15

Leu Leu Ile Lys Gly Gly Arg Ile Val Asn Asp Asp Gln Ser Phe Tyr
            20                  25                  30

Ala Asp Ile Tyr Met Glu Asp Gly Leu Ile Lys Gln Ile Gly Asp Asn
        35                  40                  45

Leu Ile Val Pro Gly Gly Val Lys Thr Ile Glu Ala Asn Gly Lys Met
    50                  55                  60

Val Ile Pro Gly Gly Ile Asp Val His Thr His Phe Gln Met Pro Tyr
65                  70                  75                  80

Lys Gly Met Thr Thr Val Asp Asp Phe Phe Gln Gly Thr Lys Ala Ala
                85                  90                  95

Leu Ala Gly Gly Thr Thr Met Ile Ile Asp His Val Val Pro Glu Pro
            100                 105                 110

Glu Ser Ser Leu Thr Glu Ala Tyr Glu Lys Trp Arg Glu Trp Ala Asp
        115                 120                 125

Gly Lys Ser Cys Cys Asp Tyr Ala Leu His Val Asp Ile Thr His Trp
    130                 135                 140

Asn Asp Ser Val Lys Gln Glu Val Gln Asn Leu Ile Lys Asp Lys Gly
145                 150                 155                 160

Val Asn Ser Phe Met Val Tyr Met Ala Tyr Lys Asp Leu Tyr Gln Val
                165                 170                 175

Ser Asn Thr Glu Leu Tyr Glu Ile Phe Thr Cys Leu Gly Glu Leu Gly
            180                 185                 190

Ala Ile Ala Gln Val His Ala Glu Asn Gly Asp Ile Ile Ala Gln Glu
        195                 200                 205

Gln Thr Arg Met Leu Glu Met Gly Ile Thr Gly Pro Glu Gly His Val
    210                 215                 220

Leu Ser Arg Pro Glu Glu Leu Glu Ala Glu Ala Val Phe Arg Ala Ile
225                 230                 235                 240

Thr Ile Ala Ser Gln Thr Asn Cys Pro Leu Tyr Val Thr Lys Val Met

245                 250                 255

Ser Lys Ser Ala Ala Asp Leu Ile Ser Gln Ala Arg Lys Lys Gly Asn
            260                 265                 270

Val Val Phe Gly Glu Pro Ile Thr Ala Ser Leu Gly Ile Asp Gly Thr
        275                 280                 285

His Tyr Trp Ser Lys Asn Trp Ala Lys Ala Ala Ala Phe Val Thr Ser
    290                 295                 300

Pro Pro Leu Ser Pro Asp Pro Thr Thr Pro Asp Tyr Ile Asn Ser Leu
305                 310                 315                 320

Leu Ala Ser Gly Asp Leu Gln Leu Ser Gly Ser Ala His Cys Thr Phe
                325                 330                 335

Ser Thr Ala Gln Lys Ala Ile Gly Lys Asp Asn Phe Thr Ala Ile Pro
            340                 345                 350

Glu Gly Thr Asn Gly Val Glu Glu Arg Met Ser Val Ile Trp Asp Lys
        355                 360                 365

Ala Val Ala Thr Gly Lys Met Asp Glu Asn Gln Phe Val Ala Val Thr
    370                 375                 380

Ser Thr Asn Ala Ala Lys Ile Phe Asn Leu Tyr Pro Arg Lys Gly Arg
385                 390                 395                 400

Ile Ser Val Gly Ser Asp Ser Asp Leu Val Ile Trp Asp Pro Asp Ala
                405                 410                 415

Val Lys Ile Val Ser Ala Lys Asn His Gln Ser Ala Ala Glu Tyr Asn
            420                 425                 430

Ile Phe Glu Gly Met Glu Leu Arg Gly Ala Pro Leu Val Val Ile Cys
        435                 440                 445

Gln Gly Lys Ile Met Leu Glu Asp Gly Asn Leu His Val Thr Gln Gly
    450                 455                 460

Ala Gly Arg Phe Ile Pro Cys Ser Pro Phe Ser Asp Tyr Val Tyr Lys
465                 470                 475                 480

Arg Ile Lys Ala Arg Arg Lys Met Ala Asp Leu His Ala Val Pro Arg
                485                 490                 495

Gly Met Tyr Asp Gly Pro Val Phe Asp Leu Thr Thr Thr Pro Lys Gly
          500                 505                 510

Gly Thr Pro Ala Gly Ser Ala Arg Gly Ser Pro Thr Arg Pro Asn Pro
        515                 520                 525

Pro Val Arg Asn Leu His Gln Ser Gly Phe Ser Leu Ser Gly Thr Gln
    530                 535                 540

Val Asp Glu Gly Val Arg Ser Ala Ser Lys Arg Ile Val Ala Pro Pro
545                 550                 555                 560

Gly Gly Arg Ser Asn Ile Thr Ser Leu Ser
                565                 570

<210>  10
<211>  572
<212>  PRT
<213>  Homo sapiens (Human)

<220>
<221>  SITE
<222>  (1)..(572)
<223>  Dihydropyrimidinase-related protein 4

<400>  10

Met Ser Phe Gln Gly Lys Lys Ser Ile Pro Arg Ile Thr Ser Asp Arg
1               5                   10                  15

Leu Leu Ile Arg Gly Gly Arg Ile Val Asn Asp Asp Gln Ser Phe Tyr
            20                  25                  30

Ala Asp Val His Val Glu Asp Gly Leu Ile Lys Gln Ile Gly Glu Asn
        35                  40                  45

Leu Ile Val Pro Gly Gly Ile Lys Thr Ile Asp Ala His Gly Leu Met
    50                  55                  60

Val Leu Pro Gly Gly Val Asp Val His Thr Arg Leu Gln Met Pro Val
65                  70                  75                  80

Leu Gly Met Thr Pro Ala Asp Asp Phe Cys Gln Gly Thr Lys Ala Ala
                85                  90                  95

Leu Ala Gly Gly Thr Thr Met Ile Leu Asp His Val Phe Pro Asp Thr
            100                 105                 110

Gly Val Ser Leu Leu Ala Ala Tyr Glu Gln Trp Arg Glu Arg Ala Asp
        115                 120                 125

Ser Ala Ala Cys Cys Asp Tyr Ser Leu His Val Asp Ile Thr Arg Trp
    130                 135                 140

His Glu Ser Ile Lys Glu Glu Leu Glu Ala Leu Val Lys Glu Lys Gly
145                 150                 155                 160

Val Asn Ser Phe Leu Val Phe Met Ala Tyr Lys Asp Arg Cys Gln Cys
                165                 170                 175

Ser Asp Ser Gln Met Tyr Glu Ile Phe Ser Ile Ile Arg Asp Leu Gly
            180                 185                 190

Ala Leu Ala Gln Val His Ala Glu Asn Gly Asp Ile Val Glu Glu Glu
        195                 200                 205

Gln Lys Arg Leu Leu Glu Leu Gly Ile Thr Gly Pro Glu Gly His Val
    210                 215                 220

Leu Ser His Pro Glu Glu Val Glu Ala Glu Ala Val Tyr Arg Ala Val
225                 230                 235                 240

Thr Ile Ala Lys Gln Ala Asn Cys Pro Leu Tyr Val Thr Lys Val Met
                245                 250                 255

Ser Lys Gly Ala Ala Asp Ala Ile Ala Gln Ala Lys Arg Arg Gly Val
            260                 265                 270

Val Val Phe Gly Glu Pro Ile Thr Ala Ser Leu Gly Thr Asp Gly Ser
        275                 280                 285

His Tyr Trp Ser Lys Asn Trp Ala Lys Ala Ala Ala Phe Val Thr Ser
    290                 295                 300

Pro Pro Val Asn Pro Asp Pro Thr Thr Ala Asp His Leu Thr Cys Leu
305                 310                 315                 320

Leu Ser Ser Gly Asp Leu Gln Val Thr Gly Ser Ala His Cys Thr Phe
                325                 330                 335

Thr Thr Ala Gln Lys Ala Val Gly Lys Asp Asn Phe Ala Leu Ile Pro
            340                 345                 350

Glu Gly Thr Asn Gly Ile Glu Glu Arg Met Ser Met Val Trp Glu Lys
        355                 360                 365

Cys Val Ala Ser Gly Lys Met Asp Glu Asn Glu Phe Val Ala Val Thr

370                 375                 380

Ser Thr Asn Ala Ala Lys Ile Phe Asn Phe Tyr Pro Arg Lys Gly Arg
385                 390                 395                 400

Val Ala Val Gly Ser Asp Ala Asp Leu Val Ile Trp Asn Pro Lys Ala
                405                 410                 415

Thr Lys Ile Ile Ser Ala Lys Thr His Asn Leu Asn Val Glu Tyr Asn
            420                 425                 430

Ile Phe Glu Gly Val Glu Cys Arg Gly Ala Pro Ala Val Val Ile Ser
        435                 440                 445

Gln Gly Arg Val Ala Leu Glu Asp Gly Lys Met Phe Val Thr Pro Gly
    450                 455                 460

Ala Gly Arg Phe Val Pro Arg Lys Thr Phe Pro Asp Phe Val Tyr Lys
465                 470                 475                 480

Arg Ile Lys Ala Arg Asn Arg Leu Ala Glu Ile His Gly Val Pro Arg
                485                 490                 495

Gly Leu Tyr Asp Gly Pro Val His Glu Val Met Val Pro Ala Lys Pro
            500                 505                 510

Gly Ser Gly Ala Pro Ala Arg Ala Ser Cys Pro Gly Lys Ile Ser Val
        515                 520                 525

Pro Pro Val Arg Asn Leu His Gln Ser Gly Phe Ser Leu Ser Gly Ser
    530                 535                 540

Gln Ala Asp Asp His Ile Ala Arg Arg Thr Ala Gln Lys Ile Met Ala
545                 550                 555                 560

Pro Pro Gly Gly Arg Ser Asn Ile Thr Ser Leu Ser
                565                 570

<210>  11
<211>  564
<212>  PRT
<213>  Homo sapiens (Human)

<220>
<221>  SITE
<222>  (1)..(564)
<223>  Dihydropyrimidinase-related protein 5

<400>  11

Met Leu Ala Asn Ser Ala Ser Val Arg Ile Leu Ile Lys Gly Gly Lys
1               5                   10                  15

Val Val Asn Asp Asp Cys Thr His Glu Ala Asp Val Tyr Ile Glu Asn
            20                  25                  30

Gly Ile Ile Gln Gln Val Gly Arg Glu Leu Met Ile Pro Gly Gly Ala
        35                  40                  45

Lys Val Ile Asp Ala Thr Gly Lys Leu Val Ile Pro Gly Gly Ile Asp
    50                  55                  60

Thr Ser Thr His Phe His Gln Thr Phe Met Asn Ala Thr Cys Val Asp
65                  70                  75                  80

Asp Phe Tyr His Gly Thr Lys Ala Ala Leu Val Gly Gly Thr Thr Met
                85                  90                  95

Ile Ile Gly His Val Leu Pro Asp Lys Glu Thr Ser Leu Val Asp Ala
            100                 105                 110

Tyr Glu Lys Cys Arg Gly Leu Ala Asp Pro Lys Val Cys Cys Asp Tyr
        115                 120                 125

Ala Leu His Val Gly Ile Thr Trp Trp Ala Pro Lys Val Lys Ala Glu
    130                 135                 140

Met Glu Thr Leu Val Arg Glu Lys Gly Val Asn Ser Phe Gln Met Phe
145                 150                 155                 160

Met Thr Tyr Lys Asp Leu Tyr Met Leu Arg Asp Ser Glu Leu Tyr Gln
                165                 170                 175

Val Leu His Ala Cys Lys Asp Ile Gly Ala Ile Ala Arg Val His Ala
            180                 185                 190

Glu Asn Gly Glu Leu Val Ala Glu Gly Ala Lys Glu Ala Leu Asp Leu
        195                 200                 205

Gly Ile Thr Gly Pro Glu Gly Ile Glu Ile Ser Arg Pro Glu Glu Leu
    210                 215                 220

Glu Ala Glu Ala Thr His Arg Val Ile Thr Ile Ala Asn Arg Thr His
225                 230                 235                 240

Cys Pro Ile Tyr Leu Val Asn Val Ser Ser Ile Ser Ala Gly Asp Val
                245                 250                 255

Ile Ala Ala Ala Lys Met Gln Gly Lys Val Val Leu Ala Glu Thr Thr
            260                 265                 270

Thr Ala His Ala Thr Leu Thr Gly Leu His Tyr Tyr His Gln Asp Trp
        275                 280                 285

Ser His Ala Ala Ala Tyr Val Thr Val Pro Pro Leu Arg Leu Asp Thr
    290                 295                 300

Asn Thr Ser Thr Tyr Leu Met Ser Leu Leu Ala Asn Asp Thr Leu Asn
305                 310                 315                 320

Ile Val Ala Ser Asp His Arg Pro Phe Thr Thr Lys Gln Lys Ala Met
                325                 330                 335

Gly Lys Glu Asp Phe Thr Lys Ile Pro His Gly Val Ser Gly Val Gln
            340                 345                 350

Asp Arg Met Ser Val Ile Trp Glu Arg Gly Val Val Gly Gly Lys Met
        355                 360                 365

Asp Glu Asn Arg Phe Val Ala Val Thr Ser Ser Asn Ala Ala Lys Leu
    370                 375                 380

Leu Asn Leu Tyr Pro Arg Lys Gly Arg Ile Ile Pro Gly Ala Asp Ala
385                 390                 395                 400

Asp Val Val Val Trp Asp Pro Glu Ala Thr Lys Thr Ile Ser Ala Ser
                405                 410                 415

Thr Gln Val Gln Gly Gly Asp Phe Asn Leu Tyr Glu Asn Met Arg Cys
            420                 425                 430

His Gly Val Pro Leu Val Thr Ile Ser Arg Gly Arg Val Val Tyr Glu
        435                 440                 445

Asn Gly Val Phe Met Cys Ala Glu Gly Thr Gly Lys Phe Cys Pro Leu
    450                 455                 460

Arg Ser Phe Pro Asp Thr Val Tyr Lys Lys Leu Val Gln Arg Glu Lys
465                 470                 475                 480

Thr Leu Lys Val Arg Gly Val Asp Arg Thr Pro Tyr Leu Gly Asp Val
                485                 490                 495

Ala Val Val Val His Pro Gly Lys Lys Glu Met Gly Thr Pro Leu Ala

500                 505                 510

Asp Thr Pro Thr Arg Pro Val Thr Arg His Gly Gly Met Arg Asp Leu
        515                 520                 525

His Glu Ser Ser Phe Ser Leu Ser Gly Ser Gln Ile Asp Asp His Val
    530                 535                 540

Pro Lys Arg Ala Ser Ala Arg Ile Leu Ala Pro Pro Gly Gly Arg Ser
545                 550                 555                 560

Ser Gly Ile Trp

<210>  12
<211>  355
<212>  PRT
<213>  Homo sapiens (Human)

<220>
<221>  SITE
<222>  (1)..(355)
<223>  Vasohibin-2

<400>  12

Met Thr Gly Ser Ala Ala Asp Thr His Arg Cys Pro His Pro Lys Gly
1               5                   10                  15

Ala Lys Gly Thr Arg Ser Arg Ser Ser His Ala Arg Pro Val Ser Leu
            20                  25                  30

Ala Thr Ser Gly Gly Ser Glu Glu Glu Asp Lys Asp Gly Gly Val Leu
        35                  40                  45

Phe His Val Asn Lys Ser Gly Phe Pro Ile Asp Ser His Thr Trp Glu
    50                  55                  60

Arg Met Trp Met His Val Ala Lys Val His Pro Lys Gly Gly Glu Met
65                  70                  75                  80

Val Gly Ala Ile Arg Asn Ala Ala Phe Leu Ala Lys Pro Ser Ile Pro
                85                  90                  95

Gln Val Pro Asn Tyr Arg Leu Ser Met Thr Ile Pro Asp Trp Leu Gln
            100                 105                 110

Ala Ile Gln Asn Tyr Met Lys Thr Leu Gln Tyr Asn His Thr Gly Thr
        115                 120                 125

Gln Phe Phe Glu Ile Arg Lys Met Arg Pro Leu Ser Gly Leu Met Glu
    130                 135                 140

Thr Ala Lys Glu Met Thr Arg Glu Ser Leu Pro Ile Lys Cys Leu Glu
145                 150                 155                 160

Ala Val Ile Leu Gly Ile Tyr Leu Thr Asn Gly Gln Pro Ser Ile Glu
                165                 170                 175

Arg Phe Pro Ile Ser Phe Lys Thr Tyr Phe Ser Gly Asn Tyr Phe His
            180                 185                 190

His Val Val Leu Gly Ile Tyr Cys Asn Gly Arg Tyr Gly Ser Leu Gly
        195                 200                 205

Met Ser Arg Arg Ala Glu Leu Met Asp Lys Pro Leu Thr Phe Arg Thr
    210                 215                 220

Leu Ser Asp Leu Ile Phe Asp Phe Glu Asp Ser Tyr Lys Lys Tyr Leu
225                 230                 235                 240

His Thr Val Lys Lys Val Lys Ile Gly Leu Tyr Val Pro His Glu Pro
                245                 250                 255

His Ser Phe Gln Pro Ile Glu Trp Lys Gln Leu Val Leu Asn Val Ser
            260                 265                 270

Lys Met Leu Arg Ala Asp Ile Arg Lys Glu Leu Glu Lys Tyr Ala Arg
        275                 280                 285

Asp Met Arg Met Lys Ile Leu Lys Pro Ala Ser Ala His Ser Pro Thr
    290                 295                 300

Gln Val Arg Ser Arg Gly Lys Ser Leu Ser Pro Arg Arg Arg Gln Ala
305                 310                 315                 320

Ser Pro Pro Arg Arg Leu Gly Arg Arg Glu Lys Ser Pro Ala Leu Pro
                325                 330                 335

Glu Lys Lys Val Ala Asp Leu Ser Thr Leu Asn Glu Val Gly Tyr Gln
            340                 345                 350

Ile Arg Ile
        355

<210>  13
<211>  66

<212>  PRT
<213>  Homo sapiens (Human)

<220>
<221>  SITE
<222>  (1)..(66)
<223>  Small vasohibin-binding protein

<400>  13

Met Asp Pro Pro Ala Arg Lys Glu Lys Thr Lys Val Lys Glu Ser Val
1               5                   10                  15

Ser Arg Val Glu Lys Ala Lys Gln Lys Ser Ala Gln Gln Glu Leu Lys
            20                  25                  30

Gln Arg Gln Arg Ala Glu Ile Tyr Ala Leu Asn Arg Val Met Thr Glu
        35                  40                  45

Leu Glu Gln Gln Gln Phe Asp Glu Phe Cys Lys Gln Met Gln Pro Pro
    50                  55                  60

Gly Glu
65

<210>  14
<211>  487
<212>  PRT
<213>  Trypanosoma brucei

<220>
<221>  SITE
<222>  (1)..(487)
<223>  Uncharacterized protein / VASH

<400>  14

Met Tyr Ser Ser Asp Arg Val Arg Thr Val Met Glu Gln Leu Ala Ser
1               5                   10                  15

Met Asn Pro Tyr Thr Asn Ala Lys Ser Leu Pro Glu Val Pro Arg Pro
            20                  25                  30

Asp Glu Leu Ser Ile Pro Arg Lys Leu Ser Ser Arg Leu Gln Ile Pro
        35                  40                  45

Tyr Val Gln Asn Ile Leu Asn Ser Leu Ser Tyr Asn Phe Leu Pro Asn
    50                  55                  60

Thr Phe Phe Cys Leu Glu Lys His Arg Ser Leu Gln Ser Ile Leu Phe
65                  70                  75                  80

Thr Ser Lys Glu Ile Leu Ala Glu Ala Leu Pro Ile Arg Cys Leu Glu
                85                  90                  95

Ala Thr Phe Val Gly Leu Tyr Leu Thr Gln Asp Leu Lys Asp Val Asp
            100                 105                 110

Arg Ile Pro Leu Ser Phe Lys Ser Arg Ala Lys Gly Arg Ala Tyr His
        115                 120                 125

His Ile Val Leu Val Ile Arg Cys Asn Ser Met Tyr Gly Ala Val Gly
    130                 135                 140

Leu Ser Arg Lys Ser Thr Leu Met Asp Lys Pro Leu Ile Tyr Asn Gly
145                 150                 155                 160

Leu Tyr Gln Leu Ile Met Glu Tyr Lys Lys Gln Tyr Glu Ala Ile Gly
                165                 170                 175

His Glu Leu Val Asp Phe Lys Leu Gly Leu Cys Val Asn His Lys Arg
            180                 185                 190

Asp Ser Arg Lys Ala Pro Cys Trp Arg Tyr Ile Ala Val Lys Leu Gly
        195                 200                 205

Arg Ser Thr Ala Pro Ser Thr Ser Thr Ser Pro Arg Pro Gln Glu Gln
    210                 215                 220

Gln Glu Glu Val Ser Thr Asn Glu Val Asp Val Ser Leu Ile Ser Asn
225                 230                 235                 240

Ser Gly Gly Cys Ser Cys Gly Asp Gly Ile Glu Lys Met Leu Lys Cys
                245                 250                 255

Tyr Ser Asp Leu Leu Cys Arg Leu Ser Glu Glu Tyr Asp Asn Leu Leu
            260                 265                 270

Thr Ser Tyr Ser Arg Gly Ala Ser Asn Asn Asn Gln Gln Val Cys Leu
        275                 280                 285

Lys Asp Leu His Asp Met Asp Asn Ser Ser Ala Lys Val Glu Asn Arg
    290                 295                 300

Arg Arg Leu Glu Glu Leu Arg Gln Gly Arg Ser Pro Cys Gly Ser Val
305                 310                 315                 320

Gly Thr Arg Cys Lys Lys Arg Ser Ala Ser Arg Ser Val Ser Lys Lys
                325                 330                 335

Arg Ala Val Ser Gln Asn Asp Val Pro Ser Thr Asp Arg Ser Arg Val
            340                 345                 350

Val Arg Leu Ser Ser Ala Val Ala Thr Lys Lys Thr Ala Arg Arg Glu
        355                 360                 365

Lys Ala Ala Ala Ala Asn Tyr Arg Gln Pro Ala Ala Ser Asp Leu Lys
    370                 375                 380

Gln Pro Arg Glu Phe Ser Ser Ala Ser Gly Glu Lys Gly Asp Gly Gln
385                 390                 395                 400

Leu Pro His Val Ser Arg Gly Thr Asn Gly Asn Ile Ser Pro Glu Arg
                405                 410                 415

Ala Cys Arg Ala Ala Glu Glu Gly Cys Ala Ala Asn Pro Gly Glu Glu
            420                 425                 430

Val Thr Gly Glu Arg Gln Ser Val Arg Asn Pro Leu Leu Ser Leu Ala
        435                 440                 445

Leu Pro Leu Leu Asp Ser Lys Gly Gly Glu Leu Gly Leu Thr Thr Cys
    450                 455                 460

Gln Ala Thr Asp Arg Trp Met Leu Pro Asn Ser Ala Lys Gly Ala Asn
465                 470                 475                 480

Ile Asn Asp Val Gly Arg Ser
                485

<210> 15
<211> 154
<212> PRT
<213> Artificial

<220>
<223> Telokin / Homo sapiens

<400> 15

Met Ala Met Ile Ser Gly Leu Ser Gly Arg Lys Ser Ser Thr Gly Ser
1               5                   10                  15

Pro Thr Ser Pro Leu Asn Ala Glu Lys Leu Glu Ser Glu Glu Asp Val
            20                  25                  30

Ser Gln Ala Phe Leu Glu Ala Val Ala Glu Glu Lys Pro His Val Lys
        35                  40                  45

Pro Tyr Phe Ser Lys Thr Ile Arg Asp Leu Glu Val Val Glu Gly Ser
    50                  55                  60

Ala Ala Arg Phe Asp Cys Lys Ile Glu Gly Tyr Pro Asp Pro Glu Val
65                  70                  75                  80

Val Trp Phe Lys Asp Asp Gln Ser Ile Arg Glu Ser Arg His Phe Gln
                85                  90                  95

Ile Asp Tyr Asp Glu Asp Gly Asn Cys Ser Leu Ile Ile Ser Asp Val
            100                 105                 110

Cys Gly Asp Asp Asp Ala Lys Tyr Thr Cys Lys Ala Val Asn Ser Leu
        115                 120                 125

Gly Glu Ala Thr Cys Thr Ala Glu Leu Ile Val Glu Thr Met Glu Glu
    130                 135                 140

Gly Glu Gly Glu Gly Glu Glu Glu Glu Glu
145                 150

<210>  16
<211>  159
<212>  PRT
<213>  Artificial

<220>
<223>  Telokin/ Mus musculus

<400>  16

Met Ala Met Ile Ser Gly Leu Ser Gly Arg Lys Ser Ser Thr Gly Ser
1               5                   10                  15

Pro Thr Ser Pro Ile Asn Ala Glu Lys Leu Glu Ser Glu Asp Asp Val
            20                  25                  30

Ser Gln Ala Phe Leu Glu Ala Val Ala Glu Glu Lys Pro His Val Lys
        35                  40                  45

Pro Tyr Phe Ser Lys Thr Ile Arg Asp Leu Glu Val Val Glu Gly Ser
    50                  55                  60

Ala Ala Arg Phe Asp Cys Lys Ile Glu Gly Tyr Pro Asp Pro Glu Val
65                  70                  75                  80

Val Trp Phe Lys Asp Asp Gln Ser Ile Arg Glu Ser Arg His Phe Gln
                85                  90                  95

Ile Asp Tyr Asp Glu Asp Gly Asn Cys Ser Leu Ile Ile Ser Asp Val
            100                 105                 110

Cys Gly Asp Asp Asp Ala Lys Tyr Thr Cys Lys Ala Val Asn Ser Leu
        115                 120                 125

Gly Glu Ala Thr Cys Thr Ala Glu Leu Ile Val Glu Thr Met Glu Glu
    130                 135                 140

Gly Glu Gly Glu Glu Gly Gly Glu Glu Glu Glu Glu Glu Glu Glu
145                 150                 155

<210>  17
<211>  154
<212>  PRT
<213>  Artificial

<220>
<223>  Fully tyrosinated variant Telokin/Mus musculus

<400>  17

Met Ala Met Ile Ser Gly Leu Ser Gly Arg Lys Ser Ser Thr Gly Ser
1               5                   10                  15

Pro Thr Ser Pro Ile Asn Ala Glu Lys Leu Glu Ser Glu Asp Asp Val
            20                  25                  30

Ser Gln Ala Phe Leu Glu Ala Val Ala Glu Glu Lys Pro His Val Lys
        35                  40                  45

Pro Tyr Phe Ser Lys Thr Ile Arg Asp Leu Glu Val Val Glu Gly Ser
    50                  55                  60

Ala Ala Arg Phe Asp Cys Lys Ile Glu Gly Tyr Pro Asp Pro Glu Val
65                  70                  75                  80

Val Trp Phe Lys Asp Asp Gln Ser Ile Arg Glu Ser Arg His Phe Gln
                85                  90                  95

Ile Asp Tyr Asp Glu Asp Gly Asn Cys Thr Leu Ile Ile Ser Asp Val
            100                 105                 110

Cys Gly Asp Asp Asp Ala Lys Tyr Thr Cys Lys Ala Val Asn Ser Leu
        115                 120                 125

Gly Glu Ala Thr Cys Thr Ala Glu Leu Ile Val Glu Thr Met Glu Glu
    130                 135                 140

Gly Glu Gly Glu Glu Glu Gly Glu Glu Tyr

145                 150

<210>  18
<211>  153
<212>  PRT
<213>  Artificial

<220>
<223>  Detyrosinated (D1) variant Telokin/Mus musculus

<400>  18

Met Ala Met Ile Ser Gly Leu Ser Gly Arg Lys Ser Ser Thr Gly Ser
1               5                   10                  15

Pro Thr Ser Pro Ile Asn Ala Glu Lys Leu Glu Ser Glu Asp Asp Val
            20                  25                  30

Ser Gln Ala Phe Leu Glu Ala Val Ala Glu Glu Lys Pro His Val Lys
        35                  40                  45

Pro Tyr Phe Ser Lys Thr Ile Arg Asp Leu Glu Val Val Glu Gly Ser
    50                  55                  60

Ala Ala Arg Phe Asp Cys Lys Ile Glu Gly Tyr Pro Asp Pro Glu Val
65                  70                  75                  80

Val Trp Phe Lys Asp Asp Gln Ser Ile Arg Glu Ser Arg His Phe Gln
                85                  90                  95

Ile Asp Tyr Asp Glu Asp Gly Asn Cys Thr Leu Ile Ile Ser Asp Val
            100                 105                 110

Cys Gly Asp Asp Asp Ala Lys Tyr Thr Cys Lys Ala Val Asn Ser Leu
        115                 120                 125

Gly Glu Ala Thr Cys Thr Ala Glu Leu Ile Val Glu Thr Met Glu Glu
    130                 135                 140

Gly Glu Gly Glu Glu Glu Gly Glu Glu
145                 150

<210>  19
<211>  451
<212>  PRT
<213>  Homo sapiens (Human)

<220>
<221>  SITE
<222>  (1)..(451)
<223>  Tubulin alpha-1A chain

<400>  19

<210>  20
<211>  451
<212>  PRT
<213>  Homo sapiens (Human)

<220>

<221>  SITE
<222>  (1)..(451)
<223>  Tubulin alpha-1B chain

<400>  20

<210>  21
<211>  449
<212>  PRT
<213>  Homo sapiens (Human)

<220>
<221>  SITE
<222>  (1)..(449)
<223>  Tubulin alpha-1C chain

<400>  21

Met Arg Glu Cys Ile Ser Ile His Val Gly Gln Ala Gly Val Gln Ile
1               5                   10                  15

Gly Asn Ala Cys Trp Glu Leu Tyr Cys Leu Glu His Gly Ile Gln Pro
            20                  25                  30

Asp Gly Gln Met Pro Ser Asp Lys Thr Ile Gly Gly Gly Asp Asp Ser
        35                  40                  45

Phe Asn Thr Phe Phe Ser Glu Thr Gly Ala Gly Lys His Val Pro Arg
    50                  55                  60

Ala Val Phe Val Asp Leu Glu Pro Thr Val Ile Asp Glu Val Arg Thr
65                  70                  75                  80

Gly Thr Tyr Arg Gln Leu Phe His Pro Glu Gln Leu Ile Thr Gly Lys
                85                  90                  95

Glu Asp Ala Ala Asn Asn Tyr Ala Arg Gly His Tyr Thr Ile Gly Lys
            100                 105                 110

Glu Ile Ile Asp Leu Val Leu Asp Arg Ile Arg Lys Leu Ala Asp Gln
        115                 120                 125

Cys Thr Gly Leu Gln Gly Phe Leu Val Phe His Ser Phe Gly Gly Gly
    130                 135                 140

Thr Gly Ser Gly Phe Thr Ser Leu Leu Met Glu Arg Leu Ser Val Asp
145                 150                 155                 160

Tyr Gly Lys Lys Ser Lys Leu Glu Phe Ser Ile Tyr Pro Ala Pro Gln
                165                 170                 175

Val Ser Thr Ala Val Val Glu Pro Tyr Asn Ser Ile Leu Thr Thr His
            180                 185                 190

Thr Thr Leu Glu His Ser Asp Cys Ala Phe Met Val Asp Asn Glu Ala
        195                 200                 205

Ile Tyr Asp Ile Cys Arg Arg Asn Leu Asp Ile Glu Arg Pro Thr Tyr
    210                 215                 220

Thr Asn Leu Asn Arg Leu Ile Ser Gln Ile Val Ser Ser Ile Thr Ala
225                 230                 235                 240

Ser Leu Arg Phe Asp Gly Ala Leu Asn Val Asp Leu Thr Glu Phe Gln
                245                 250                 255

Thr Asn Leu Val Pro Tyr Pro Arg Ile His Phe Pro Leu Ala Thr Tyr
            260                 265                 270

Ala Pro Val Ile Ser Ala Glu Lys Ala Tyr His Glu Gln Leu Thr Val
        275                 280                 285

Ala Glu Ile Thr Asn Ala Cys Phe Glu Pro Ala Asn Gln Met Val Lys
    290                 295                 300

Cys Asp Pro Arg His Gly Lys Tyr Met Ala Cys Cys Leu Leu Tyr Arg
305                 310                 315                 320

Gly Asp Val Val Pro Lys Asp Val Asn Ala Ala Ile Ala Thr Ile Lys
                325                 330                 335

Thr Lys Arg Thr Ile Gln Phe Val Asp Trp Cys Pro Thr Gly Phe Lys
            340                 345                 350

Val Gly Ile Asn Tyr Gln Pro Pro Thr Val Val Pro Gly Gly Asp Leu
        355                 360                 365

Ala Lys Val Gln Arg Ala Val Cys Met Leu Ser Asn Thr Thr Ala Val
    370                 375                 380

Ala Glu Ala Trp Ala Arg Leu Asp His Lys Phe Asp Leu Met Tyr Ala
385                 390                 395                 400

Lys Arg Ala Phe Val His Trp Tyr Val Gly Glu Gly Met Glu Glu Gly
                405                 410                 415

Glu Phe Ser Glu Ala Arg Glu Asp Met Ala Ala Leu Glu Lys Asp Tyr
            420                 425                 430

Glu Glu Val Gly Ala Asp Ser Ala Asp Gly Glu Asp Glu Gly Glu Glu
        435                 440                 445

Tyr

<210>  22

<211>  450
<212>  PRT
<213>  Homo sapiens (Human)

<220>
<221>  SITE
<222>  (1)..(450)
<223>  Tubulin alpha-3C/D chain

<400>  22

Met Arg Glu Cys Ile Ser Ile His Val Gly Gln Ala Gly Val Gln Ile
1               5                   10                  15

Gly Asn Ala Cys Trp Glu Leu Tyr Cys Leu Glu His Gly Ile Gln Pro
            20                  25                  30

Asp Gly Gln Met Pro Ser Asp Lys Thr Ile Gly Gly Gly Asp Asp Ser
        35                  40                  45

Phe Asn Thr Phe Phe Ser Glu Thr Gly Ala Gly Lys His Val Pro Arg
    50                  55                  60

Ala Val Phe Val Asp Leu Glu Pro Thr Val Val Asp Glu Val Arg Thr
65                  70                  75                  80

Gly Thr Tyr Arg Gln Leu Phe His Pro Glu Gln Leu Ile Thr Gly Lys
                85                  90                  95

Glu Asp Ala Ala Asn Asn Tyr Ala Arg Gly His Tyr Thr Ile Gly Lys
            100                 105                 110

Glu Ile Val Asp Leu Val Leu Asp Arg Ile Arg Lys Leu Ala Asp Leu
        115                 120                 125

Cys Thr Gly Leu Gln Gly Phe Leu Ile Phe His Ser Phe Gly Gly Gly
    130                 135                 140

Thr Gly Ser Gly Phe Ala Ser Leu Leu Met Glu Arg Leu Ser Val Asp
145                 150                 155                 160

Tyr Gly Lys Lys Ser Lys Leu Glu Phe Ala Ile Tyr Pro Ala Pro Gln
                165                 170                 175

Val Ser Thr Ala Val Val Glu Pro Tyr Asn Ser Ile Leu Thr Thr His
            180                 185                 190

Thr Thr Leu Glu His Ser Asp Cys Ala Phe Met Val Asp Asn Glu Ala
        195                 200                 205

Ile Tyr Asp Ile Cys Arg Arg Asn Leu Asp Ile Glu Arg Pro Thr Tyr
    210                 215                 220

Thr Asn Leu Asn Arg Leu Ile Gly Gln Ile Val Ser Ser Ile Thr Ala
225                 230                 235                 240

Ser Leu Arg Phe Asp Gly Ala Leu Asn Val Asp Leu Thr Glu Phe Gln
                245                 250                 255

Thr Asn Leu Val Pro Tyr Pro Arg Ile His Phe Pro Leu Ala Thr Tyr
            260                 265                 270

Ala Pro Val Ile Ser Ala Glu Lys Ala Tyr His Glu Gln Leu Ser Val
        275                 280                 285

Ala Glu Ile Thr Asn Ala Cys Phe Glu Pro Ala Asn Gln Met Val Lys
    290                 295                 300

Cys Asp Pro Arg His Gly Lys Tyr Met Ala Cys Cys Met Leu Tyr Arg
305                 310                 315                 320

Gly Asp Val Val Pro Lys Asp Val Asn Ala Ala Ile Ala Thr Ile Lys
                325                 330                 335

Thr Lys Arg Thr Ile Gln Phe Val Asp Trp Cys Pro Thr Gly Phe Lys
            340                 345                 350

Val Gly Ile Asn Tyr Gln Pro Pro Thr Val Val Pro Gly Gly Asp Leu
        355                 360                 365

Ala Lys Val Gln Arg Ala Val Cys Met Leu Ser Asn Thr Thr Ala Ile
    370                 375                 380

Ala Glu Ala Trp Ala Arg Leu Asp His Lys Phe Asp Leu Met Tyr Ala
385                 390                 395                 400

Lys Arg Ala Phe Val His Trp Tyr Val Gly Glu Gly Met Glu Glu Gly
                405                 410                 415

Glu Phe Ser Glu Ala Arg Glu Asp Leu Ala Ala Leu Glu Lys Asp Tyr
            420                 425                 430

Glu Glu Val Gly Val Asp Ser Val Glu Ala Glu Ala Glu Glu Gly Glu
        435                 440                 445

Glu Tyr
    450

<210>  23
<211>  450
<212>  PRT
<213>  Homo sapiens (Human)

<220>
<221>  SITE
<222>  (1)..(450)
<223>  Tubulin alpha-3E chain

<400>  23

Met Arg Glu Cys Ile Ser Ile His Val Gly Gln Ala Gly Val Gln Ile
1               5                   10                  15

Gly Asn Ala Cys Trp Glu Leu Tyr Cys Leu Glu His Gly Ile Gln Pro
            20                  25                  30

Asp Gly Gln Met Pro Ser Asp Lys Thr Ile Gly Gly Gly Asp Asp Ser
        35                  40                  45

Phe Asn Thr Phe Phe Ser Glu Thr Gly Ala Gly Lys His Val Pro Arg
    50                  55                  60

Ala Val Phe Val Asp Leu Glu Pro Thr Val Val Asp Glu Val Arg Thr
65                  70                  75                  80

Gly Thr Tyr Arg Gln Leu Phe His Pro Glu Gln Leu Ile Thr Gly Lys
                85                  90                  95

Glu Asp Ala Ala Ser Asn Tyr Ala Arg Gly His Tyr Thr Ile Gly Lys
            100                 105                 110

Glu Ile Val Asp Leu Val Leu Asp Arg Ile Arg Lys Leu Ala Asp Leu
        115                 120                 125

Cys Thr Gly Leu Gln Gly Phe Leu Ile Phe His Ser Phe Gly Gly Gly
    130                 135                 140

Thr Gly Ser Gly Phe Ala Ser Leu Leu Met Glu Arg Leu Ser Val Asp
145                 150                 155                 160

Tyr Ser Lys Lys Ser Lys Leu Glu Phe Ala Ile Tyr Pro Ala Pro Gln
                165                 170                 175

Val Ser Thr Ala Val Val Glu Pro Tyr Asn Ser Ile Leu Thr Thr His
            180                 185                 190

Thr Thr Leu Glu His Ser Asp Cys Ala Phe Met Val Asp Asn Glu Ala
        195                 200                 205

Ile Tyr Asp Ile Cys Arg Arg Asn Leu Asp Ile Glu Arg Pro Thr Tyr
    210                 215                 220

Thr Asn Leu Asn Arg Leu Ile Gly Gln Ile Val Ser Ser Ile Thr Ala
225                 230                 235                 240

Ser Leu Arg Phe Asp Gly Ala Leu Asn Val Asp Leu Thr Glu Phe Gln
                245                 250                 255

Thr Asn Leu Val Pro Tyr Pro Arg Ile His Phe Pro Leu Ala Thr Tyr
            260                 265                 270

Ala Pro Val Ile Ser Ala Glu Lys Ala Tyr His Glu Gln Leu Ser Val
        275                 280                 285

Ala Glu Ile Thr Asn Ala Cys Phe Glu Pro Ala Asn Gln Met Val Lys
    290                 295                 300

Cys Asp Pro Arg His Gly Lys Tyr Met Ala Cys Cys Met Leu Tyr Arg
305                 310                 315                 320

Gly Asp Val Val Pro Lys Asp Val Asn Ala Ala Ile Ala Thr Ile Lys
                325                 330                 335

Thr Lys Arg Thr Ile Gln Phe Val Asp Trp Cys Pro Thr Gly Phe Lys
            340                 345                 350

Val Gly Ile Asn Tyr Gln Pro Pro Thr Val Val Pro Gly Gly Asp Leu
        355                 360                 365

Ala Lys Val Gln Arg Ala Val Cys Met Leu Ser Asn Thr Thr Ala Ile
    370                 375                 380

Ala Glu Ala Trp Ala Arg Leu Val His Lys Phe Asp Leu Met Tyr Ala
385                 390                 395                 400

Lys Trp Ala Phe Val His Trp Tyr Val Gly Glu Gly Met Glu Glu Gly
                405                 410                 415

Glu Phe Ser Glu Ala Arg Glu Asp Leu Ala Ala Leu Glu Lys Asp Cys
            420                 425                 430

Glu Glu Val Gly Val Asp Ser Val Glu Ala Glu Ala Glu Glu Gly Glu
        435                 440                 445

Ala Tyr
    450

<210>  24
<211>  449
<212>  PRT
<213>  Homo sapiens (Human)

<220>
<221>  SITE
<222>  (1)..(449)
<223>  Tubulin alpha-8 chain

<400>  24

Met Arg Glu Cys Ile Ser Val His Val Gly Gln Ala Gly Val Gln Ile
1               5                   10                  15

Gly Asn Ala Cys Trp Glu Leu Phe Cys Leu Glu His Gly Ile Gln Ala
            20                  25                  30

Asp Gly Thr Phe Asp Ala Gln Ala Ser Lys Ile Asn Asp Asp Asp Ser
        35                  40                  45

Phe Thr Thr Phe Phe Ser Glu Thr Gly Asn Gly Lys His Val Pro Arg
    50                  55                  60

Ala Val Met Ile Asp Leu Glu Pro Thr Val Val Asp Glu Val Arg Ala
65                  70                  75                  80

Gly Thr Tyr Arg Gln Leu Phe His Pro Glu Gln Leu Ile Thr Gly Lys
                85                  90                  95

Glu Asp Ala Ala Asn Asn Tyr Ala Arg Gly His Tyr Thr Val Gly Lys
            100                 105                 110

Glu Ser Ile Asp Leu Val Leu Asp Arg Ile Arg Lys Leu Thr Asp Ala
        115                 120                 125

Cys Ser Gly Leu Gln Gly Phe Leu Ile Phe His Ser Phe Gly Gly Gly
    130                 135                 140

Thr Gly Ser Gly Phe Thr Ser Leu Leu Met Glu Arg Leu Ser Leu Asp
145                 150                 155                 160

Tyr Gly Lys Lys Ser Lys Leu Glu Phe Ala Ile Tyr Pro Ala Pro Gln
                165                 170                 175

Val Ser Thr Ala Val Val Glu Pro Tyr Asn Ser Ile Leu Thr Thr His

180                 185                 190

Thr Thr Leu Glu His Ser Asp Cys Ala Phe Met Val Asp Asn Glu Ala
        195                 200                 205

Ile Tyr Asp Ile Cys Arg Arg Asn Leu Asp Ile Glu Arg Pro Thr Tyr
    210                 215                 220

Thr Asn Leu Asn Arg Leu Ile Ser Gln Ile Val Ser Ser Ile Thr Ala
225                 230                 235                 240

Ser Leu Arg Phe Asp Gly Ala Leu Asn Val Asp Leu Thr Glu Phe Gln
                245                 250                 255

Thr Asn Leu Val Pro Tyr Pro Arg Ile His Phe Pro Leu Val Thr Tyr
            260                 265                 270

Ala Pro Ile Ile Ser Ala Glu Lys Ala Tyr His Glu Gln Leu Ser Val
        275                 280                 285

Ala Glu Ile Thr Ser Ser Cys Phe Glu Pro Asn Ser Gln Met Val Lys
    290                 295                 300

Cys Asp Pro Arg His Gly Lys Tyr Met Ala Cys Cys Met Leu Tyr Arg
305                 310                 315                 320

Gly Asp Val Val Pro Lys Asp Val Asn Val Ala Ile Ala Ala Ile Lys
                325                 330                 335

Thr Lys Arg Thr Ile Gln Phe Val Asp Trp Cys Pro Thr Gly Phe Lys
            340                 345                 350

Val Gly Ile Asn Tyr Gln Pro Pro Thr Val Val Pro Gly Gly Asp Leu
        355                 360                 365

Ala Lys Val Gln Arg Ala Val Cys Met Leu Ser Asn Thr Thr Ala Ile
    370                 375                 380

Ala Glu Ala Trp Ala Arg Leu Asp His Lys Phe Asp Leu Met Tyr Ala
385                 390                 395                 400

Lys Arg Ala Phe Val His Trp Tyr Val Gly Glu Gly Met Glu Glu Gly
                405                 410                 415

Glu Phe Ser Glu Ala Arg Glu Asp Leu Ala Ala Leu Glu Lys Asp Tyr
            420                 425                 430

Glu Glu Val Gly Thr Asp Ser Phe Glu Glu Glu Asn Glu Gly Glu Glu
        435                 440                 445

Phe

<210>  25
<211>  451
<212>  PRT
<213>  Trypanosoma brucei

<220>
<221>  SITE
<222>  (1)..(451)
<223>  Tubulin alpha chain

<400>  25

Met Arg Glu Ala Ile Cys Ile His Ile Gly Gln Ala Gly Cys Gln Val
1               5                   10                  15

Gly Asn Ala Cys Trp Glu Leu Phe Cys Leu Glu His Gly Ile Gln Pro
            20                  25                  30

Asp Gly Ala Met Pro Ser Asp Lys Thr Ile Gly Val Glu Asp Asp Ala
        35                  40                  45

Phe Asn Thr Phe Phe Ser Glu Thr Gly Ala Gly Lys His Val Pro Arg
    50                  55                  60

Ala Val Phe Leu Asp Leu Glu Pro Thr Val Val Asp Glu Val Arg Thr
65                  70                  75                  80

Gly Thr Tyr Arg Gln Leu Phe His Pro Glu Gln Leu Ile Ser Gly Lys
                85                  90                  95

Glu Asp Ala Ala Asn Asn Tyr Ala Arg Gly His Tyr Thr Ile Gly Lys
            100                 105                 110

Glu Ile Val Asp Leu Cys Leu Asp Arg Ile Arg Lys Leu Ala Asp Asn
        115                 120                 125

Cys Thr Gly Leu Gln Gly Phe Leu Val Tyr His Ala Val Gly Gly Gly
    130                 135                 140

Thr Gly Ser Gly Leu Gly Ala Leu Leu Leu Glu Arg Leu Ser Val Asp
145                 150                 155                 160

Tyr Gly Lys Lys Ser Lys Leu Gly Tyr Thr Val Tyr Pro Ser Pro Gln
                165                 170                 175

Val Ser Thr Ala Val Val Glu Pro Tyr Asn Ser Val Leu Ser Thr His
        180                 185                 190

Ser Leu Leu Glu His Thr Asp Val Ala Ala Met Leu Asp Asn Glu Ala
        195                 200                 205

Ile Tyr Asp Leu Thr Arg Arg Asn Leu Asp Ile Glu Arg Pro Thr Tyr
    210                 215                 220

Thr Asn Leu Asn Arg Leu Ile Gly Gln Val Val Ser Ser Leu Thr Ala
225                 230                 235                 240

Ser Leu Arg Phe Asp Gly Ala Leu Asn Val Asp Leu Thr Glu Phe Gln
                245                 250                 255

Thr Asn Leu Val Pro Tyr Pro Arg Ile His Phe Val Leu Thr Ser Tyr
            260                 265                 270

Ala Pro Val Ile Ser Ala Glu Lys Ala Tyr His Glu Gln Leu Ser Val
        275                 280                 285

Ser Glu Ile Ser Asn Ala Val Phe Glu Pro Ala Ser Met Met Thr Lys
    290                 295                 300

Cys Asp Pro Arg His Gly Lys Tyr Met Ala Cys Cys Leu Met Tyr Arg
305                 310                 315                 320

Gly Asp Val Val Pro Lys Asp Val Asn Ala Ala Val Ala Thr Ile Lys
                325                 330                 335

Thr Lys Arg Thr Ile Gln Phe Val Asp Trp Ser Pro Thr Gly Phe Lys
            340                 345                 350

Cys Gly Ile Asn Tyr Gln Pro Pro Thr Val Val Pro Gly Gly Asp Leu
        355                 360                 365

Ala Lys Val Gln Arg Ala Val Cys Met Ile Ala Asn Ser Thr Ala Ile
    370                 375                 380

Ala Glu Val Phe Ala Arg Ile Asp His Lys Phe Asp Leu Met Tyr Ser
385                 390                 395                 400

Lys Arg Ala Phe Val His Trp Tyr Val Gly Glu Gly Met Glu Glu Gly
                405                 410                 415

Glu Phe Ser Glu Ala Arg Glu Asp Leu Ala Ala Leu Glu Lys Asp Tyr

420                 425                 430

Glu Glu Val Gly Ala Glu Ser Ala Asp Met Asp Gly Glu Glu Asp Val
        435                 440                 445

Glu Glu Tyr
    450

<210>  26
<211>  442
<212>  PRT
<213>  Trypanosoma brucei

<220>
<221>  SITE
<222>  (1)..(442)
<223>  Tubulin beta chain

<400>  26

Met Arg Glu Ile Val Cys Val Gln Ala Gly Gln Cys Gly Asn Gln Ile
1               5                   10                  15

Gly Ser Lys Phe Trp Glu Val Ile Ser Asp Glu His Gly Val Asp Pro
            20                  25                  30

Thr Gly Thr Tyr Gln Gly Asp Ser Asp Leu Gln Leu Glu Arg Ile Asn
        35                  40                  45

Val Tyr Phe Asp Glu Ala Thr Gly Gly Arg Tyr Val Pro Arg Ser Val
    50                  55                  60

Leu Ile Asp Leu Glu Pro Gly Thr Met Asp Ser Val Arg Ala Gly Pro
65                  70                  75                  80

Tyr Gly Gln Ile Phe Arg Pro Asp Asn Phe Ile Phe Gly Gln Ser Gly
                85                  90                  95

Ala Gly Asn Asn Trp Ala Lys Gly His Tyr Thr Glu Gly Ala Glu Leu
            100                 105                 110

Ile Asp Ser Val Leu Asp Val Cys Cys Lys Glu Ala Glu Ser Cys Asp
        115                 120                 125

Cys Leu Gln Gly Phe Gln Ile Cys His Ser Leu Gly Gly Gly Thr Gly
    130                 135                 140

Ser Gly Met Gly Thr Leu Leu Ile Ser Lys Leu Arg Glu Gln Tyr Pro
145                 150                 155                 160

Asp Arg Ile Met Met Thr Phe Ser Ile Ile Pro Ser Pro Lys Val Ser
                165                 170                 175

Asp Thr Val Val Glu Pro Tyr Asn Thr Thr Leu Ser Val His Gln Leu
            180                 185                 190

Val Glu Asn Ser Asp Glu Ser Met Cys Ile Asp Asn Glu Ala Leu Tyr
        195                 200                 205

Asp Ile Cys Phe Arg Thr Leu Lys Leu Thr Thr Pro Thr Phe Gly Asp
    210                 215                 220

Leu Asn His Leu Val Ser Ala Val Val Ser Gly Val Thr Cys Cys Leu
225                 230                 235                 240

Arg Phe Pro Gly Gln Leu Asn Ser Asp Leu Arg Lys Leu Ala Val Asn
                245                 250                 255

Leu Val Pro Phe Pro Arg Leu His Phe Phe Met Met Gly Phe Ala Pro
            260                 265                 270

Leu Thr Ser Arg Gly Ser Gln Gln Tyr Arg Gly Leu Ser Val Pro Glu
        275                 280                 285

Leu Thr Gln Gln Met Phe Asp Ala Lys Asn Met Met Gln Ala Ala Asp
    290                 295                 300

Pro Arg His Gly Arg Tyr Leu Thr Ala Ser Ala Leu Phe Arg Gly Arg
305                 310                 315                 320

Met Ser Thr Lys Glu Val Asp Glu Gln Met Leu Asn Val Gln Asn Lys
                325                 330                 335

Asn Ser Ser Tyr Phe Ile Glu Trp Ile Pro Asn Asn Ile Lys Ser Ser
            340                 345                 350

Val Cys Asp Ile Pro Pro Lys Gly Leu Lys Met Ala Val Thr Phe Ile
        355                 360                 365

Gly Asn Asn Thr Cys Ile Gln Glu Met Phe Arg Arg Val Gly Glu Gln
    370                 375                 380

Phe Thr Leu Met Phe Arg Arg Lys Ala Phe Leu His Trp Tyr Thr Gly
385                 390                 395                 400

Glu Gly Met Asp Glu Met Glu Phe Thr Glu Ala Glu Ser Asn Met Asn
                405                 410                 415

Asp Leu Val Ser Glu Tyr Gln Gln Tyr Gln Asp Ala Thr Ile Glu Glu
            420                 425                 430

Glu Gly Glu Phe Asp Glu Glu Glu Gln Tyr
        435                 440

<210>  27
<211>  453
<212>  PRT
<213>  Toxoplasma gondii

<220>
<221>  SITE
<222>  (1)..(453)
<223>  Tubulin alpha chain

<400>  27

Met Arg Glu Val Ile Ser Ile His Val Gly Gln Ala Gly Ile Gln Ile
1               5                   10                  15

Gly Asn Ala Cys Trp Glu Leu Phe Cys Leu Glu His Gly Ile Gln Pro
            20                  25                  30

Asp Gly Gln Met Pro Ser Asp Lys Thr Ile Gly Gly Gly Asp Asp Ala
        35                  40                  45

Phe Asn Thr Phe Phe Ser Glu Thr Gly Ala Gly Lys His Val Pro Arg
    50                  55                  60

Cys Val Phe Leu Asp Leu Glu Pro Thr Val Val Asp Glu Val Arg Thr
65                  70                  75                  80

Gly Thr Tyr Arg His Leu Phe His Pro Glu Gln Leu Ile Ser Gly Lys
                85                  90                  95

Glu Asp Ala Ala Asn Asn Phe Ala Arg Gly His Tyr Thr Ile Gly Lys
            100                 105                 110

Glu Ile Val Asp Leu Ser Leu Asp Arg Ile Arg Lys Leu Ala Asp Asn
        115                 120                 125

Cys Thr Gly Leu Gln Gly Phe Leu Met Phe Asn Ala Val Gly Gly Gly
    130                 135                 140

Thr Gly Ser Gly Leu Gly Cys Leu Leu Leu Glu Arg Leu Ser Val Asp
145                 150                 155                 160

Tyr Gly Lys Lys Ser Lys Leu Asn Phe Cys Ser Trp Pro Ser Pro Gln
                165                 170                 175

Val Ser Thr Ala Val Val Glu Pro Tyr Asn Ser Val Leu Ser Thr His
            180                 185                 190

Ser Leu Leu Glu His Thr Asp Val Ala Val Met Leu Asp Asn Glu Ala
        195                 200                 205

Ile Tyr Asp Ile Cys Arg Arg Asn Leu Asp Ile Glu Arg Pro Thr Tyr
    210                 215                 220

Thr Asn Leu Asn Arg Leu Ile Ala Gln Val Ile Ser Ser Leu Thr Ala
225                 230                 235                 240

Ser Leu Arg Phe Asp Gly Ala Leu Asn Val Asp Val Thr Glu Phe Gln
                245                 250                 255

Thr Asn Leu Val Pro Tyr Pro Arg Ile His Phe Met Leu Ser Ser Tyr
            260                 265                 270

Ala Pro Ile Ile Ser Ala Glu Lys Ala Tyr His Glu Gln Leu Ser Val
        275                 280                 285

Ala Glu Ile Thr Asn Ser Ala Phe Glu Pro Ala Ser Met Met Ala Lys
    290                 295                 300

Cys Asp Pro Arg His Gly Lys Tyr Met Ala Cys Cys Leu Met Tyr Arg
305                 310                 315                 320

Gly Asp Val Val Pro Lys Asp Val Asn Ala Ala Val Ala Thr Ile Lys
                325                 330                 335

Thr Lys Arg Thr Ile Gln Phe Val Asp Trp Cys Pro Thr Gly Phe Lys
            340                 345                 350

Cys Gly Ile Asn Tyr Gln Pro Pro Thr Val Val Pro Gly Gly Asp Leu
        355                 360                 365

Ala Lys Val Met Arg Ala Val Cys Met Ile Ser Asn Ser Thr Ala Ile
    370                 375                 380

Ala Glu Val Phe Ser Arg Met Asp His Lys Phe Asp Leu Met Tyr Ala
385                 390                 395                 400

Lys Arg Ala Phe Val His Trp Tyr Val Gly Glu Gly Met Glu Glu Gly
                405                 410                 415

Glu Phe Ser Glu Ala Arg Glu Asp Leu Ala Ala Leu Glu Lys Asp Tyr
            420                 425                 430

Glu Glu Val Gly Ile Glu Thr Ala Glu Gly Glu Gly Glu Glu Glu Gly
        435                 440                 445

Tyr Gly Asp Glu Tyr
    450

<210>  28
<211>  450
<212>  PRT
<213>  Spodoptera frugiperda

<220>
<221>  SITE
<222>  (1)..(450)
<223>  Tubulin alpha chain

<400>  28

Met Arg Glu Cys Ile Ser Val His Val Gly Gln Ala Gly Val Gln Ile
1               5                   10                  15

Gly Asn Ala Cys Trp Glu Leu Tyr Cys Leu Glu His Gly Ile Gln Pro
            20                  25                  30

Asp Gly Gln Met Pro Thr Asp Lys Thr Val Gly Gly Gly Asp Asp Ser
        35                  40                  45

Phe Asn Thr Phe Phe Ser Glu Thr Gly Ala Gly Lys His Val Pro Arg
    50                  55                  60

Ala Val Phe Val Asp Leu Glu Pro Thr Val Val Asp Glu Val Arg Thr
65                  70                  75                  80

Gly Thr Tyr Arg Gln Leu Phe His Pro Glu Gln Leu Ile Thr Gly Lys
                85                  90                  95

Glu Asp Ala Ala Asn Asn Tyr Ala Arg Gly His Tyr Thr Ile Gly Lys
            100                 105                 110

Glu Ile Val Asp Leu Val Leu Asp Arg Ile Arg Lys Leu Ala Asp Gln
        115                 120                 125

Cys Thr Gly Leu Gln Gly Phe Leu Ile Phe His Ser Phe Gly Gly Gly
    130                 135                 140

Thr Gly Ser Gly Phe Thr Ser Leu Leu Met Glu Arg Leu Ser Val Asp

145                 150                 155                 160

Tyr Gly Lys Lys Ser Lys Leu Glu Phe Ala Ile His Pro Ala Pro Gln
                165                 170                 175

Val Ser Thr Ala Val Val Glu Pro Tyr Asn Ser Ile Leu Thr Thr His
            180                 185                 190

Thr Thr Leu Glu His Pro Asp Cys Ala Phe Met Val Asp Asn Glu Ala
        195                 200                 205

Ile Tyr Asp Ile Cys Arg Arg Asn Leu Asp Ile Glu Arg Pro Thr Tyr
    210                 215                 220

Thr Asn Leu Asn Arg Leu Ile Gly Gln Ile Val Ser Ser Ile Thr Ala
225                 230                 235                 240

Ser Leu Arg Phe Asp Gly Ala Leu Asn Val Asp Leu Thr Glu Phe Gln
                245                 250                 255

Thr Asn Leu Val Pro Tyr Pro Arg Ile His Phe Pro Leu Val Thr Tyr
            260                 265                 270

Ala Pro Val Ile Ser Thr Glu Lys Ala Tyr His Glu Gln Leu Ser Val
        275                 280                 285

Ala Glu Ile Thr Asn Ala Cys Phe Glu Pro Ala Asn Gln Met Val Lys
    290                 295                 300

Cys Asp Pro Arg His Gly Lys Tyr Met Ala Cys Cys Met Leu Tyr Arg
305                 310                 315                 320

Gly Asp Val Val Pro Lys Asp Val Asn Ala Ala Ile Ala Thr Ile Lys
                325                 330                 335

Thr Lys Arg Thr Ile Gln Phe Val Asp Trp Cys Pro Thr Gly Phe Lys
            340                 345                 350

Val Gly Ile Asn Tyr Gln Pro Pro Thr Val Val Pro Gly Gly Asp Leu
        355                 360                 365

Ala Lys Val Gln Arg Ala Val Cys Met Leu Ser Asn Thr Thr Ala Ile
    370                 375                 380

Ala Glu Ala Trp Ala Arg Leu Asp His Lys Phe Asp Leu Met Tyr Ala
385                 390                 395                 400

Lys Arg Ala Phe Val His Trp Tyr Val Gly Glu Gly Met Glu Glu Gly
                405                 410                 415

Glu Phe Ser Glu Ala Arg Glu Asp Leu Ala Ala Leu Glu Lys Asp Tyr
            420                 425                 430

Glu Glu Val Gly Met Asp Ser Ala Glu Gly Glu Gly Glu Gly Ala Glu
        435                 440                 445

Glu Tyr
    450

<210>  29
<211>  450
<212>  PRT
<213>  Drosophila melanogaster

<220>
<221>  SITE
<222>  (1)..(450)
<223>  Tubulin alpha chain

<400>  29

Met Arg Glu Cys Ile Ser Ile His Val Gly Gln Ala Gly Val Gln Ile
1               5                   10                  15

Gly Asn Ala Cys Trp Glu Leu Tyr Cys Leu Glu His Gly Ile Gln Pro
            20                  25                  30

Asp Gly Gln Met Pro Ser Asp Lys Thr Val Gly Gly Gly Asp Asp Ser
        35                  40                  45

Phe Asn Thr Phe Phe Ser Glu Thr Gly Ala Gly Lys His Val Pro Arg
    50                  55                  60

Ala Val Phe Val Asp Leu Glu Pro Thr Val Val Asp Glu Val Arg Thr
65                  70                  75                  80

Gly Thr Tyr Arg Gln Leu Phe His Pro Glu Gln Leu Ile Thr Gly Lys
                85                  90                  95

Glu Asp Ala Ala Asn Asn Tyr Ala Arg Gly His Tyr Thr Ile Gly Lys
            100                 105                 110

Glu Ile Val Asp Leu Val Leu Asp Arg Ile Arg Lys Leu Ala Asp Gln
        115                 120                 125

Cys Thr Gly Leu Gln Gly Phe Leu Ile Phe His Ser Phe Gly Gly Gly
    130                 135                 140

Thr Gly Ser Gly Phe Thr Ser Leu Leu Met Glu Arg Leu Ser Val Asp
145                 150                 155                 160

Tyr Gly Lys Lys Ser Lys Leu Glu Phe Ala Val Tyr Pro Ala Pro Gln
                165                 170                 175

Val Ser Thr Ala Val Val Glu Pro Tyr Asn Ser Ile Leu Thr Thr His
            180                 185                 190

Thr Thr Leu Glu His Ser Asp Cys Ala Phe Met Val Asp Asn Glu Ala
        195                 200                 205

Ile Tyr Asp Ile Cys Arg Arg Asn Leu Asp Ile Glu Arg Pro Thr Tyr
    210                 215                 220

Thr Asn Leu Asn Arg Leu Ile Gly Gln Ile Val Ser Ser Ile Thr Ala
225                 230                 235                 240

Ser Leu Arg Phe Asp Gly Ala Leu Asn Val Asp Leu Thr Glu Phe Gln
                245                 250                 255

Thr Asn Leu Val Pro Tyr Pro Arg Ile His Phe Pro Leu Val Thr Tyr
            260                 265                 270

Ala Pro Val Ile Ser Ala Glu Lys Ala Tyr His Glu Gln Leu Ser Val
        275                 280                 285

Ala Glu Ile Thr Asn Ala Cys Phe Glu Pro Ala Asn Gln Met Val Lys
    290                 295                 300

Val Asp Pro Arg His Gly Lys Tyr Met Ala Cys Cys Met Leu Tyr Arg
305                 310                 315                 320

Gly Asp Val Val Pro Lys Asp Val Asn Ala Ala Ile Ala Thr Ile Lys
                325                 330                 335

Thr Lys Arg Thr Ile Gln Phe Val Asp Trp Cys Pro Thr Gly Phe Lys
            340                 345                 350

Val Gly Ile Asn Tyr Gln Pro Pro Thr Val Val Pro Gly Gly Asp Leu
        355                 360                 365

Ala Lys Val Gln Arg Ala Val Cys Met Leu Ser Asn Thr Thr Ala Ile
    370                 375                 380

Ala Glu Ala Trp Ala Arg Leu Asp His Lys Phe Asp Leu Met Tyr Ala

385                 390                 395                 400

Lys Arg Ala Phe Val His Trp Tyr Val Gly Glu Gly Met Glu Glu Gly
                405                 410                 415

Glu Phe Ser Glu Ala Arg Glu Asp Leu Ala Ala Leu Glu Lys Asp Tyr
            420                 425                 430

Glu Glu Val Gly Met Asp Ser Gly Asp Gly Glu Gly Glu Gly Ala Glu
        435                 440                 445

Glu Tyr
    450

<210>  30
<211>  268
<212>  PRT
<213>  Homo sapiens (Human)

<220>
<221>  SITE
<222>  (1)..(268)
<223>  Microtubule-associated protein RP/EB family member 1

<400>  30

Met Ala Val Asn Val Tyr Ser Thr Ser Val Thr Ser Asp Asn Leu Ser
1               5                   10                  15

Arg His Asp Met Leu Ala Trp Ile Asn Glu Ser Leu Gln Leu Asn Leu
            20                  25                  30

Thr Lys Ile Glu Gln Leu Cys Ser Gly Ala Ala Tyr Cys Gln Phe Met
        35                  40                  45

Asp Met Leu Phe Pro Gly Ser Ile Ala Leu Lys Lys Val Lys Phe Gln
    50                  55                  60

Ala Lys Leu Glu His Glu Tyr Ile Gln Asn Phe Lys Ile Leu Gln Ala
65                  70                  75                  80

Gly Phe Lys Arg Met Gly Val Asp Lys Ile Ile Pro Val Asp Lys Leu
                85                  90                  95

Val Lys Gly Lys Phe Gln Asp Asn Phe Glu Phe Val Gln Trp Phe Lys
            100                 105                 110

Lys Phe Phe Asp Ala Asn Tyr Asp Gly Lys Asp Tyr Asp Pro Val Ala
        115                 120                 125

Ala Arg Gln Gly Gln Glu Thr Ala Val Ala Pro Ser Leu Val Ala Pro
    130                 135                 140

Ala Leu Asn Lys Pro Lys Lys Pro Leu Thr Ser Ser Ser Ala Ala Pro
145                 150                 155                 160

Gln Arg Pro Ile Ser Thr Gln Arg Thr Ala Ala Ala Pro Lys Ala Gly
                165                 170                 175

Pro Gly Val Val Arg Lys Asn Pro Gly Val Gly Asn Gly Asp Asp Glu
            180                 185                 190

Ala Ala Glu Leu Met Gln Gln Val Asn Val Leu Lys Leu Thr Val Glu
        195                 200                 205

Asp Leu Glu Lys Glu Arg Asp Phe Tyr Phe Gly Lys Leu Arg Asn Ile
    210                 215                 220

Glu Leu Ile Cys Gln Glu Asn Glu Gly Glu Asn Asp Pro Val Leu Gln
225                 230                 235                 240

Arg Ile Val Asp Ile Leu Tyr Ala Thr Asp Glu Gly Phe Val Ile Pro
                245                 250                 255

Asp Glu Gly Gly Pro Gln Glu Glu Gln Glu Glu Tyr
            260                 265

<210>  31
<211>  327
<212>  PRT
<213>  Homo sapiens (Human)

<220>
<221>  SITE
<222>  (1)..(327)
<223>  Microtubule-associated protein RP/EB family member 2

<400>  31

Met Pro Gly Pro Thr Gln Thr Leu Ser Pro Asn Gly Glu Asn Asn Asn
1               5                   10                  15

Asp Ile Ile Gln Asp Asn Asn Gly Thr Ile Ile Pro Phe Arg Lys His
            20                  25                  30

Thr Val Arg Gly Glu Arg Ser Tyr Ser Trp Gly Met Ala Val Asn Val
        35                  40                  45

Tyr Ser Thr Ser Ile Thr Gln Glu Thr Met Ser Arg His Asp Ile Ile

50                  55                  60

Ala Trp Val Asn Asp Ile Val Ser Leu Asn Tyr Thr Lys Val Glu Gln
65                  70                  75                  80

Leu Cys Ser Gly Ala Ala Tyr Cys Gln Phe Met Asp Met Leu Phe Pro
                85                  90                  95

Gly Cys Ile Ser Leu Lys Lys Val Lys Phe Gln Ala Lys Leu Glu His
            100                 105                 110

Glu Tyr Ile His Asn Phe Lys Leu Leu Gln Ala Ser Phe Lys Arg Met
        115                 120                 125

Asn Val Asp Lys Val Ile Pro Val Glu Lys Leu Val Lys Gly Arg Phe
    130                 135                 140

Gln Asp Asn Leu Asp Phe Ile Gln Trp Phe Lys Lys Phe Tyr Asp Ala
145                 150                 155                 160

Asn Tyr Asp Gly Lys Glu Tyr Asp Pro Val Glu Ala Arg Gln Gly Gln
                165                 170                 175

Asp Ala Ile Pro Pro Pro Asp Pro Gly Glu Gln Ile Phe Asn Leu Pro
            180                 185                 190

Lys Lys Ser His His Ala Asn Ser Pro Thr Ala Gly Ala Ala Lys Ser
        195                 200                 205

Ser Pro Ala Ala Lys Pro Gly Ser Thr Pro Ser Arg Pro Ser Ser Ala
    210                 215                 220

Lys Arg Ala Ser Ser Ser Gly Ser Ala Ser Lys Ser Asp Lys Asp Leu
225                 230                 235                 240

Glu Thr Gln Val Ile Gln Leu Asn Glu Gln Val His Ser Leu Lys Leu
                245                 250                 255

Ala Leu Glu Gly Val Glu Lys Glu Arg Asp Phe Tyr Phe Gly Lys Leu
            260                 265                 270

Arg Glu Ile Glu Leu Leu Cys Gln Glu His Gly Gln Glu Asn Asp Asp
        275                 280                 285

Leu Val Gln Arg Leu Met Asp Ile Leu Tyr Ala Ser Glu Glu His Glu
    290                 295                 300

Gly His Thr Glu Glu Pro Glu Ala Glu Glu Gln Ala His Glu Gln Gln
305                 310                 315                 320

Pro Pro Gln Gln Glu Glu Tyr
                325

<210>  32
<211>  281
<212>  PRT
<213>  Homo sapiens (Human)

<220>
<221>  SITE
<222>  (1)..(281)
<223>  Microtubule-associated protein RP/EB family member 3

<400>  32

Met Ala Val Asn Val Tyr Ser Thr Ser Val Thr Ser Glu Asn Leu Ser
1               5                   10                  15

Arg His Asp Met Leu Ala Trp Val Asn Asp Ser Leu His Leu Asn Tyr
            20                  25                  30

Thr Lys Ile Glu Gln Leu Cys Ser Gly Ala Ala Tyr Cys Gln Phe Met
        35                  40                  45

Asp Met Leu Phe Pro Gly Cys Val His Leu Arg Lys Val Lys Phe Gln
    50                  55                  60

Ala Lys Leu Glu His Glu Tyr Ile His Asn Phe Lys Val Leu Gln Ala
65                  70                  75                  80

Ala Phe Lys Lys Met Gly Val Asp Lys Ile Ile Pro Val Glu Lys Leu
                85                  90                  95

Val Lys Gly Lys Phe Gln Asp Asn Phe Glu Phe Ile Gln Trp Phe Lys
            100                 105                 110

Lys Phe Phe Asp Ala Asn Tyr Asp Gly Lys Asp Tyr Asn Pro Leu Leu
        115                 120                 125

Ala Arg Gln Gly Gln Asp Val Ala Pro Pro Pro Asn Pro Gly Asp Gln
    130                 135                 140

Ile Phe Asn Lys Ser Lys Lys Leu Ile Gly Thr Ala Val Pro Gln Arg
145                 150                 155                 160

Thr Ser Pro Thr Gly Pro Lys Asn Met Gln Thr Ser Gly Arg Leu Ser
                165                 170                 175

Asn Val Ala Pro Pro Cys Ile Leu Arg Lys Asn Pro Pro Ser Ala Arg
            180                 185                 190

Asn Gly Gly His Glu Thr Asp Ala Gln Ile Leu Glu Leu Asn Gln Gln
        195                 200                 205

Leu Val Asp Leu Lys Leu Thr Val Asp Gly Leu Glu Lys Glu Arg Asp
    210                 215                 220

Phe Tyr Phe Ser Lys Leu Arg Asp Ile Glu Leu Ile Cys Gln Glu His
225                 230                 235                 240

Glu Ser Glu Asn Ser Pro Val Ile Ser Gly Ile Ile Gly Ile Leu Tyr
                245                 250                 255

Ala Thr Glu Glu Gly Phe Ala Pro Pro Glu Asp Asp Glu Ile Glu Glu
            260                 265                 270

His Gln Gln Glu Asp Gln Asp Glu Tyr
        275                 280

<210>  33
<211>  51
<212>  PRT
<213>  Artificial

<220>
<223>  50 amino acids Last C-ter alpha-tubulin 1A/1B

<400>  33

Lys Arg Ala Phe Val His Trp Tyr Val Gly Glu Gly Met Glu Glu Gly
1               5                   10                  15

Glu Phe Ser Glu Ala Arg Glu Asp Met Ala Ala Leu Glu Lys Asp Tyr
            20                  25                  30

Glu Glu Val Gly Val Asp Ser Val Glu Gly Glu Gly Glu Glu Glu Gly
        35                  40                  45

Glu Glu Tyr
    50

<210>  34
<211>  12
<212>  PRT
<213>  Artificial

<220>
<223>  12 amino acids Last C-ter alpha-tubulin 1A/1B

<400>  34

Val Glu Gly Glu Gly Glu Glu Glu Gly Glu Glu Tyr
1               5                   10


<210>  35
<211>  12
<212>  PRT
<213>  Artificial

<220>
<223>  12 amino acids Last C-ter alpha-tubulin 3C

<400>  35

Ser Val Glu Ala Glu Ala Glu Glu Gly Glu Glu Tyr
1               5                   10


<210>  36
<211>  12
<212>  PRT
<213>  Artificial

<220>
<223>  12 amino acids Last C-ter alpha-tubulin 1C

<400>  36

Asp Ser Ala Asp Gly Glu Asp Glu Gly Glu Glu Tyr
1               5                   10


<210>  37
<211>  12
<212>  PRT
<213>  Artificial

<220>
<223>  12 amino acids Last C-ter alpha-tubulin 3E

<400>  37

Ser Val Glu Ala Glu Ala Glu Glu Gly Glu Ala Tyr
1               5                   10


<210>  38
<211>  8
<212>  PRT
<213>  Artificial

<220>
<223>  8 amino acids last C-ter alpha-tubulin 1A/1B

<400>  38

Gly Glu Glu Glu Gly Glu Glu Tyr
1               5

<210>  39
<211>  11
<212>  PRT
<213>  Artificial

<220>
<223>  11 amino acids  last C-ter alpha-tubulin 1A/1B

<400>  39

Glu Gly Glu Gly Glu Glu Glu Gly Glu Glu Tyr
1               5                   10


<210>  40
<211>  8
<212>  PRT
<213>  Artificial

<220>
<223>  7 amino acids last C-ter alpha tubulin from Spodoptera frugiperda
       without the most C-terminal Y (DeTyr)

<400>  40

Gly Glu Gly Glu Gly Ala Glu Glu
1               5

## Description

### FIELD OF THE INVENTION

The present invention relates to *in vitro* screening assay for identification of TCPase modulating compounds, in particular *in vitro* screening immuno-assay.

### BACKGROUND OF THE INVENTION

Microtubules (MTs) are the major types of cytoskeleton elements. They are formed by heterodimeric polymerization of two globular proteins called α-and β-tubuiin. Within a cell, MTs grow, retract, and as such support numerous cellular processes including intracellular transport (cargo transport), cell motility, cell division, cell morphogenesis and mechanotransduction. Each particular MT function requires the recruitment of a specific set of MT associated proteins (MAPs) and molecular motors, many of which interact specifically with the C-terminal tails of tubulins that protrude from MT surface (Ciferri et al., 2008; Roll-Mecak and Vale 2008 (Nature)). One important way in which MTs are adapted to different functions is by alterations of the tubulin C-terminal tails through posttranslational modifications (PTMs) . Almost half a century ago, detyrosination, i.e. the removal of the C-terminal tyrosine of alpha tubulins resulting in generation of so-called Δ1-tubulin, was the earliest reported tubulin PTMs. This PTM is reversible and addition of tyrosine is carried out by tubulin-tyrosine-ligase (TTL) which is already known for a long time. In contrast, tubulin detyrosinases have just very recently been identified (Aillaud et al 2017), allowing direct functional studies of tubulin detyrosination. The cycle of tyrosination/detyrosination is evolutionary conserved from unicellular early-branching eukaryotes such as Trypanosoma to humans (Preston et al., 1979).

In proliferating animal cells, the rapid MT turnover does not allow detyrosination to accumulate. As a result, the majority of MTs in interphasic cells are composed of tyrosinated tubulin while Δ1-tubulin is present only in a small subset of highly stable mostly perinuclear MTs. During mitosis, Δ1-tubulin is found exclusively in the peripheral regions of half spindles and it appears to be absent from astral fibers. Recent studies have shown that spindle detyrosination plays an important role in chromosome congregation during mitosis and is essential for meiotic drive. In addition, stable MT assemblages such as axonemes, centrioles and basal bodies, contain high levels of Δ1-tubulin. Furthermore, detyrosination has been found to play an essential role in generation of stable MT arrays during differentiation events such as cell polarization, myogenesis, and neurogenesis (Aillaud et al 2017). Thus, in general, detyrosination is associated with stable MTs.

Considering the key role of MTs for various cellular processes, it is not surprising that MT targeting compounds such as paclitaxel (taxol) are among most widely used drugs in anticancer therapy. The chemotherapeutic drug taxol is known to interact within a specific site on β-tubulin and as such stabilise microtubule and impact primarily dividing cells. However, MT-targeting drugs suffer from several drawbacks such as high toxicity and eventual development of drug-resistant cancer cells. For that reason, it is important to continue developing compounds that impact MT function. One novel approach, appears to be the targeting of the enzymes involved in tubulin posttranslational modifications, among which detyrosination seems hitherto to be the most promising venue.

Misregulation of tyrosination/detyrosination cycle of tubulin, frequently observed during cancer progression, is associated with increased tumor aggressiveness (Mialhe et al., 2001 ; Kato et al., 2004). The carboxyl-terminus of α-tubulin may be involved in the control of cell cycle progression (Maihato et al. 2015) and in the association/disassociation of motor proteins during cell division. The spindle-related defects are particularly interesting since several studies have shown that absence or downregulation of TTL activity is associated with cancer progression (Mialhe et al., 2001 ; Kato et al., 2004). Besides, accumulating studies show that microtubule-associated proteins can regulate taxol sensitivity in a wide range of cancer types.

Exploiting the alpha-tubulin sequence as a bait, two closely related proteins of the vasohibin family, VASH1 and VASH2 were identified as tubulin detyrosinases (Aillaud et al 2017; Nieuwenhuis et al., 2017). Interestingly, treatment of HEK293T cells deficient for VASH1 and VASH2 with Taxol, a microtubules stabilising agent, results in a robust increase in alpha-tubulin detyrosination demonstrating a yet unidentified activity (Nieuwenhuis et al., 2017).

The identification of specific modulators of enzymes with TCPase activity is of particular interest for treating disorders involving microtubule detyrosination, such as neurodegenerative diseases, neuronal regeneration disorders, cancers, muscular dystrophies, heart diseases, vascular disorders, retinal degeneration, infertility or ciliopathies. Importantly, the modulators can also be developed to target infectious parasites such as but not limited to *Tryponosoma Brucei, Toxoplasma gondii* and as such be designed to act on the parasite specific detyrosination enzyme and not on the human homolog.

Next to their use in pharmaceutical development, the modulators could also be used as tools to better understand the role of tubulin detyrosination in e.g. cancer occurrence, aggressiveness and progression. Overall, there is a strong need for compounds that may selectively modulate detyrosination mediated by a TCPase.

Detyrosinated tubulin was recently found to be decreased in fetal vessels of preeclampsia placentas. The reduction of placental detyrosination in preeclampsia could suggest a deficit in villous vascular plasticity and might be associated with the impaired arborization of the disease. In this setting, a compound that could activate tubulin carboxypeptidase activity, ie an activator of TCPase, could be of therapeutic interest.

Given the potential wide application of compounds targeting tubulin detyrosinases, there is still a need to develop a new method for *in vitro* screening of such compounds, preferably a high throughput screening (HTS) for detection of such compounds.

The present invention answers this expectation and proposes a method allowing detection of compounds that directly act on tubulin carboxypeptidases, in particular by high throughput screening (HTS). Validated hits can be identified within large chemical libraries and assay may serve lead optimization. The immuno-assay according to the invention is based on specifically generated antibodies raised to detect cleaved substrate of TCPase, in particular detyrosinated substrate of TCPase and identification of easy to produce substrate of TCPase containing a C-terminal tyrosine 'Y' including in particular polymerized microtubules, recombinant alpha-tubulin, engineered telokin protein (Q15746-8) and peptide use. The assay may be miniaturized and adapted to dedicated robotic workstations including microfluidic systems. The screen is reproducible, reliable, robust, and, most importantly, employ techniques that ensure there is reasonable confidence that the results will be biologically relevant.

The method of the present invention can be used for making a kit composed of all reagents necessary to perform the assay on standard plate reader equipment. The assay can easily be implemented in high throughput screening (HTS) methods for hit identification within large chemical libraries and following lead optimization in the pharmaceutical company. Moreover, the method of the present invention can also be used for making a kit composed of all reagents modulators of the activity on various different substrates (such as recombinant alpha-tubulin, purified tubulin dimers from diverse sources such as from *Spodoptera frugiperda* Sf9 cell culture, polymerized microtubule, telokin substrate and more).

### SUMMARY OF THE INVENTION

A first object of the present invention is an *in vitro* screening assay for identification of modulators of tubulin carboxypeptidase (TCPase) activity comprising the steps of:
(i) Contacting (a) a substrate of TCPase enzyme comprising an amino acids sequence having at least the last 4 amino acids residues of the C-terminal sequence of an α-tubulin and/or a Microtubule Associated Protein (MAP) and, as ultimate C-terminal amino acid residue, an aromatic amino acid residue, preferably a tyrosine (Y), and b) an isolated or recombinant TCPase enzyme; in the presence or absence (control) of the modulator compound to be tested, and under conditions for substrate cleavage, preferably detyrosination, and/or liberation of a C-terminal free aromatic amino acid residue, preferably a C-terminal free tyrosine;
(ii) Using reagents for detecting and measuring the signal related to substrate cleavage, preferably detyrosination, and/or liberation of the C-terminal free aromatic amino acid residue, preferably the C-terminal free tyrosine ;
(iii) Measuring and comparing the level of substrate cleavage, preferably detyrosination and/or the level of the C-terminal free aromatic amino acid residue, preferably the C-terminal free tyrosine in presence, and in absence (control) of the compound to be tested, and
(iv) Selecting the modulators of TCPase for which the level of substrate cleavage, preferably detyrosination or liberation of the C-terminal free aromatic amino acid residue, preferably the C-terminal free tyrosine is increased in the presence of the compound to be tested (activators of TCPase) or decreased in the presence of the compound to be tested (inhibitors of TCPase).

In a particular embodiment, the *in vitro* screening assay is an immuno-assay, in particular a dot-blot or an ELISA assay, and more preferably a High Throughput Screening (HTS) assay based on ELISA assay.

Another object of the present invention is a kit for performing the *in vitro* screening immuno-assay, comprising reagents and instructions as disclosed hereunder.

Another object of the invention is a kit for performing the *in vitro* screening assay based on detection of a C-terminal aromatic residue via a colorimetric assay or using fluorescent properties of the aromatic residues, preferably a colorimetric assay using a tyrosinase to detect a C-terminal free tyrosine, comprising reagents and instructions as disclosed hereunder.

### DETAILED DESCRIPTION OF THE INVENTION

### DEFINITIONS

As used herein, the term "contacting" intends bringing the reagents, ie TCPase enzyme and its substrate according to the invention, into close proximity with each other so that a chemical or biochemical reaction can occur among the reagents. In one aspect, the term intends admixing the components, either in a fluid reaction (ex: vessel) or on solid support (ex: plate).

The terms "modulators" of TCPase include any compound that interacts with or which modulates (increases or decreases) the activity of a TCPase or which modulates (increase or decrease) the binding of another compound to a TCPase. This includes agonists, antagonists, and their homologs and mimetics.

Inhibitors are compounds that, e.g., bind to, partially or totally block activity, decrease, prevent, delay activation, inactivate, desensitize, or downregulate TCPase or activity, e.g., antagonists.

Activators or enhancers are compounds that, e.g., bind to, stimulate, increase, open, activate, facilitate, enhance activation, sensitize, or upregulate TCPase or activity, e.g., agonists. TCPase modulators can include genetically modified versions of TCPase family members, e.g., with altered activity, as well as naturally occurring and synthetic ligands, antagonists, aptamers, agonists, small chemical molecules and the like.

In particular, the term 'modulator of TCPase activity' according to the present invention means a compound which directly alters the activity of the TCPase when brought into contact. During the course of the *in vitro* screening assay, the TCPase enzyme is put in contact with the substrate of TCPase in absence or presence of compounds to be tested, and the level of detyrosination is measured, for example by enzyme-linked immunosorbent assay (ELISA) system. In that context, a compound that reduces overall level of detyrosination compared to the control situation (in absence of the compound to be tested) is termed an 'inhibitor'. Oppositely, a compound that increases overall level of detyrosination compared to the control situation is termed an 'activator'.

The term "isolated" is used herein to refer to polypeptides and proteins that are separated from constituents, cellular and otherwise.

The peptides or proteins, described herein, may be isolated, or otherwise associated with structures or compounds to which they are not normally associated in nature, according to a variety of methods and processes known to those of skill in the art.

The term "purified" refers to a composition being substantially free from undesired contamination.

As used herein, "recombinant" refers to a polynucleotide synthesized or otherwise manipulated *in vitro* (e.g., "recombinant polynucleotide"), to methods of using recombinant gene products in cells or other biological systems to produce a polypetide.; the recombinant polynucleotide or recombinant protein may be similar to natural polynucleotide or protein or not.

The term "engineered" refers to a polynucleotide or polypeptide obtained by *in vitro* human manipulations, such as for example insertion of nucleotide sequence into another nucleotide sequence to produce a recombinant engineered polypeptide comprising the both fused sequences.

The term "synthetic" refers to non-natural polynucleotide or polypeptide.

The term "polypeptide" refers to a compound of two or more amino acid subunits, amino acid analogs. The subunits may be linked by peptide bonds. As used herein the term "amino acid" refers to natural and/or unnatural or synthetic amino acids, amino acid analogs. A peptide of three or more amino acids is commonly called an oligopeptide if the peptide chain is short. If the peptide chain is long, the peptide is commonly called a polypeptide or a protein.

The amino acid sequences defined herein use the one letter code as following : A : Ala (alanine) ; R : Arg (arginine) ; N : Asn (asparagine) ; D : Asp (aspartic acid); C : Cys (cysteine) ; Q : Gln (glutamine) ; E : Glu (glutamic acid); G : Gly (glycine); H : His (histidine); I : Ile (isoleucine); L : Leu (leucine); K : Lys (lysine); M : Met (methionine); F : Phe (phenylalanine); P : Pro (proline); S : Ser (serine); T : Thr (threonine); W : Trp (tryptophan); Y : Tyr (tyrosine); V: Val (valine).

The term "aromatic amino acid residue" refers to F : Phe (phenylalanine); W : Trp (tryptophan); Y : Tyr (tyrosine),.

The proteins and peptides according to the invention also encompass "conservative variant" or "analog" which refer to a polypeptide which has a modified amino acid sequence, such that the change(s) do not substantially alter the polypeptide's (the conservative variant's) structure and/or activity, as defined herein. These include conservatively modified variations of an amino acid sequence, i.e., amino acid substitutions, additions or deletions of those residues that are not critical for protein activity, or substitution of amino acids with residues having similar properties (e.g., acidic, basic, positively or negatively charged, polar or non-polar, etc.) such that the substitutions of even critical amino acids does not substantially alter structure and/or activity.

For example we may use the following six groups, each containing amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (I); 5) Isoleucine (I), Leucine (L), Methionine (M), Valine (V); and 6) Phenylalanine (F), Tyrosine (Y), Tryptophan (W). In addition, individual substitutions, deletions or additions that alter, add or delete a single amino acid or a small percentage of amino acids in an encoded sequence can also be considered "conservatively modified variations." Because of the degeneracy of the genetic code, a large number of functionally identical nucleic acids encode any given protein.

In particular, isolated or recombinant peptides from 5 to 50 amino acids (substrate of TCPase) according to the invention also encompass variants having at least 50%, 60%, 70%, 80%, 90%, 95%, 96%, 97%, 98%, 99% of sequence identity with the described amino acids sequences identified in the description as "SEQ ID N°:", with condition that the most C-terminal EEY or EDY or EAY or Y, preferably EEY or Y is 100% identity.

In particular, isolated or recombinant proteins (substrate of TCPase) according to the invention also encompass variants having at least 50%, 60%, 70%, 80%, 90%, 95% of sequence identity with the described amino acids sequences identified in the description as "SEQ ID N°:", with condition that the most C-terminal ends with an aromatic amino acid residue such as tyrosine (Y), phenylalanine (F) or tryptophan (W). In a preferred embodiment, the sequence ends with a C-terminal tyrosine residue.

The term variant thereof' in the rest of the description encompasses the above defined % of identity with the described amino acids sequences identified in the description as "SEQ ID N°:".

The percent identities referred to in the context of the disclosure of the present invention are determined on the basis of a global alignment of sequences to be compared, i.e., on an alignment of the sequences taken in their entirety over their entire length using any algorithm well-known to a person skilled in the art, such as the algorithm of Needleman and Wunsch (1970). This sequence comparison may be performed using any software well-known to a person skilled in the art, for example the Needle software by using the "Gap open" parameter equal to 10.0, the "Gap extend" parameter equal to 0.5 and a "Blosum 62" matrix. The Needle software is for example available on the website ebi.ac.uk under the name "Align".

The term "substrate cleavage" means the proteolysis of the substrate by the TCPase enzyme, in particular the cleavage of the ultimate C-terminal aromatic amino acid residue, preferably the tyrosine (Y) residue (also named "detyrosination').

### IN VITRO SCREENING ASSAY

A first object of the invention is an *in vitro* screening assay for identification of modulators of tubulin carboxypeptidase (TCPase) activity comprising the steps of:
(i) Contacting (a) a substrate of TCPase enzyme comprising an amino acids sequence having at least the last 4 amino acids residues of the C-terminal sequence of an α-tubulin and/or a Microtubule Associated Protein (MAP) and, as ultimate C-terminal amino acid residue, an aromatic amino acid residue, preferably a tyrosine (Y), and b) an isolated or recombinant TCPase enzyme; in the presence or absence (control) of the modulator compound to be tested, and under conditions for substrate cleavage, preferably detyrosination, and/or liberation of a C-terminal free aromatic amino acid residue, preferably a C-terminal free tyrosine;
(ii) Using reagents for detecting and measuring the signal related to substrate cleavage, preferably detyrosination, and/or liberation of a C-terminal free aromatic amino acid residue, preferably a C-terminal free tyrosine ;
(iii) Measuring and comparing the level of substrate cleavage, preferably detyrosination and/or the level of the C-terminal free aromatic amino acid residue, preferably the C-terminal free tyrosine in presence and in absence (control) of the compound to be tested, and
(iv) Selecting the modulators of TCPase for which the level of substrate cleavage, preferably detyrosination or liberation of the C-terminal free aromatic amino acid residue, preferably the C-terminal free tyrosine is increased in the presence of the compound to be tested (activators of TCPase) or decreased in the presence of the compound to be tested (inhibitors of TCPase).

The steps (i) to (iv) of the screening generally follow a sequential order as disclosed above but some of the steps may occur simultaneously or with another order than the one illustrated above.

### Substrates of TCPase enzymes

In a particular embodiment of the invention, the substrate of TCPase enzyme comprising an amino acid sequence having at least the last 4 amino acids residues of the C-terminal sequence of an α-tubulin and/or a Microtubule Associated Protein (MAP) and, as ultimate C-terminal amino acid residue, an aromatic amino acid residue, preferably a tyrosine (Y), is selected from the group consisting of:
a) Isolated or recombinant microtubules,
b) Isolated or recombinant tubulin dimers of alpha and beta tubulin,
c) Microtubule-associated proteins (MAP) such as microtubule-associated proteins RP/EB family members 1 to 3,
d) Purified or recombinant α-tubulin,
e) Recombinant engineered telokin,
f) Isolated or recombinant peptide from 4 to 50 amino acid residues comprising an amino acid sequence having at least the last 4 amino acids residues of the C-terminal sequence of an α-tubulin and/or a Microtubule Associated Protein (MAP) and, as ultimate C-terminal amino acid residue, an aromatic amino acid residue, preferably a tyrosine (Y), or
g) Mixtures thereof.

Non-limitative examples of such TCPase enzyme substrates as defined above and comprising a most C-terminal aromatic amino acid residue, preferably a tyrosine 'Y' and used according to the present invention are disclosed in the following Table 1 :

**Table 1**

| SEQ ID NO : | **Sequence description** | **Origin** | **Uniprot Reference** | **Function** |
|---|---|---|---|---|
| 17 | Fully tyrosinated variant Telokin/Mus musculus | Artificial | Artificial | Engineered Telokin (Uniprot Q6PDN3 -4). The C-terminal sequence of alpha-tubulin (Tubulin alpha-1A chain - human) has been introduced in the Telokin sequence. Fully tyrosinated variant. |
| 19 | Tubulin alpha-1A chain | Homo sapiens | Q71U36 | Tubulin alpha-1A chain. Tubulin is the major constituent of microtubules. It binds two moles of GTP, one at an exchangeable site on the beta chain and one at a non-exchangeable site on the alpha chain. |
| 20 | Tubulin alpha-1B chain | Homo sapiens | P68363 | Tubulin alpha-1B chain. |
| 21 | Tubulin alpha-1C chain | Homo sapiens | Q9BQE3 | Tubulin alpha-1C chain. |
| 22 | Tubulin alpha-3C/D chain | Homo sapiens | Q13748 | Tubulin alpha-3C/D chain. |
| 23 | Tubulin alpha-3E chain | Homo sapiens | Q6PEY2 | Tubulin alpha-3E chain |
| 24 | Tubulin alpha-8 chain | Homo sapiens | Q9NY65 | Tubulin alpha-8 chain |
| 25 | Tubulin alpha chain | Trypanosoma brucei | Q4GYY5 | Tubulin alpha chain |
| 26 | Tubulin beta chain | Trypanosoma brucei | Q4GYY6 | Tubulin beta chain |
| 27 | Tubulin alpha chain | Toxoplasma gondii | P10873 | Tubulin alpha chain |
| 28 | Tubulin alpha chain | Spodoptera frugiperda | G3CKA7 | Tubulin alpha chain. Spodoptera frugiperda. |
| 29 | Tubulin alpha chain | Drosophila melanogaster | P06605 | Tubulin alpha chain |
| 30 | Microtubule-associated protein RP/EB family member 1 | Homo sapiens | Q15691 | Microtubule-associated protein RP/EB family member 1. May be involved in spindle function by stabilizing microtubules and anchoring them at centrosomes |
| 31 | Microtubule-associated protein RP/EB family member 2 | Homo sapiens | Q15555 | Microtubule-associated protein RP/EB family member 2. May be involved in microtubule polymerization, and spindle function by stabilizing microtubules and anchoring them at centrosomes. May play a role in cell migration |
| 32 | Microtubule-associated protein RP/EB family member 3 | Homo sapiens | Q9UPY8 | Microtubule-associated protein RP/EB family member 3 : acts as a regulator of minus-end microtubule organization: interacts with the complex formed by AKAP9 and PDE4DIP, leading to recruit CAMSAP2 to the Golgi apparatus, thereby tethering non-centrosomal minus-end microtubules to the Golgi, an important step for polarized cell movement |
| 33 | 50 amino acids Last C-ter alpha-tubulin 1A/1 B | Artificial | 1A/1B | KRAFVHWYVGEGMEEGE FSEAREDMAAL EKDYEEVGVDSVEGEGE EEGEEY |
| 34 | 12 amino acids Last C-ter alpha-tubulin 1A/1 B | Artificial | 1A/1B | VEGEGEEEGEEY |
| 35 | 12 amino acids Last C-ter alpha-tubulin 3C | Artificial | 3C | SVEAEAEEGEEY |
| 36 | 12 amino acids Last C-ter alpha-tubulin 1C | Artificial | 1C | DSADGEDEGEEY |
| 37 | 12 amino acids Last C-ter alpha-tubulin 3E | Artificial | 3E | SVEAEAEEGEAY |
| 38 | 8 amino acids last C-ter alpha-tubulin 1A/1 B | Artificial | 1A/1B | GEEEGEEY |
| 39 | 11 amino acids last C-ter alpha-tubulin 1A/1 B | Artificial | 1A/1B | EGEGEEEGEEY |

### Recombinant engineered Telokin as preferred substrate (containing C-terminal tubulin sequence)

In a particular and preferred embodiment, the substrate of TCPase is a recombinant engineered telokin having at least the last 4 amino acids residues of the C-terminal sequence of an α-tubulin and a tyrosine (Y) as ultimate C-terminal amino acid residue.

In particular, a recombinant engineered telokin comprises the most C-terminal tail of alpha-tubulin (the 11 most C-terminal residues EGEGEEEGEEY= SEQ ID NO: 39).

Telokin protein (Q15746, SEQ ID NO: 15, *Homo sapiens*) is a small isoform of myosin-light chain kinase protein family. The protein function is involved in calcium/calmodulin-dependent myosin light chain kinase implicated in smooth muscle contraction via phosphorylation of myosin light chains (MLC). The specific Telokin isoform (Q6PDN3-4, SEQ ID NO: 16, *Mus musculus*) is transcribed from an alternative promoter resulting in the usage of Met-1761 as initiator codon. It has no catalytic activity and is highly produced in bacteria. The use of telokin gene to modify it's ending in any potential substrate (human alpha tubulins, trypanosoma alpha and beta tubulin etc.) make it a substrate of choice to adapt by molecular engineering. A recombinant engineered tyrosinated telokin has been obtained as illustrated further in the description (Examples).

In a particular and preferred embodiment, the substrate of TCPase is a recombinant tyrosinated telokin comprising the amino acid sequence SEQ ID NO:17 (Mus musculus, fully tyrosinated variant) or a variant thereof. In another embodiment, the substrate of TCPase is a recombinant telokin comprising *Trypanosoma Brucei* alpha tubulin (SEQ ID NO: 25) or *Trypanosoma Brucei* beta tubulin (SEQ ID NO: 26).

### Additional substrates of TCPase enzyme

As an alternative, the substrate of TCPase according to the invention is selected from isolated or recombinant microtubules (MTs), which are formed by the polymerization of a dimer of two globular proteins, α- and β-tubulin heterodimers.

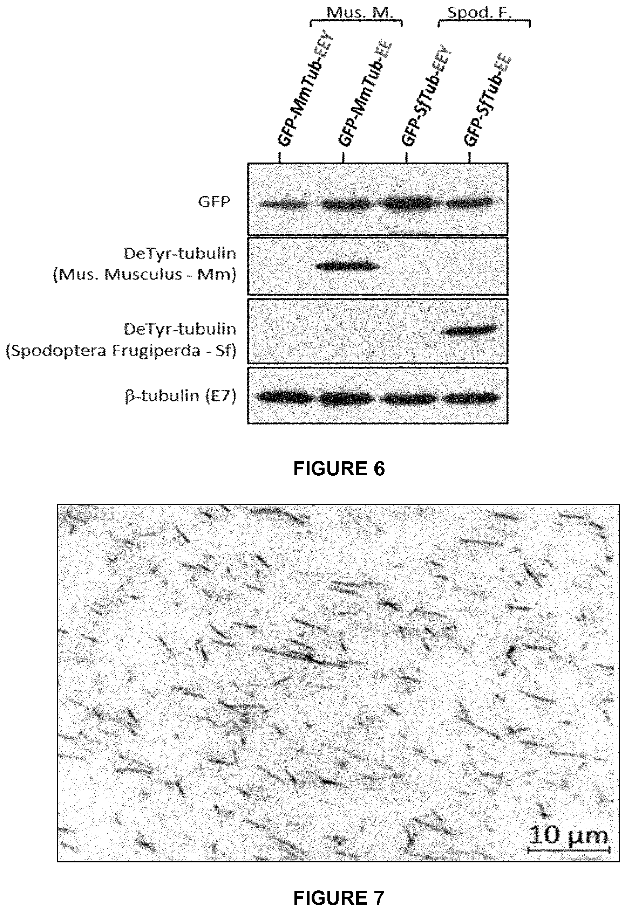

Tubulin dimers can be extracted and purified from any eukaryotic cell system such as human cell lines (HEK, Hela, RPE, MCF-7, etc.), mouse cell-line (C2C12, etc.), *Trypanosoma Brucei, Spodoptera frugiperda* cell line (Sf9, Sf21, etc.), and many more. Obtained tubulin dimers should be stored at -80°C and used for generating microtubules (MTs) with compounds that lower the critical concentration (glycerol, GTP etc.). Tubulin polymerizations were generally carried out in 80 mM PIPES pH 6.9, 0.5 mM EGTA, 2 mM MgCI2, 1 mM GTP, and the reactions occur at 37°C (**Figure 7**).

By 'recombinant microtubules' or 'synthetic microtubules' according to the invention, it means microtubules that are chemically constructed, for example by methods of synthetic biology, including solid phase peptide synthesis (SPPS), prior thiol capture strategy or native chemical ligation (NCL).

As an alternative, the substrate of TCPase according to the invention is selected from isolated or recombinant binding protein, in particular an isolated or recombinant microtubule-associated protein ("MAP proteins") RP/EB from human. In particular, mention may be made of microtubule-associated protein RP/EB family member 1 comprising the amino acid sequence SEQ ID NO:30, microtubule-associated protein RP/EB family member 2 comprising the amino acid sequence SEQ ID NO: 31 or microtubule-associated protein RP/EB family member 3 comprising the amino acid sequence SEQ ID NO: 32, or variants thereof.

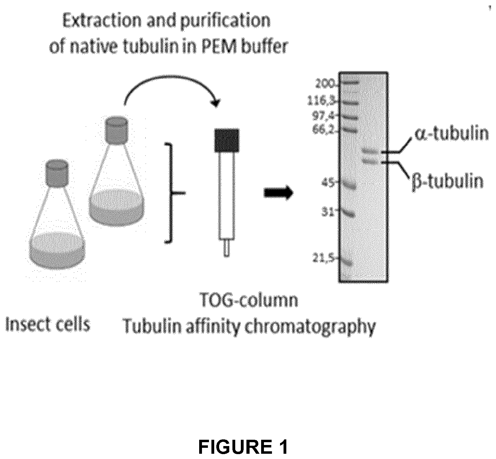

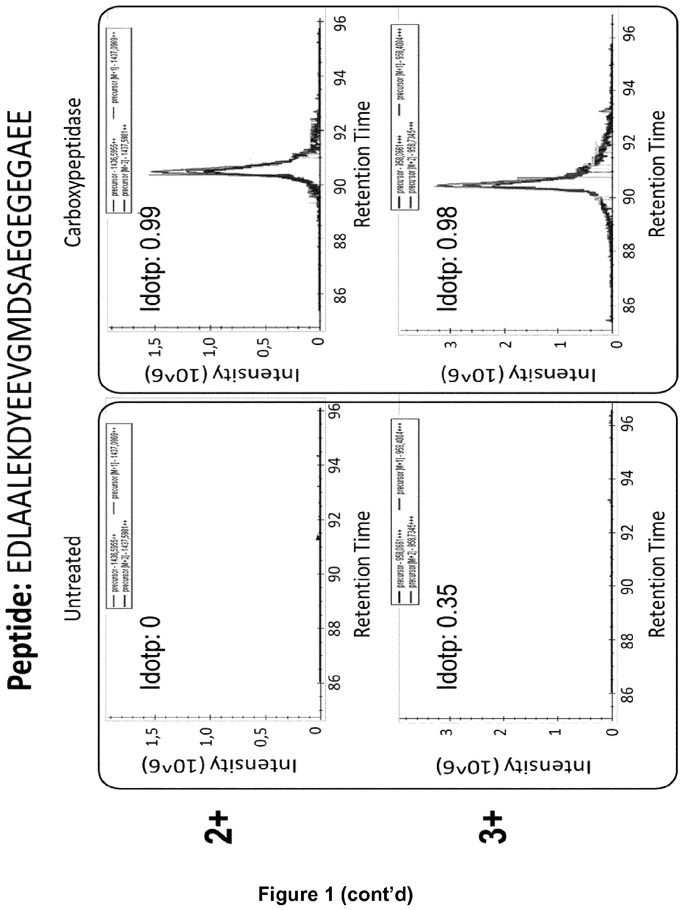

As an alternative, the substrate of TCPase according to the invention is selected from isolated or recombinant tubulin dimers, in particular purified tubulin dimers obtained from any eukaryotic cell containing tubulin, such as human cell lines (HEK, Hela, RPE, MCF-7, etc.), mouse cell-line (C2C12, etc.), *Trypanosoma Brucei, Spodoptera frugiperda* cell line (Sf9, Sf21, etc.).In a first and preferred embodiment, the tubulin dimers are purified tyrosinated tubulin from *Spodoptera Frugiperda* (Sf) cells (Sf9, Sf21 etc.). While searching for a reliable source of tyrosinated tubulin we found that tubulin purified from *Spodoptera Frugiperda* (Sf) cells (Sf9, Sf21 etc.) represented an unlimited source of fully tyrosinated tubulin (**Figure 1**). Tubulin was purified using a TOG column (Widlund et al. 2012 MBoC) and analyzed by mass spectrometry. Analysis of untreated Sf9 purified tubulin by mass spectrometry did not detect any detyrosinated alpha-tubulin. This purified tubulin of *Spodoptera Frugiperda* is polymerization competent and can be used as substrate in *in vitro* detyrosination assays. So the tubulin purified from Sf9 cells represents an unlimited source of polymerization competent fully tyrosinated tubulin.

In a particular embodiment of the invention, the substrate of TCPase is a purified or recombinant tyrosinated tubulin comprising the amino acid sequence of SEQ ID NO:28 (Uniprot G3CKA7, *Spodoptera Frugiperda*) or variant thereof.

In another particular and preferred embodiment, the α-tubulin is a recombinant alpha tubulin (a-tubulin) which can be produced in bacteria according to known methods. In particular, we may use a purified or recombinant α-tubulin from human in particular comprising the amino acid sequences SEQ ID NO: 19 to SEQ ID NO: 24), or from *Trypanosoma brucei* in particular comprising the amino acids sequences SEQ ID NO: 25 and SEQ ID NO: 26, or from *Toxoplasma gondii* in particular comprising the amino acids sequence SEQ ID NO: 27, or from *Drosophila melanogaster* in particular comprising the amino acids sequence (SEQ ID NO: 29).

As another alternative, the substrate of TCPase according to the invention is selected from isolated or recombinant peptides from 4 to 50 amino acid residues, in particular 5 to 20 amino acids, preferably 6 to 12 amino acid residues comprising an amino acid sequence having at least the last 4 amino acids residues of the C-terminal sequence of an α-tubulin and/or a Microtubule Associated Protein (MAP) and, as ultimate C-terminal amino acid residue, an aromatic amino acid residue, preferably a tyrosine (Y), in particular comprising an amino acid sequence selected from SEQ ID NO: 33 to SEQ ID NO: 39 or variant thereof.

In a particular embodiment, the TCPase according to the invention is a peptide GEEEGEEY (SEQ ID NO: 38).

The substrate of TCPase described above may be use alone or in mixture.

In a particular embodiment, the substrate of TCPase enzyme is selected from recombinant telokin comprising a C-terminal sequence of alpha-tubulin, in particular recombinant tyrosinated telokin comprising the amino acid sequence SEQ ID NO: 17 or variant thereof, and recombinant peptides thereof from 5 to 20 amino acids residues, in particular recombinant peptide comprising the amino acid sequence SEQ ID NO: 33 to SEQ ID NO: 39 or variants thereof.

In a particular and preferred embodiment, the substrate of TCPase enzyme is selected from recombinant engineered telokin, in particular comprising an amino acid sequence SEQ ID NO: 17 or variant thereof.

### TCPase enzymes or TCPase proteins

In the context of the present invention, the terms 'TCPase enzyme' or TCPase protein' or protein having a tubulin carboxypeptidase activity' or 'protein having a TCP activity' are used for referring to a class of proteins that are able to cleave off the ultimate C-terminal aromatic amino acid residue, in particular the tyrosine to release the C-terminal aromatic amino acid residue, in particular C-terminal tyrosine residue (Y) from a native or recombinant substrate, in particular a native or recombinant tyrosinated tubulin or preferably a recombinant engineered tyrosinated telokin.

In the rest of the description, we will use the terms 'substrate cleavage' or in particular 'detyrosination', and the terms 'cleaved substrate' or in particular 'detyrosinated substrate'.

Non-limitative examples of such TCPase enzyme used according to the present invention are disclosed in the following Table 2 :

**Table 2**

| **SEQ ID NO** : | **Sequence description** | **Origin** | **Uniprot Reference** | **Function** |
|---|---|---|---|---|
| 1 | Ubiquitin carboxyl-terminal hydrolase 14 | Homo sapiens | P54578 | Indispensable for synaptic development and function at neuromuscular junctions (NMJs). |
| 2 | Ubiquitin carboxyl-terminal hydrolase 5 | Homo sapiens | P45974 | Thiol-dependent hydrolysis of ester, thioester, amide, peptide and isopeptide bonds formed by the C-terminal Gly of ubiquitin (a 76-residue protein attached to proteins as an intracellular targeting signal). |
| 3 | Methionine aminopeptidase 2 | Homo sapiens | P50579 | The enzyme is active with cobalt, zinc, manganese or divalent iron ions. Most likely, methionine aminopeptidases function as mononuclear Fe2+-metalloproteases under physiological conditions, and the catalytically relevant metal-binding site has been assigned to the histidine-containing high-affinity site. |
| 4 | Xaa-Pro aminopeptidase 1 | Homo sapiens | Q9NQW7 | Catalyzes the removal of a penultimate prolyl residue from the N-termini of peptides, such as Arg-Pro-Pro. |
| 5 | Tripeptidyl-peptidase 2 | Homo sapiens | P29144 | May be able to complement the 26S proteasome function to some extent under conditions in which the latter is inhibited. |
| 6 | Vasohibin-1 | Homo sapiens | Q7L8A9 | Angiogenesis inhibitor. Inhibits migration, proliferation and network formation by endothelial cells as well as angiogenesis. |
| 7 | Dihydropyrimidinase-related protein 1 (Collapsin response mediator protein 1 - CRMP1) | Homo sapiens | Q14194 | Necessary for signaling by class 3 semaphorins and subsequent remodeling of the cytoskeleton. Plays a role in axon guidance, invasive growth and cell migration. May participate in cytokinesis. |
| 8 | Dihydropyrimidinase-related protein 2 | Homo sapiens | Q16555 | Plays a role in neuronal development and polarity, as well as in axon growth and guidance, neuronal growth cone collapse and cell migration. Necessary for signaling by class 3 semaphorins and subsequent remodeling of the cytoskeleton. May play a role in endocytosis |
| 9 | Dihydropyrimidinase-related protein 3 | Homo sapiens | Q14195 | Necessary for signaling by class 3 semaphorins and subsequent remodeling of the cytoskeleton. Plays a role in axon guidance, neuronal growth cone collapse and cell migration |
| 10 | Dihydropyrimidinase-related protein 4 | Homo sapiens | 014531 | Necessary for signaling by class 3 semaphorins and subsequent remodeling of the cytoskeleton. Plays a role in axon guidance, neuronal growth cone collapse and cell migration |
| 11 | Dihydropyrimidinase-related protein 5 | Homo sapiens | Q9BPU6 | May have a function in neuronal differentiation and/or axon growth. |
| 12 | Vasohibin-2 | Homo sapiens | Q86V25 | Angiogenesis inhibitor. Inhibits network formation by endothelial cells. |
| 13 | Small vasohibin-binding protein | Homo sapiens | Q8N300 | Enhances the solubility and secretion of VASH1 and prevents its ubiquitination. Also enhances secretion of VASH2 |
| 14 | Uncharacterized protein / VASH | Trypanosoma brucei | Q387E4 | |

In particular, the TCPase enzyme according to the invention is selected from proteins having at least 30%, 40%, 50%, 60%, 70%, 80%, 90%, 95%, 99%, amino acid sequence identity with the amino acid sequence selected from SEQ ID NO: 1 (Ubiquitin carboxyl-terminal hydrolase 14), SEQ ID NO: 2 (Ubiquitin carboxyl-terminal hydrolase 5), SEQ ID NO: 3 (Methionine aminopeptidase 2), SEQ ID NO: 4 (Xaa-Pro aminopeptidase 1), SEQ ID NO: 5 (Tripeptidyl-peptidase 2), SEQ ID NO: 6 (Vasohibin-1), SEQ ID NO: 7 (Dihydropyrimidinase-related protein 1), SEQ ID NO: 8 (Dihydropyrimidinase-related protein 2), SEQ ID NO:9 (Dihydropyrimidinase-related protein 3), SEQ ID NO: 10 (Dihydropyrimidinase-related protein 4), SEQ ID NO: 11 (Dihydropyrimidinase-related protein 5), SEQ ID NO: 12 (Vasohibin-2), or SEQ ID NO: 14 (Uncharacterized protein / VASH of *Trypanosoma brucei*), preferably SEQ ID NO: 6 (Vasohibin-1) or SEQ ID NO: 12 (Vasohibin-2) in presence or absence of Small vasohibin-binding protein (SEQ ID NO: 13).

In a particular embodiment, the isolated proteins with a tubulin carboxypeptidase activity are obtained from a brain extract, such as a brain extract from pigs, and the mass spectrometric data are aligned with human reference sequences, in order to identify corresponding human proteins.

In a particular embodiment, a) the substrate of TCPase enzyme is selected from purified or recombinant α-tubulin from parasites, in particular comprising an amino acid sequence selected from SEQ ID NO: 25 and SEQ ID NO: 26 *(Trypanosoma brucei*) or variants thereof, and b) the isolated or recombinant TCPase enzyme is selected from parasites proteins, in particular having at least 30%, 40%, 50%, 60%, 70%, 80%, 90%, 95%, 99%, amino acid sequence identity with the amino acid sequence SEQ ID NO: 14 (Uncharacterized protein / VASH of *Trypanosoma brucei*).

The term 'parasite' encompasses living organism on (ectoparasite) or in (endoparasite) another organism that feeds at the expense of the host. Mainly, any eukaryotic pathogen that is deemed emerging or re-emerging such as Plasmodium or Neospora and the trypanosomatides. As examples of parasites that may induce human infections, mention may be made of Trypanosoma, Toxoplasma, Leishmania, Ameobe, Giardia lamblia, Trichomonas, Microsporidia....

In a particular and preferred embodiment, a) the substrate of TCPase enzyme is a recombinant engineered telokin, in particular comprising an amino acid sequence SEQ ID NO: 17 or variant thereof, and b) the isolated or recombinant TCPase enzyme is selected from proteins having at least 30%, 40%, 50%, 60%, 70%, 80%, 90%, 95%, 99%, amino acid sequence identity with the amino acid sequence SEQ ID NO: 6 (Vasohibin-1) or SEQ ID NO: 12 (Vasohibin-2) in presence or absence of Small vasohibin-binding protein (SEQ ID NO: 13).

### Conditions for substrate cleavage, in particular detvrosination

The man skilled in the art will adapt and optimize the concentrations of each reagents, in particular substrate of TCPase and TCPase enzyme to favor the substrate cleavage reaction, in particular the detyrosination reaction.

In a particular embodiment, the concentration of TCPase enzymes or fractions thereof contacted with substrate of TCPase is in the range of 0.1 µM to 1 mM, preferably 0.25 µM to 500 µM, more preferably 0.5 µM to 300 µM, and even more preferably 0,5 µM to 200µM, in order to provide optimal conditions for substrate cleavage (e.g detyrosination) of the substrate of TCPase.

In a particular embodiment, the reaction temperature is maintained in the range of 1 °C to 70°C, preferably 5 °C to 65 °C, more preferably 10 °C to 60 °C, even more preferably 15 °C to 55 °C, most preferably 19 °C to 43°C, and for example 19°C to 37 °C in order to provide optimal conditions for the putative TCPase enzyme to cleave the C-terminal aromatic amino acid residue, in particular to detyrosinate the substrate of TCPase.

The man skilled in the art will use the *in vitro* methods of screening available and adapted to detection of modulators of TCPase by measuring substrate cleavage, in particular detyrosination.

### Detection of cleaved C-terminal free aromatic residue

In a first embodiment, the step (ii) of the *in vitro* screening assay of the invention comprises detection of the C-terminal free aromatic amino-acid via a colorimetric assay or using fluorescent properties of the aromatic residues, preferably detection of the C-terminal free-tyrosine via a colorimetric assay using a tyrosinase.

In a preferred embodiment, the *in vitro* screening assay is a tyrosinase-based assay, in particular a colorimetric tyrosinase-based assay. In such an assay according to the invention, step (ii) comprises the use of a tyrosinase and conditions for detecting and measuring free-tyrosine.

Such assay is developed by applying well-described tyrosinase assays that allow quantitative measurements of free tyrosine in a sample (Park et al. 2003, journal of protein chemistry). Tyrosinase is a multicatalytic enzyme participating in several enzymatic reactions in melanin synthetic pathway: the hydroxylation of tyrosine to 3,4-dihydroxyphenylalanine (DOPA), the oxidation of DOPA to DOPAquinone, and the oxidation of 5,6-dihydroxyindole to 5,6-dihydroxyquinone.

In a particular embodiment, we measured dynamic changes of dopaquinone content in the reaction by spectrophotometric analysis. Tyrosinase catalyzes metabolism of both L-tyrosine and L-dopa to dopaquinone.

The original aspect of such assay is the use of a TCPase enzyme, in particular VASH2 and a tyrosine containing substrate such as microtubules, recombinant alpha-tubulin protein, recombinant tyrosinated Telokin or peptides according to the present invention. The use of recombinant tyrosinase that enzymatically oxidize tyrosine to a colorimetric intermediate. Tyrosinase is a polyphenol oxidase that is the rate-limiting enzyme for controlling the production of melanin, which is then possible to measure with a standard 96/384/1536 spectrophotometric plate reader. Samples are compared to a known concentration of tyrosine standard within the microtiter plate format.

In a particular embodiment, the substrate of TCPase is a recombinant engineered telokin (SEQ ID NO: 17), the enzyme TCPase is vasohibin-2 (VASH2) (SEQ ID NO: 12) in the presence or absence of SVBP (SEQ ID NO: 13), and the step (ii) of detecting and measuring the level of free-tyrosine comprises a colorimetric assay, in particular an assay detecting dopaquinone as a result of tyrosinase activity. As such the levels of dopaquinone depend on the TCPase mediated detyrosination leading to an increase in the level of free-tyrosine and as such in the conversion to dopaquinone due to the excess presence of tyrosinase.

In another particular embodiment, the *in vitro* screening assay is based on a non-enzymatic detection method, for detecting cleaved C-terminal free tryptophan (W). Detection of tryptophan can be achieved by fluorescent analysis specific for the aromatic side chain, such as the one disclosed in Gordon et al. (1949).

### Immuno-assav (detection of detyrosinated substrate)

In another and preferred embodiment, the *in vitro* screening assay according to the invention is an immunoassay.

As used herein, the term 'immunoassay' is an assay based on the antigen-antibody binding specificity which allows detecting and optionally quantifying antigens (such as cleaved substrate or detyrosinated substrate) or antibodies. Immunoassays can be classified as immunoassays using non-labeled reagents (antigens or antibodies) and immunoassays using labeled reagents wherein the reagent (antigen or antibody) is labeled with a marker, such as a radioactive isotope, an enzyme, a fluorophore or a substance involved in a (chemo) luminescent reaction, giving rise to so-called radioimmunoassays (e.g., RIA, IRMA), enzyme immunoassays (e.g., EMIT, CEDIA, ELISA for enzyme-linked immunosorbent assay), fluoroimmunoassays (e.g., FPIA, SLFIA, FETI, DELFIA) or luminescent immunoassays, respectively.

In a particular embodiment of the invention, said immunoassay is an ELISA.

In another particular embodiment of the invention, said immunoassay is a Dot-blot.

The phrase "specifically (or selectively) binds" to an antibody, when referring to a protein or peptide, refers to a binding reaction that is determinative of the presence of the protein in a heterogeneous population of proteins and other biologics. Thus, under designated immunoassay conditions, the specified antibodies bind to a particular protein at least two times the background and do not substantially bind in a significant amount to other proteins present in the sample. In the context of the present invention, the 'antigen' is cleaved substrate' in particular 'detyrosinated substrate' which is recognized by an antibody raised against said 'cleaved substrate' in particular 'detyrosinated substrate'.

Included among the immunoassays that use labeled reagents are homogeneous immunoassays and heterogeneous immunoassays; the latter comprise performing one or more steps of separating the antigen or antibody not bound to the other member of the binding pair (antibody or antigen) which will generally be bound to a solid phase or support, for example, microplates, magnetic particles, nitrocellulose membranes, etc. Furthermore, depending on the design of the assay, heterogeneous immunoassays can be competitive or non-competitive, depending on if the labeled reagent (antigen or antibody) is added in a limited amount or in excess, respectively.

Immunoassays allow identifying antigens (direct techniques) or antibodies against an antigen (indirect techniques).

In the context of the present invention, the 'antigen' or 'target protein or peptide' is a cleaved substrate of TCPase, in particular detyrosinated substrate of TCPase, ie a substrate of TCPase cleaved or detyrosinated by the TCPase enzyme present in the reaction solution, in presence or absence (control) of the compounds to be tested (putative modulators of TCPase), and under conditions known by persons skilled in the art allowing the formation of a cleaved substrate of TCPase -antibody complex, preferably detyrosinated substrate of TCPase -antibody complex. This antibody is named 'detection antibody', distinct from 'capture antibody' that may be fixed on solid support.

The detection antibody may be labelled or coupled to a marker.

As it is used herein, the term "marker" relates to an indicator reagent which allows detecting the cleaved or detyrosinated substrate of TCPase - detection antibody complex. By way of non-limiting illustration, said marker can be an enzyme (e.g., peroxidase, glycosidase, alkaline phosphatase, glucose-6-phosphate dehydrogenase, β-galactosidase, β-glucosidase, β-glucuronidase, etc.), a fluorescent compound or fluorophore (e.g., fluorescein, rhodamine, etc.), a (chemo)luminescent compound (e.g., dioxetanes, acridiniums, phenanthridiniums, ruthenium, luminol, etc.), radioactive elements (sulfur, iodine, etc.), etc.

In a particular embodiment, said marker is a peroxidase.

The selection of a particular marker is not critical provided it is able to produce a signal by itself or together with one or more additional substances.

By way of illustration, when the marker is an enzyme the detection of the complex can be carried out by contacting said complex with a suitable substrate, and optionally with suitable enzymatic amplification agents and/or activators. Illustrative examples of said substrates include for example for peroxidases:
Chromogenic: substrates based on 2,2-azinobis(3-ethylbenzthiazoline-6-sulfonic) (ABTS) acid, o-phenylenediamine (OPD), 3,3',5,5'-tetramethylbenzidine (TMB), o-dianisidine, 5-aminosalicylic acid, 3-dimethylaminobenzoic (DMAB) acid and 3-methyl-2-benzothiazolinehydrazone (MBTH), 3-amino-9-ethylcarbazole (AEC) and 3,3'-diaminobenzidine (DAB) tetrachloride, etc.
Fluorogenic: 4-hydroxy-3-methoxyphenylacetic acid, reduced phenoxazines and reduced benzothiazines, including Amplex® Red reagent, Amplex UltraRed reagent, reduced dihydroxanthens, etc.

In a particular embodiment, said marker is a peroxidase, such as the horseradish peroxidase, and the chromogenic substrate is TMB.

### Indirect technique (e.q indirect ELISA)

In a first embodiment of the invention, the substrate of TCPase (which will give the 'target cleaved or detyrosinated substrate' in presence of TCPase enzyme) is directly or indirectly immobilized on a support, such as a well of a microtiter plate, magnetic beads, non-magnetic beads, columns, matrices, membranes, etc.

These materials can be used in suitable forms, such as films, sheets, plates, etc., or it can be used to coat inert carriers (e.g., paper, glass, plastic films, etc.). The immobilization of the substrate of TCPase (which will give the 'target cleaved or detyrosinated substrate' in presence of TCPase enzyme) on said support includes ionic, hydrophobic, and/or similar interactions.

The said substrate of TCPase will be recognized by a detection antibody specific to cleaved or detyrosinated substrate of TCPase. This detection antibody may be labelled or coupled to a secondary labelled antibody specific to first antibody.

### Direct technique (e.g direct ELISA)

In another embodiment of the invention, the capture antibody specific to cleaved or detyrosinated substrate of TCPase is directly or indirectly immobilized on a support, such as a well of a microtiter plate, magnetic beads, non-magnetic beads, columns, matrices, membranes, as disclosed above. The said substrate of TCPase will be recognized by a detection antibody specific to cleaved or detyrosinated substrate of TCPase. This detection antibody may be labelled or coupled to a secondary labelled antibody specific to first antibody

The detection antibodies specific to said 'cleaved target or detyrosinated substrate' are incubated with said 'cleaved target or detyrosinated substrate' for a time period and under conditions suitable in order for cleaved substrate-antibody complexe or detyrosinated substrate -antibody complexes to be formed.

Furthermore, if desired, positive and negative controls can be used for putting the immunoassay of the invention into practice.

In a particular embodiment, Epoxy-Y compound is used as positive control.

In a particular embodiment, non-tyrosinated isolated or recombinant telokin such as the one comprising the amino acid sequence SEQ ID NO: 15, SEQ ID NO: 16 or SEQ ID NO: 18, is used as a negative control.

In a particular embodiment of the invention, the immunoassay is an enzyme immunoassay, a fluoroimmunoassay, a luminescent immunoassay or a radioimmunoassay, preferably an enzyme immunoassay (ELISA).

### Enzyme-Linked Immunosorbent Assay ELISA (preferred one):

In a particular and preferred embodiment, the *in vitro* method of screening of modulators of TCPase according to the invention is an ELISA method. This immunoassay utilizes an antibody labeled with an enzyme marker such as horseradish peroxidase.

In a usual immunoassay ELISA, samples, including a standard containing protein of interest, control specimens, and unknowns, are pipetted into these wells. During the first incubation, the protein antigen binds to the solid support or a capture antibody. After washing, a detection antibody is added to the wells, and this antibody binds to the immobilized protein captured during the first incubation. After removal of excess detection antibody, an HRP conjugate (secondary antibody or streptavidin) is added and binds to the detection antibody. After a third incubation and washing to remove the excess HRP conjugate, a substrate solution is added and is converted by the enzyme to a detectable form (color signal). The intensity of this colored product is directly proportional to the concentration of antigen present in the original specimen. ELISA substrates differ in ease-of-use, sensitivity (i.e., lower limit of detection) and compatibility with imaging equipment. Colorimetric, chemiluminescent and fluorescent ELISA substrates are usable in the context of the test.

In a particular embodiment of the invention, standardized amount of substrate of TCPase according to the invention, preferably engineered Telokin protein containing the C-terminal sequence of alpha-tubulin as disclosed above is coated to a multiwell plate (96/384/1536). Recombinant TCPase enzyme, in particular VASH2 is brought into contact with the substrate in absence or presence of the compound to be tested for a specific time. The enzyme is removed and the well is abundantly and repeatedly washed with a mild detergent solution to remove all VASH2 proteins. HRP-coupled antibody directed against the detyrosinated Telokin engineered substrate is incubated in each well and excess appropriately washed off with a mild detergent solution to remove antibodies that are non-specifically bound. After the final wash step, the plate is developed by adding Chromogenic HRP substrate (the exact type of substrate used for detection will depend on the particular assay and mode of detection) is added and microplate measurement is performed accordingly. High sensitivity may be reached by using classical enhancers (many different enhancers exist and may increase signal profoundly).

In a particular embodiment, the step (ii) of the *in vitro* screening immuno-assay of the invention comprises :
- adding an effective amount of specific labelled antibody raised against cleaved substrate, in particular detyrosinated alpha-tubulin (dTyr-Ab), under conditions that favor the formation of a complex antibody - cleaved substrate; and
- means for revealing the labelled signal;
or alternatively
- adding an effective amount of a primary specific antibody raised against cleaved substrate, in particular detyrosinated alpha-tubulin (dTyr-Ab), under conditions that favor the formation of a complex antibody - cleaved substrate;
- adding an effective amount of a secondary labelled antibody specific of the primary antibody, under conditions that favor the formation of a complex primary antibody- cleaved substrate- labelled secondary antibody, and
- means for revealing the labelled signal,
and wherein the reaction occurs in soluble (fluid) phase or solid phase.

In a particular embodiment of the invention, the antibody is labelled with a marker selected in the group consisting of an enzyme, a fluorescent compound or fluorophore, a (chemo)luminescent compound or a radioactive element, preferably an enzyme and more preferably a peroxidase.

### Soluble or fluid phase

The in vitro assay and reaction may occur in soluble phase (fluid vessel) or solid phase. By 'solid phase' or 'solid support' according to the invention, it encompasses for example, microplates, magnetic particles, nitrocellulose membranes, etc.

In a particular embodiment, microplates are used as solid support.

In a particular embodiment of the invention, the immuno-assay occurs in solid phase, and wherein:
- substrate of TCPase is coated on solid support, in particular on microplate, and the TCPase enzyme, the compound(s) to be tested and the antibodies raised against cleaved substrate, in particular detyrosinated α-tubulin (dTyr-Ab), optionally labelled or combined with labelled secondary antibodies raised against primary antibodies are added in the reaction solution, or
- alternatively, the antibodies raised against cleaved substrate, in particular detyrosinated α-tubulin (dTyr-Ab), are coated on solid support, in particular on microplate, and TCPase enzyme, substrate of TCPase, the compound(s) to be tested, and secondary labelled antibodies raised against cleaved substrate, are added in the reaction solution, or
- alternatively, the TCPase enzyme is coated on solid support, in particular on microplate, and the substrate of TCPase, the compound(s) to be tested, and the antibodies raised against cleaved substrate, in particular detyrosinated α-tubulin (dTyr-Ab), optionally labelled or combined with labelled secondary antibodies raised against primary antibodies, are added in the reaction solution.

In a particular and preferred embodiment of *in vitro* immuno-assay of the invention, (a) the substrate of TCPase comprises dimers of alpha-tubulin extracted from SF9 cells (*Spodoptera frugiperda* origin), in particular comprising the amino acid sequence SEQ ID NO:27 or a variant thereof, (b) the TCPase enzyme is vasohibin-2 (VASH2) (SEQ ID NO: 12) in the presence or absence of SVBP (SEQ ID : 13), and the step (ii) of detecting and measuring the level of detyrosinated-substrate comprises at least an antibody raised against detyrosinated α-tubulin SF9 (dTyr-Ab SF9) coupled to horseradish peroxidase (HRP).

### Dot blot/ Slot blot

In another embodiment, the immunoassay according to the invention is a dotblot.

In this technique, a reaction mixture containing the substrate and the enzyme in absence or in presence of the compound is directly spotted onto the filter. The filter is allowed to dry and the membrane is incubated in blocking buffer to prevent non-specific binding. Similarly to ELISA, HRP-coupled antibody directed against the cleaved substrate is incubated over the membrane, excess is washed-off and the membrane is developed using e.g. chromogenic HRP substrate. Intensities of the signals on autoradiograms were quantified by densitometric scanning. Alternatively, we designed a method using a slot blot approach. The steps are identical as for the dot blot approach, this is the samples are applied to a nitrocellulose or PVDF membrane with a vacuum manifold to produce an orderly grid of samples. Once dry, dot blots and slot blots are subjected to the same immunodetection steps used for Western blotting.

### Microfluidic

In another embodiment, the immunoassay according to the invention is a microfluidic assay.

The ELISA assay can be adapted to microfluidic equipment/platform. Those miniaturized analytical platforms are capable of manually performing parallel liquid transfer, reaction, and analysis. Multiple examples exist of miniaturized ELISA tests.

### High Throughput Screening (HTS)

In a particular embodiment, the *in vitro* screening assay is a High Throughput Screening (HTS) assay, in particular a High Throughput Screening (HTS) assay based on ELISA assay. HTS is a method for scientific experimentation especially used in drug discovery and relevant to the fields of biology and chemistry (High-throughput screening assays for the identification of chemical probes. Inglese J, et al. Nat Chem Biol. 2007 Aug;3(8):466-79. Review). Using robotics, data processing/control software, liquid handling devices, and sensitive detectors, high-throughput screening allows a researcher to quickly conduct millions of chemical, genetic, or pharmacological tests. Through this process one can rapidly identify active compounds, antibodies, or genes that modulate a particular biomolecular pathway. The results of these experiments provide starting points for drug design and for understanding the interaction or role of a particular biochemical process in biology.

In a particular embodiment, the invention provides solid phase based in vitro assays in a high throughput format, where the TCPase or substrates of TCPase (ie microtubules, alpha-tubulin, tubulin dimers, recombinant tyrosinated telokin, peptides...) are attached to a solid phase substrate.

In the high throughput assays of the invention, it is possible to screen up to several thousand different modulators in a single day. In particular, each well of a microtiter plate can be used to run a separate assay against a selected potential modulator, or, if concentration or incubation time effects are to be observed, every 5-10 wells can test a single modulator. Thus, a single standard microtiter plate can assay about 100 (e.g., 96) modulators. If 1536 well plates are used, then a single plate can easily assay from about 1000 to about 1500 different compounds. It is also possible to assay multiple compounds in each plate well. It is possible to assay several different plates per day; assay screens for up to about 6,000-20,000 different compounds are possible using the integrated systems of the invention. Also, microfluidic reagent manipulation may be used.

The spectrum of low- throughput screening (10,000-50,000 assay points) medium-throughput screening (50,000-100,000 data points) and high- throughput screening (100,000-500,000 data points) can be defined. The scale of implementation of a given screen is greatly influenced by format, application of technology (e.g. automation), time and resource constraints.

### Antibodies

As used herein, the terms "antibody," "antibodies" includes whole antibodies and any antigen binding fragment or a single chain thereof. Thus the term "antibody" includes any protein or peptide containing molecule that comprises at least a portion of an immunoglobulin molecule. The terms "antibody," "antibodies" also include immunoglobulins of any isotype, fragments of antibodies which retain specific binding to antigen, including, but not limited to, Fab, Fab', F(ab)2, Fv, scFv, dsFv, Fd fragments, dAb, VH, VL, VhH, and V-NAR domains; minibodies, nanobodies, diabodies, triabodies, tetrabodies and kappa bodies; multispecific antibody fragments formed from antibody fragments and one or more isolated. Examples of such include, but are not limited to a complementarity determining region (CDR) of a heavy or light chain or a ligand binding portion thereof, a heavy chain or light chain variable region, a heavy chain or light chain constant region, a framework (FR) region, or any portion thereof, at least one portion of a binding protein, chimeric antibodies, humanized antibodies, single-chain antibodies, and fusion proteins comprising an antigen-binding portion of an antibody and a non-antibody protein. The variable regions of the heavy and light chains of the immunoglobulin molecule contain a binding domain that interacts with an antigen. The constant regions of the antibodies (Abs) may mediate the binding of the immunoglobulin to host tissues.

The antibodies can be polyclonal, monoclonal, multispecific (e.g., bispecific antibodies), and antibody fragments, so long as they exhibit the desired biological activity. Antibodies can be isolated from any suitable biological source, e.g., murine, rat, rabbit, etc. As used herein, "monoclonal antibody" refers to an antibody obtained from a substantially homogeneous antibody population. Monoclonal antibodies are highly specific, as each monoclonal antibody is directed against a single determinant on the antigen. The antibodies may be detectably labeled, e.g., with a radioisotope, an enzyme which generates a detectable product, a fluorescent protein, and the like. The antibodies may be further conjugated to other moieties, such as members of specific binding pairs, e.g., biotin (member of biotin-avidin specific binding pair), and the like.

Monoclonal antibodies may be generated using hybridoma techniques or recombinant DNA methods known in the art. A hybridoma is a cell that is produced in the laboratory from the fusion of an antibody-producing lymphocyte and a non-antibody producing cancer cell, usually a myeloma or lymphoma. A hybridoma proliferates and produces a continuous supply of a specific monoclonal antibody. Alternative techniques for generating or selecting antibodies include in vitro exposure of lymphocytes to antigens of interest, and screening of antibody display libraries in cells, phage, or similar systems.

In the context of the present invention, specific antibodies directed against cleaved or detyrosinated tubulin were generated. The antibody was raised by injecting rabbits with a peptide composed of the very C-terminal sequence of detyrosinated tubulin (without the most C-terminal Y (DeTyr)). In a particular and preferred embodiment, we use a *Spodoptera frugiperda* (SF) tubulin, wherein the sequence of the peptide contains the 7 most C-terminal sequence of the protein without the most C-terminal Y (DeTyr) and an additional cysteine at the N-terminal for practical reasons (Seq: C-EGEGAEE). Characterization of the antibody is performed by expression of full-length mouse or *Spodoptera frugiperda* alpha-tubulin or detyrosinated counterpart in HEK cells (**Figure 6**).

### KIT

The present invention also concerns the kits comprising the reagents and instructions for performing the in vitro screening assay disclosed above.

In particular, a kit of the invention comprises substrates TCPases (preferably lyophilized), reaction buffer, substrate solvent, stabilized enzyme TCPases in appropriate long-storage buffer, specific antibodies directed against cleaved or detyrosinated substrate coupled to detection system (e.g. HRP) (for immuno-assay), a compound known to inhibit TCPase (positive control), a standard (negative control) and a detailed protocol.

The kit may be used as a solution-based assay featuring the speed, high sensitivity, and convenience required for screening TCPase modulators in a high-throughput format (96/384/1536).

In a particular embodiment, the invention concerns a kit for performing the *in vitro* screening immuno-assay of the invention, comprising :
(i) a TCPase enzyme substrate as defined in the present invention, preferably a recombinant engineered telokin as substrate of TCPase enzyme, in particular comprising an amino acid sequence SEQ ID NO: 17 or variant thereof,
(ii) a TCPase enzyme as defined in the present invention, preferably an isolated or recombinant TCPase enzyme selected from proteins having at least 30%, 40%, 50%, 60%, 70%, 80%, 90%, 95%, 99%, amino acid sequence identity with the amino acid sequence of Vasohibin-2 (SEQ ID NO: 12) in presence or absence of Small vasohibin-binding protein (SEQ ID NO: 13),
(iii) antibodies raised against cleaved substrate, preferably detyrosinated α-tubulin (dTyr-Ab), in particular raised against detyrosinated α-tubulin SF9 (dTyr-Ab SF9) and coupled to horseradish peroxidase (HRP), and optionally secondary antibodies raised against the cleaved substrate antibodies, preferably the detyrosinated α-tubulin antibodies,
(iv) a negative control, in particular comprising a non-tyrosinated isolated or recombinant telokin,
(v) a positive control, in particular comprising Epoxy-Y compound,
(vi) a fluid vessel or alternatively a solid support for coating or pre-coating either the substrate of TCPase either the TCPase enzyme, preferably membranes or microplates,
(vii) reagents for allowing contact of said substrate with TCPase enzyme in reaction conditions for substrate cleavage, preferably detyrosination,
(viii) reagents for detecting and measuring the level of substrate cleavage, preferably detyrosination ; and
(ix) optionally a notice of use.

Another object of the invention is a kit for performing the *in vitro* screening assay for detection of the free C-terminal aromatic amino-acid via a colorimetric assay or using fluorescent properties of the aromatic residues, comprising :
(i) a TCPase enzyme substrate, preferably a recombinant engineered telokin as substrate of TCPase enzyme, in particular comprising an amino acid sequence SEQ ID NO: 17 or variant thereof,
(ii) a TCPase enzyme, preferably an isolated or recombinant TCPase enzyme selected from proteins having at least 30%, 40%, 50%, 60%, 70%, 80%, 90%, 95%, 99%, amino acid sequence identity with the amino acid sequence of Vasohibin-2 (SEQ ID NO: 12) in presence or absence of Small vasohibin-binding protein (SEQ ID NO: 13),
(iii) optionally a tyrosinase for the detection of a C-terminal free-tyrosine via colorimetric assay,
(iv) a fluid vessel or alternatively a solid support for coating or pre-coating either the substrate of TCPase either the TCPase enzyme, preferably membranes or microplates,
(v) reagents for allowing contact of said substrate with TCPase enzyme in reaction conditions for liberation of a C-terminal free-aromatic amino acid residue, preferably a C-terminal free-tyrosine,
(vi) reagents for detecting and measuring the level of the C-terminal free-aromatic amino acid residue, preferably the C-terminal free-tyrosine; and
(vii) optionally a notice of use.

The *in vitro* screening assay can easily be implemented in high throughput screening (HTS) methods for hit identification within large chemical libraries and following lead optimization in the pharmaceutical company.

### THERAPEUTIC APPLICATIONS

Detyrosination of microtubules is associated with cancer progression, aberrant neuronal networks, weak neuronal remodeling, plasticity and/or adaptation.

Accordingly, the use of such modulators of TCPase, selected by the *in vitro* screening assay of the present invention, may have a positive impact in the treatment of disorders involving microtubule detyrosination, in particular heart disorder, vascular disorder, cancers, neurodegenerative disorders, muscle disorders, infertility, ciliopathies, more generally a disorder involving altered microtubule detyrosination in an animal, preferably but not limited to a mammal. For instance, inhibitors of TCPase may be used for increasing the microtubule dynamics and thereby impacting neuroregeneration, constituting good candidates for treating neurodegenerative diseases, preferably selected from Alzheimer disease, Parkinson disease, psychiatric disorders, and neural disorders, neuronal regeneration disorders, cancers, preferably selected from colon cancer and neuroblastoma, muscle disorders such as muscular dystrophies, retinal degeneration, heart diseases, vascular disorders, infertility, and ciliopathies (Cell 2018 review Magiera et al.). In another and particular application, the use of such modulators of TCPase from parasites may help finding a treatment against human parasites. The term 'parasite' encompasses living organism on (ectoparasite) or in (endoparasite) another organism that feeds at the expense of the host. Mainly, any eukaryotic pathogen that is deemed emerging or re-emerging such as Plasmodium or Neospora and the trypanosomatids. As examples of parasites that may induce human infections, mention may be made of Trypanosoma, Toxoplasma, Ameobe, Giardia lamblia, Trichomonas, Microsporidia...

In particular, such modulators compounds selected from the *in vitro* screening assay according to the present invention may be use in a pharmaceutical composition for the prevention and/or treatment of disorders related to alterations of TCPase activity, in particular neurodegenerative disorders or muscular disorders, in particular Alzheimer disease, Parkinson disease and/or dementia, cancer, Duchenne myopathy dystrophy, Chronic obstructive pulmonary disease (COPD).

### DESCRIPTION OF THE FIGURES

Figure 1: Schematic representation of tubulin purification procedure from *Spodoptera Frugiperda* (SF) cells (Insect cells). Tubulin was mock- or carboxypeptidase treated and analyzed on by gel electrophoresis. Next tubulin was in-gel digested using trypsin (0.5 µg/band; Gold; Promega). Peptides were then analyzed online by nano-flow HPLC-nanoelectrospray ionization using a Qexactive Plus mass spectrometer (Thermo Fisher Scientific) coupled to a nano- LC system (U3000-RSLC, Thermo Fisher Scientific).
Figure 2: Coomassie staining of samples collected at various steps during the purification protocol of bacterially produced CRMP1 recombinant protein. Purification consists of immobilized metal affinity chromatography (IMAC). Arrow indicates recombinant CRMP1 protein.
Figure 3: Coomassie staining of samples collected at various steps during the two-step purification protocol of bacterially produced VASH1 recombinant protein. A first step consisting of immobilized metal affinity chromatography (IMAC) was followed by ion exchange (IEX) chromatography in order to further increase purity of recombinant protein. Arrow indicates recombinant VASH1 protein.
Figure 4: Coomassie staining of samples collected at various steps during the two-step purification protocol of bacterially produced VASH2 recombinant protein. A first step consisting of immobilized metal affinity chromatography (IMAC) was followed by ion exchange (IEX) chromatography in order to further increase purity of recombinant protein. Arrow indicates recombinant VASH2 protein.
Figure 5: Coomassie staining of samples collected at various steps during the two-step purification protocol of bacterially produced *Trypanosoma brucei* VASH (TbVASH) recombinant protein. A first step consisting of immobilized metal affinity chromatography (IMAC) was followed by ion exchange (IEX) chromatography in order to further increase purity of recombinant protein. Arrow indicates recombinant TbVASH protein.
Figure 6: Characterization of the *Spodoptera frugiperda* (Sf) antibody. The antibody was generated by injection of the detyrosinated tubulin sequence in rabbits (Seq: C-EGEGAEE). Immunoblot analysis of HEK cells transfected with indicated tubulin constructs. Twenty-four hours post transfection samples were collected and analyzed by immunoblot with indicated antibodies. Immunoblot analysis showed that the mouse (Mm) antibody failed to recognize *Spodoptera Frugiperda* (Sf) detyrosinated tubulin and inversely
Figure 7: Polymerization of tubulin into microtubules. Microscopy analysis of rhodamine labeled microtubules. Polymerization was achieved in presence of 20µM Taxol at 37°C during 30 minutes. Representative imaged of the obtained microtubules fibers.
Figure 8: VASH2 acts as autonomous TCPase. *In vitro* detyrosination assay using recombinant VASH1 and VASH2 proteins alone or coexpressed with Small Vasohibin Binding Protein (SVBP). *In vitro* detyrosination was performed using *Spodoptera Frugiperda* (Sf) purified tubulin.
Figure 9: Activation of TCPase by SVBP. In cellulo (HEK) detyrosination assay using coexpression of VASH1 and VASH2 without or in presence of Small Vasohibin Binding Protein (SVBP). Coexpression of SVBP induces VASH1 and VASH2 detyrosination activity.
Figure 10: Recombinant human VASH2 produced in bacteria detyrosinates polymerized microtubules (MT), tubulin dimers (TD) and recombinant alpha tubulin. Oppositely, VASH1 was much less effective on microtubules and tubulin dimers and almost inactive on recombinant alpha tubulin produced in bacteria.
Figure 11: Recombinant human VASH2 and catalytically dead (VASH2D) produced in bacteria detyrosinates a 8 amino acid peptide consisting of GEEEGEEY. The samples were doted on nitrocellulose and assessed for detyrosination of the peptide using a specific antibody detecting detyrosination.
Figure 12: Time course analysis of VASH2-mediated detyrosination. *In vitro* detyrosination assay of bacterially purified VASH2. Reactions were stopped at indicated time and analyzed by immunoblot and relative optical density was measured.
Figure 13: Immuno-assay based detyrosination assay. *In vitro* detyrosination assay using recombinant VASH2 brought in contact with increasing dose (0.1; 1 and 10 µM) of previously described peptide-based inhibitor (Epoxy-Y). The assay is performed using *Spodoptera Frugiperda* (Sf) purified tubulin.
Figure 14: Schematic representation of preferred HTS immuno-assay. The substrate coated 96 wells plate is first brought into contact, in absence (control) or presence of a coumpound to be tested, with the recombinant active bacterially produced VASH2 for 15 minutes. The enzymes is washed off using appropriate buffer (preferred is phosphate buffer), and HRP-coupled antibody specifically targeting detyrosinated tubulin is brought in contact with substrate. Excess antibody is washed off and detection is performed in plate reader.
Figure 15: Colorimetric analysis of tyrosine concentration in the reaction mixture. (A) shows the ideal excitation wavelength (300nm). (B) Linearity of colorimetric signal obtained when free tyrosine is measured at 305nm. The concentration of tyrosine is displayed in molarity.
Figure 16: Colorimetric analysis of free tyrosine released by VASH2 detyrosination of recombinant human alpha tubulin. OD305 was measured of control samples (filled histogram) and VASH2-mediated tyrosine release.
   A. control sample containing tyrosinase,
   B. control sample containing recombinant human alpha tubulin,
   C. control samples containing tyrosinase (Tyr) and VASH2 as detyrosinase,
   D reaction mixture containing recombinant human alpha tubulin, VASH2 mediating tyrosine release and tyrosinase to allow detection of free tyrosine.
   OD305 was measured 30 min after onset of reaction at room temperature.

The present invention will be illustrated by the following non-limitative examples.

### EXAMPLES

### Material and methods

### Preparation of substrates of TCPase:

### Preferred substrate of TCPase:

In the context of the present invention, a recombinant tyrosinated telokin has been obtained as following: the C-terminal tail of alpha-tubulin (the 11 most C-terminal residues EGEGEEEGEEY= SEQ ID NO: 39) has been engineered at the C-terminal part of the Telokin isoform (Q6PDN3-4Q15746-8). Mouse cDNA was obtained using prime script RT-PCR kit (Takara) and telokin was amplified using adapted forward and reverse primers containing cloning sites. The amplicon was extracted and purified and the expression plasmid as well as the amplicon digested adapted restriction enzymes for cloning in bacterial expression vectors (pET-28a(+) and pGEX). In particular the both sequences are recombinantly expressed in a host cell, such as an E. coli. Obtained plasmid containing the last C-terminal amino acid sequence of human alpha tubulin 1A (N-EGEGEEEGEEY= SEQ ID NO: 39) at the very end of the telokin gene was sequence verified by double digestion diagnostics and by sequencing. Next, bacteria were transformed and selected on an antibiotic containing LB-dish. A single antibiotic-resistant colony was picked and grown to confluency as a starter overnight. Next day, fresh LB medium was inoculated with the bacteria and optical density (OD600) measured at regular interval. Once the bacteria OD600 reached between 0.4 and 0.6, IPTG was added for inducing protein production. After 4 hours, cells were harvested and telokin substrate purified using IMAC technology.

We obtained a recombinant tyrosinated telokin comprising the sequence SEQ ID NO:17 (Mus musculus, fully tyrosinated variant).

Negative control Telokin lacking the most C-terminal tyrosine (Y) residue can easily be implemented. In particular, we used an isolated (non-tyrosinated) telokin comprising the sequence SEQ ID NO:15 (Homo sapiens, Uniprot Q15746-8) or SEQ ID NO:16 (Mus musculus, Uniprot Q6PDN3-4) or a recombinant (detyrosinated V1 variant) telokin comprising the amino acid sequence SEQ ID NO: 18 (the C-terminal sequence of alpha-tubulin (Tubulin alpha-1A chain - human) without the ultimate Y, has been introduced in the Telokin sequence). Various substrates can be engineered to generate additional needed controls in the assay.

### Additional substrates of TCPase:

- Purified or recombinant tyrosinated tubulin from *Spodoptera Frugiperda* (Sf) cells (Sf9, Sf21 etc.) : Tubulin was purified using a TOG column (Widlund et al. 2012 MBoC) and analyzed by mass spectrometry. In particular, we used a purified or recombinant tyrosinated tubulin comprising the amino acid sequence of SEQ ID NO:28 (Uniprot G3CKA7); Tubulin was polymerized to obtain fully purified microtubules.
- Recombinant alpha tubulin (α-tubulin) produced in bacteria according to known methods: in particular, we used a purified or recombinant α-tubulin from human in particular comprising the amino acid sequences SEQ ID NO: 19 to SEQ ID NO: 24), or from *Trypanosoma brucei* in particular comprising the amino acids sequences SEQ ID NO: 25 and SEQ ID NO: 26, or from *Toxoplasma gondii* in particular comprising the amino acids sequence SEQ ID NO: 27, or from *Drosophila melanogaster* in particular comprising the amino acids sequence (SEQ ID NO:29).
- Tubulin dimers purified from any eukaryotic cells.
- Isolated or recombinant peptides from 4 to 50 amino acid residues, in particular 5 to 20 amino acids, preferably 6 to 12 amino acid residues comprising an amino acid sequence having at least the last 4 amino acids residues of the C-terminal sequence of an α-tubulin and/or a Microtubule Associated Protein (MAP) and, as ultimate C-terminal amino acid residue, an aromatic amino acid residue, preferably a tyrosine (Y), in particular comprising an amino acid sequence selected from SEQ ID NO: 33 to SEQ ID NO: 39 or variant thereof.

### Preparation of recombinant TCPases (VASH1, VASH2, CRMP1):

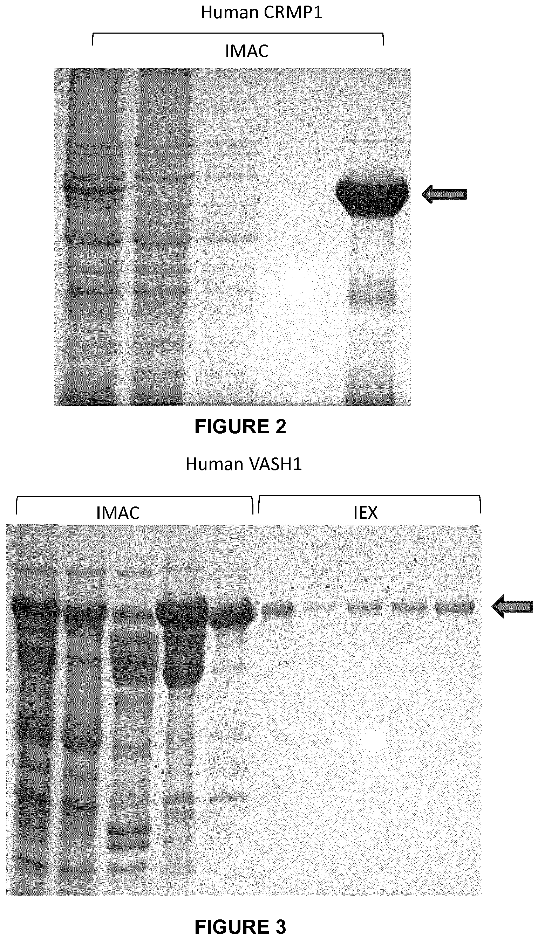

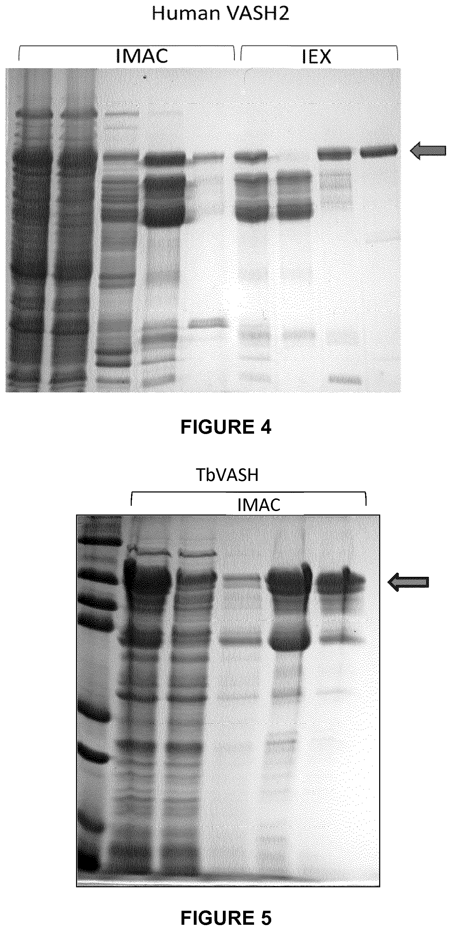

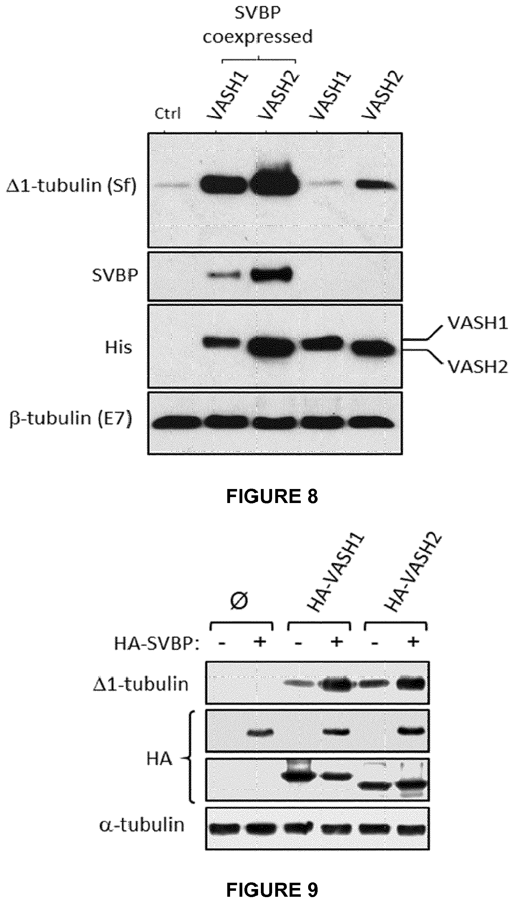

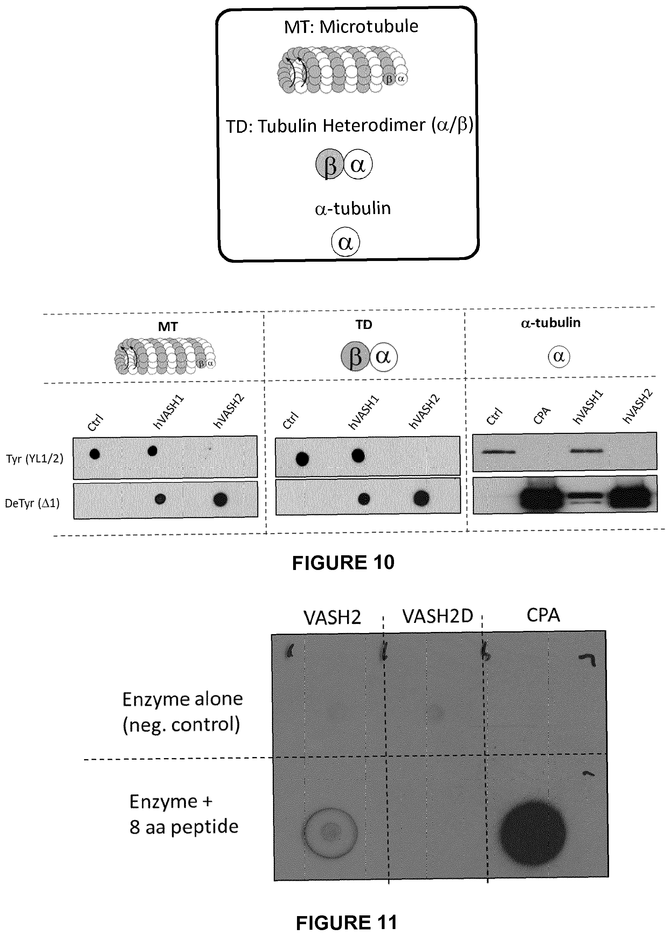

Enzymes are produced in *E. coli* bacteria using expression vectors such as pET-28a(+), pGEX or any other adapted expression vectors. The different enzymes (human VASH1, VASH2, CRMP1, *Trypanosoma brucei* VASH) have been cloned and bacteria have been induced according to manufacturer's guidelines. Bacteria were grown until OD600 reached between 0.4-0.8, quickly cooled in ice water for 30 minutes. Protein production was induced using IPTG and incubated at 15°C for 24 hours under rigorous shaking. The bacteria were pelleted by centrifugation and stored at -80°C until further use. Bacteria were resuspended in disruption buffer and mechanically disrupted using a French press. Recombinant protein was purified by immobilized metal affinity chromatography (IMAC) and if needed purity was further increased by ion-exchange column (**Figure 2-5**). The recombinant proteins have been assessed for their TCPase activity on various substrates. Importantly we found that VASH2 acted as an autonomous enzyme that did not require the small vasohibin binding protein as a chaperone to enzymatically be active (**Figure 8**). Advantageously, recombinant purified VASH2 protein could be stored at 4°C for at least 3 months without noticeable loss of activity. More advantageously, recombinant VASH2 protein showed detyrosination activity on polymerized microtubule, tubulin dimers, recombinant alpha tubulin, engineered Telokin protein containing C-terminal sequence of alpha tubulin and on synthetic peptides (**Figure 10**). As such recombinant VASH2 presented unexpected advantageous characteristics allowing its use in various applications such as HTS assays for identification of detyrosinase modulating compounds.

### Compounds to be tested (putative modulators of TCPase)

The compounds to be tested may come from chemical libraries of compounds containing a large number of diverse and different compounds. Many of these libraries may be commercially available. Alternatively, smaller libraries may be tested on different substrates (microtubule versus tubulin dimer).

### Reagents and antibodies

For western blot or immunoblot, all antibodies were applied overnight at 4 °C in phosphate-buffered saline (PBS) containing 1% BSA and 0.1% Tween (Sigma).

The antibodies used were directed against: Δ1-tubulin (Abcam)), GFP (Millipore), VASH2 (Millipore), beta-tubulin (E7-DSHB), SVBP (Sigma), YL1/2 (Synaptic Systems). The antibody directed against Sf9 Δ1-tubulin (deTyr-tub) were raised in rabbits against the peptide EGEGAEE (= SEQ ID NO: 40).

Specific antibodies directed against detyrosinated tubulin were generated. The antibody was raised by injecting rabbits with a peptide composed of the very C-terminal sequence of *Spodoptera frugiperda* (SF) tubulin. The sequence of the peptide contains the 7 most C-terminal sequence of the protein without the most C-terminal Y (DeTyr) and an additional cysteine at the N-terminal for practical reasons (Seq: C-EGEGAEE= (= SEQ ID NO: 40). Characterization of the antibody was performed by expression of full-length mouse or *Spodoptera frugiperda* alpha-tubulin or detyrosinated counterpart in HEK cells (**Figure 6**).

Advantageously, the said antibody is labelled, ie coupled with a peroxidase, such as the horseradish peroxidase (HRP), and the chromogenic substrate is 3,3',5,5'-tetramethylbenzidine TMB. For the colorimetric assay the tyrosinase enzyme was purchased at Sigma-Aldrich (Tyrosinase from mushroom) and spectrophotometric analysis was performed using Nanodrop (Thermo Fisher).

### Mass spectrometry

Proteins were separated on SDS-PAGE gels (10% polyacrylamide; Mini-Protean TGX Precast Gels; Bio-Rad) and stained with Page Blue Stain (Fermentas). Gel lanes were cut into several gel pieces and destained by three washes in 50% acetonitrile and 50 mM triethylammonium bicarbonate. Proteins were in-gel digested using trypsin (0.5 µg/band; Gold; Promega).

Peptides were then analyzed online by nano-flow HPLC-nanoelectrospray ionization using a Qexactive Plus mass spectrometer (Thermo Fisher Scientific) coupled to a nano- LC system (U3000-RSLC, Thermo Fisher Scientific). Desalting and preconcentration of samples were performed on-line on a Pepmap® precolumn (0.3 × 10 mm; Dionex). A gradient consisting of 0-40% B in A for 100 min (A: 0.1% formic acid, 2% acetonitrile in water, and B: 0.1% formic acid in 80% acetonitrile) at 300 nl/min, was used to elute peptides from the capillary reverse-phase column (0.075 × 150 mm, Pepmap®, Dionex). Data were acquired using the Xcalibur software (version 4.0). A cycle of one full-scan mass spectrum (375-1,500 m/z) at a resolution of 70,000 (at 200 m/z), followed by 12 data-dependent MS/MS spectra (at a resolution of 17,500, isolation window 1.2 m/z) was repeated continuously throughout the nanoLC separation.

Raw data analysis was performed using the MaxQuant software (version 1.5.5.1) with standard settings. Used database consist of *Spodoptera frugiperda* entries from Uniprot, G3CKA7 sequence (Uniprot) deleted from 1 to 6 amino acids in Cterm and 250 classical contaminants (MaxQuant contaminant database). Relative abundance of peptide was estimated using Skyline 3.6.0.

### E. coli bacterial and Sf insect cells culture and protein production (VASH1, VASH2 and SVBP)

Both bacterial and insect cells (Sf9 and Sf21) were used for recombinant protein production. Insect cells Sf9 and Sf21 cells were grown in EX-CELL® 420 Serum-free medium (14420C, Sigma-Aldrich). Cells were maintained between 1 and 10 million cells/ml in shake flasks, at 28 degrees and 140 rpm agitation. For production of human VASH1, VASH2 and SVBP cDNAs were cloned into pFastBac vector MAX efficiency DH10Bac™ Next, competent bacteria (10361-012, Invitrogen) were transformed to produce bacmids. To generate baculoviruses, 12 million Sf21 cells (1 ml) were transfected with bacmid using Cellfectin™ (P/N 58760, Invitrogen). After 5 hours incubation, medium was added and cultures were incubated for 2,5 days. Supernatant (P1) was collected by centrifugation and used to infect 200 million Sf21 cells. Cells were incubated for 2 days and supernatant (P2) was collected by centrifugation. For infection, 40 million Sf21 cells (in 20 ml) were infected with combinations of P2 encoding VASH1, VASH2 and SVBP at 1:50 dilution. Coexpression of VASH1 and VASH2 with SVBP was performed by mixing 1:1 with respective P2 supernatants. After 48 hours, infected cells were collected by centrifugation and analyzed by immunoblotting. For bacterial production of recombinant protein, competent *E. coli* bacterial cells were transformed with plasmid expression vector containing the protein of interest (VASH1, VASH2, human alpha tubulin, engineered telokin, CRMP1, etc.) and grown on antibiotic containing LB medium. Bacteria were allowed to grow at 37°C and a single colony was picked. A starter was allowed to grow to confluence in a small LB volume containing the antibiotic overnight. Next, the *E. coli* bacterial cells were inoculated in appropriate volume and OD600 was monitored regularly using a spectrometer. Once *E. coli* bacterial cell confluency reached between 0.4 and 0.8 at OD600, bacteria were IPTG induced at 16°C for 24 hours and collected by centrifugation. Cell pellets were stored at -80°C until further processed. Purification of recombinant protein was performed using IMAC. The purity of all recombinant proteins were assessed by Coomassie staining.

### Example 1: In vitro immunoassay (Immunoblot and ELISA) for identification of detvrosinase (TCPase) modulators compounds

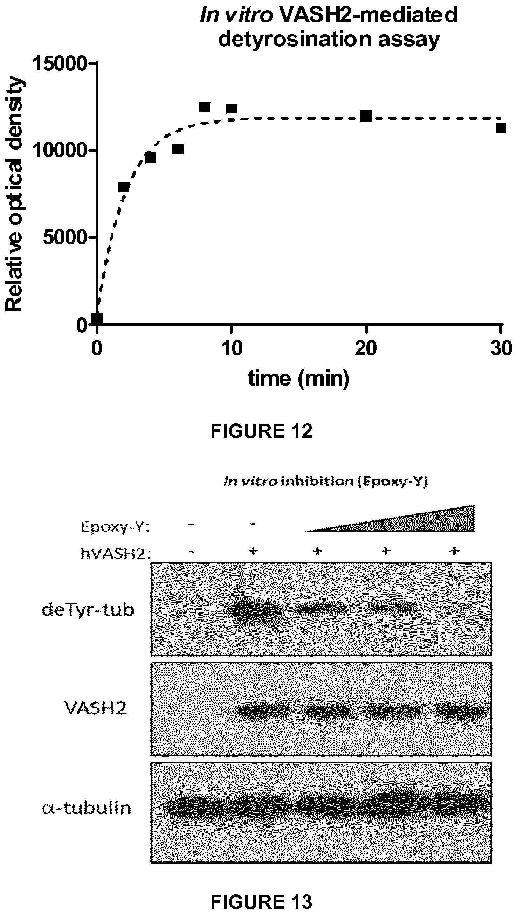

The proof of principle of an immunoassay such as ELISA assay has been obtained by using VASH2 recombinant protein for enzymatic description (**Figure 12**). Advantageously the assay has been used in a dose-response analysis using a previously described Epoxy-Y inhibitor (Science 2017, Aillaud et al.). As expected increasing amounts of Epoxy-Y inhibitor resulted in a dose dependent inhibition of VASH2 detyrosinase activity. Oppositely SVBP induces tubulin carboxypeptidase activity of VASH2 (**Figure 9**). Next, Tubulin purified from Sf9 insect cells (fully tyrosinated; **Figure 1**) was polymerized in presence of Taxol at 37°C for 30 min, washed and stored in -80°C until further use. Recombinant VASH2 protein was pre-incubated 5 min at room temperature in presence or absence of Epoxy-Y. The previously prepared microtubules (MTs) (substrate of TCPase, ie VASH2 protein) were added to the reaction mixture in a phosphate buffer containing 100mM NaCl at pH7.0. The reaction was performed for 10 minutes at room temperature, stopped using sample buffer and analyzed by immunoblotting using specific labelled antibodies directed against Sf9 tubulin (**Figure 13**).

The same protocol is further reproduced with the compounds to be tested (putative modulators of TCPase, ie VASH2) in replacement to Epoxy-Y, previously used to validate the assay. So recombinant VASH2 protein was pre-incubated 5 min at room temperature in presence or absence of the compound to be tested. The previously prepared microtubules (MTs) (substrate of TCPase, ie VASH2 protein) were added to the reaction mixture in a phosphate buffer containing 100mM NaCl at pH7.0. The reaction was performed for 10 minutes at room temperature, stopped using sample buffer and analyzed by immunoblotting using specific labelled antibodies directed against detyrosinated Sf9 tubulin (dTyr-tub). If the signal level (detyrosination) is lower than the control (in absence of the compound to be tested), it means that the compound to be tested is an inhibitor of VASH2, as Epoxy-Y, whereas if the signal level (detyrosination) is higher than the control (in absence of the compound to be tested), it means that the compound to be tested is an activator of VASH2. The man skilled in the art will adapt the concentration of enzyme, substrate and compounds to be tested, in order to favor the detyrosination of substrate (microtubules MT). In an alternative, other substrates of VASH2 are used instead of micrcotubules MT, in particular the ones disclosed in Material & Methods above, and preferably a recombinant tyrosinated telokin such as the one comprising the amino acids sequence SEQ ID NO: 17.

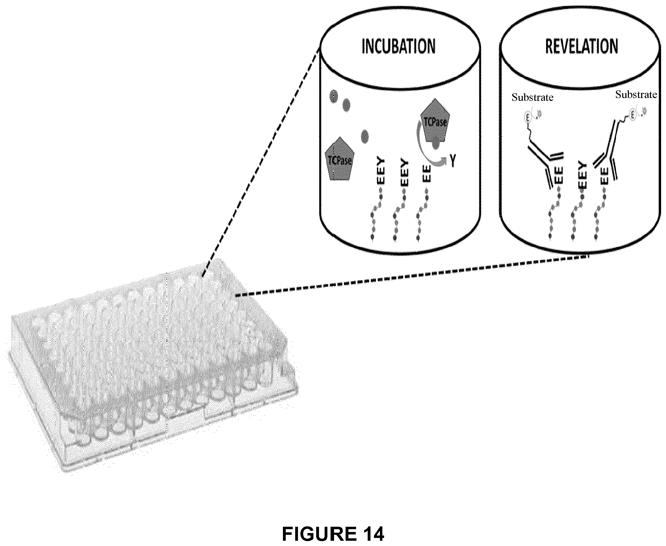

The method then allowed the development of an ELISA-based assay screening of chemical compounds (putative modulator compounds) in a microtiter plate. A schematic representation of an ELISA-based assay for HTS assay is shown in **Figure 14****.**

The substrate of TCPase is selected from the ones disclosed above in Material & Methods. In particular, we used a recombinant tyrosinated telokin comprising the sequence SEQ ID NO:17 (Mus musculus, fully tyrosinated variant).

As negative control, we used an isolated (non-tyrosinated) telokin comprising the sequence SEQ ID NO:16 Uniprot Q6PDN3-4) without the ultimate Y .

We use the reagents and instructions of commercial ELISA kit.

Standardized amount of recombinant Telokin protein containing the C-terminal sequence of alpha-tubulin as disclosed above is coated to a multiwell plate (96/384/1536).

The substrate coated 96 wells plate is first brought into contact, in absence (control) or presence of a compound to be tested (putative modulator of TCPase), with the recombinant active bacterially produced VASH2 for 15 minutes. The enzyme is washed off using appropriate buffer (preferred is phosphate buffer), and HRP-coupled antibody specifically targeting detyrosinated α-tubulin SF9 (dTyr-Ab SF9), is brought in contact with substrate in each well and excess appropriately washed off with a mild detergent solution to remove antibodies that are non-specifically bound. After the final wash step, the plate is developed by adding Chromogenic HRP substrate TMB is added and microplate measurement is performed accordingly.

### Example 2: In vitro colorimetric assay (spectrophotometric assay) for identification of detyrosinase (TCPase) modulators compounds

The assay relies on the well-document tyrosinase activity on free tyrosine. Free tyrosine can be converted by tyrosinase into a colorimetric detectable compound. This reaction has been described for almost a century (Lichtman, JBC 1929) and applied to measure free tyrosine in various samples.

Tyrosinase catalyzes tyrosine to DOPO and DOPA to DOPAquinone subsequently. During DOPAquinone formation, protons (H+) are produced and these protons could be monitored by substrate (ex: thymol blue) color change (Park et al., 2003).

Current method relies on the use of VASH2 and appropriate substrate of TCPase as disclosed in the above Material & Methods to release free tyrosine in the reaction mixture that can in a second step be used for tyrosinase-dependent analysis and spectrophotometric quantification.

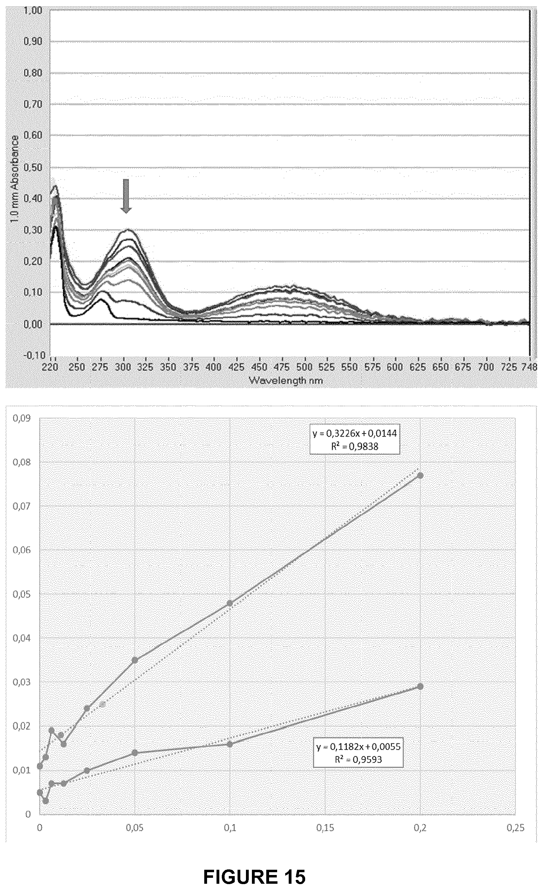

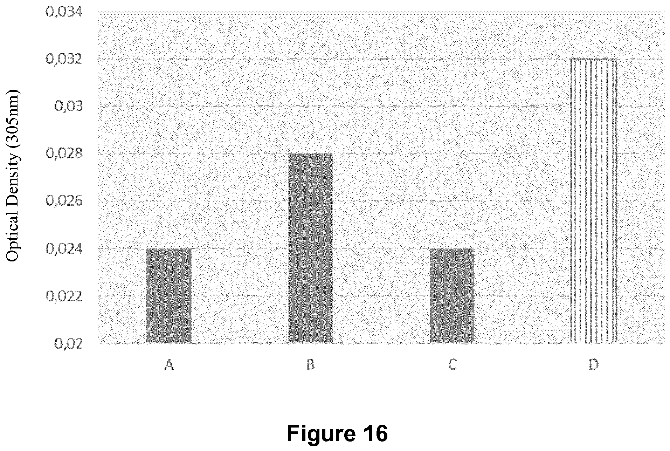

A reaction mixture was prepared by pipetting a phosphate buffer, deionized water, VASH2 recombinant protein,TCPase substrate such as MTs or human alpha tubulin and tyrosinase. The TCPase activity is directly correlated to the increase in absorption due to tyrosinase-mediated generation of dopaquinone. The absorption may be measured with a SPECTRAmax PLUS spectrophotometer. The released C-terminal tyrosine residue by VASH2 becomes substrate of tyrosinase that catalyzes a colorimetric compound that can be measured using a spectrophotometer. We used recombinant tyrosinase (Sigma Aldrich) to determine the reaction kinetics by measuring optical density at 305nm (**Figure 15**). Increasing amounts of free tyrosine were mixed with recombinant tyrosinase in a phosphate buffer pH=7.0, incubated at room temperature for 15min and analyzed by spectrophotometer at 305nm (**Figure 15**). Next, the proof of concept was performed using recombinant VASH2. Recombinant VASH2 was mixed with bacterially produced recombinant human alpha tubulin , in particular recombinant tubulin alpha-1A chain comprising the amino acid sequence SEQ ID NO: 19 (Uniprot Q71U36) and incubated for 15 min at room temperature in a phosphate buffer ph=7.0, 100mM NaCl. Next, tyrosinase enzyme was added, mixed and incubated at room temperature for 30min and analyzed using a spectrophotometer at 305nm (**Figure 16**).

All controls containing the individual components of the reaction were measured (samples A-C) as well.
A. control sample containing tyrosinase,
B. control sample containing recombinant human alpha tubulin,
C. control samples containing tyrosinase (Tyr) and VASH2 as detyrosinase,
D reaction mixture containing recombinant human alpha tubulin, VASH2 mediating tyrosine release and tyrosinase to allow detection of free tyrosine.
OD305 was measured 30 min after onset of reaction at room temperature.

Release of tyrosine by VASH2 could be observed (Sample D - **Figure 16**.).

### Example 3: Dot-blot assay for identification of detyrosinase (TCPase) modulators compounds

In this technique, 2µl of the reaction mixture containing the substrate (8 amino acid peptide consisting of GEEEGEEY) and the enzyme (Recombinant human VASH2) in absence or in presence of the compound were doted on nitrocellulose and assessed for detyrosination of the peptide using a specific antibody detecting detyrosination.

The filter is allowed to dry and the membrane is incubated in blocking buffer to prevent non-specific binding. Similarly to ELISA, HRP-coupled antibody directed against the detyrosinated Telokin engineered substrate (as disclosed above) is incubated over the membrane, excess is washed-off and the membrane is developed using e.g. chromogenic HRP substrate. Intensities of the signals on autoradiograms were quantified by densitometric scanning. Alternatively, we designed a method using a slot blot approach. The steps are identical as for the dot blot approach, this is the samples are applied to a nitrocellulose or PVDF membrane with a vacuum manifold to produce an orderly grid of samples. Once dry, dot blots and slot blots are subjected to the same immunodetection steps used for Western blotting (**Figure 11**).

### REFERENCES:

Aillaud, C., Bosc, C., Peris, L., Bosson, A., Heemeryck, P., Van Dijk, J., Le Friec, J., Boulan, B., Vossier, F., Sanman, L.E., et al. (2017). Vasohibins/SVBP are tubulin carboxypeptidases (TCPs) that regulate neuron differentiation. Science 358, 1448-1453 Ciferri, C., Pasqualato, S., Screpanti, E., Varetti, G., Santaguida, S., Dos Reis, G., Maiolica, A., Polka, J., De Luca, J.G., De Wulf, P., et al. (2008). Implications for kinetochore-microtubule attachment from the structure of an engineered Ndc80 complex. Cell 133, 427-439.

Gordon, Malcolm and Mitchell, Herschel K. (1949) A fluorometric method for the estimation of tryptophan. Journal of Biological Chemistry, 180 (3). pp. 1065-1070. ISSN 0021-9258.

Inglese J, et al. Nat Chem Biol. 2007 Aug;3(8):466-79. Review

Kato et al., 2004 (Int J Cancer), Nov 10;112(3):365-75. Low expression of human tubulin tyrosine ligase and suppressed tubulin tyrosination/detyrosination cycle are associated with impaired neuronal differentiation in neuroblastomas with poor prognosis.

Lichtman, SS, Sobotka H. J.Biol.Chem 1929, 85:261-273

Magiera MM, Singh P, Gadadhar S, Janke C. Cell. 2018 May 31;173(6):1323-1327. doi: 10.1016/j.cell.2018.05.018. Epub 2018 May 31. Review. Tubulin Posttranslational Modifications and Emerging Links to Human Disease.

Barisic M, Silva e Sousa R, Tripathy SK, Magiera MM, Zaytsev AV, Pereira AL, Janke C, Grishchuk EL, Maiato H. Science. 2015 May 15;348(6236):799-803. Mialhe et al., 2001 (Cancer Res), 2001 Jul 1;61(13):5024-7. Tubulin detyrosination is a frequent occurrence in breast cancers of poor prognosis.

Nieuwenhuis, J., Adamopoulos, A., Bleijerveld, O.B., Mazouzi, A., Stickel, E., Celie, P., Altelaar, M., Knipscheer, P., Perrakis, A., Blomen, V.A., et al. (2017). Vasohibins encode tubulin detyrosinating activity. Science 358, 1453-1456.

Park et al. 2003, Journal of Protein Chemistry, Vol.22, N°5, 473-480. A new continuous spectrophotometric assay method for DOPA oxidase activity of tyrosinase.

Preston, S.F., Deanin, G.G., Hanson, R.K., and Gordon, M.W. (1979). The phylogenetic distribution of tubulin:tyrosine ligase. J Mol Evol 13, 233-244.

Roll-Mecak, A., and Vale, R.D. (2008). Structural basis of microtubule severing by the hereditary spastic paraplegia protein spastin. Nature 451, 363-367.

Widlund, P.O., Podolski, M., Reber, S., Alper, J., Storch, M., Hyman, A.A., Howard, J., and Drechsel, D.N. (2012). One-step purification of assembly-competent tubulin from diverse eukaryotic sources. Mol Biol Cell 23, 4393-4401.

## Claims

1. An *in vitro* screening assay for identification of modulators of tubulin carboxypeptidase (TCPase) activity comprising the steps of:
(i) Contacting (a) a substrate of TCPase enzyme comprising an amino acids sequence having at least the last 4 amino acids residues of the C-terminal sequence of an α-tubulin and/or a Microtubule Associated Protein (MAP) and, as ultimate C-terminal amino acid residue, an aromatic amino acid residue, preferably a tyrosine (Y), and b) an isolated or recombinant TCPase enzyme; in the presence or absence (control) of the modulator compound to be tested, and under conditions for substrate cleavage, preferably detyrosination, and/or liberation of a C-terminal free aromatic amino acid residue, preferably a C-terminal free tyrosine;
(ii) Using reagents for detecting and measuring the signal related to substrate cleavage, preferably detyrosination, and/or liberation of the C-terminal free aromatic amino acid residue, preferably the C-terminal free tyrosine ;
(iii) Measuring and comparing the level of substrate cleavage, preferably detyrosination and/or the level of the C-terminal free aromatic amino acid residue, preferably the C-terminal free tyrosine, in presence and in absence (control) of the compound to be tested, and
(iv) Selecting the modulators of TCPase for which the level of substrate cleavage, preferably detyrosination or liberation of the C-terminal free aromatic amino acid residue, preferably the C-terminal free tyrosine, is increased in the presence of the compound to be tested (activators of TCPase) or decreased in the presence of the compound to be tested (inhibitors of TCPase).

2. The *in vitro* screening assay of claim 1, wherein the substrate of TCPase enzyme comprising an amino acid sequence having at least the last 4 amino acids residues of the C-terminal sequence of an α-tubulin and/or a Microtubule Associated Protein (MAP) and, as ultimate C-terminal amino acid residue, an aromatic amino acid residue, preferably a tyrosine (Y) is selected from the group consisting of:
a) Isolated or recombinant microtubules,
b) Isolated or recombinant tubulin dimers of alpha and beta tubulin,
c) Microtubule-associated proteins (MAP) such as microtubule-associated proteins RP/EB family members 1 to 3, in particular comprising an amino acid sequence selected from SEQ ID NO: 30 to SEQ ID NO: 32 *(Homo sapiens)* or variants thereof,
d) Purified or recombinant α-tubulin, in particular comprising an amino acid sequence selected from SEQ ID NO: 19 to SEQ ID NO: 24 (Homo sapiens), or SEQ ID NO: 25 and SEQ ID NO: 26 *(Trypanosoma brucei*), or SEQ ID NO: 27 (*Toxoplasma gondii*), or SEQ ID NO: 28 (*Spodoptera frugiperda*) or SEQ ID NO: 29 (*Drosophila melanogaster*) or variants thereof,
e) Recombinant engineered telokin, in particular comprising an amino acid sequence SEQ ID NO: 17 or variant thereof,
f) Isolated or recombinant peptide from 4 to 50 amino acid residues, in particular 5 to 20 amino acids, preferably 6 to 12 amino acid residues comprising an amino acid sequence having at least the last 4 amino acids residues of the C-terminal sequence of an α-tubulin and/or a Microtubule Associated Protein (MAP) and, as ultimate C-terminal amino acid residue, an aromatic amino acid residue, preferably a tyrosine (Y), in particular comprising an amino acid sequence selected from SEQ ID NO: 33 to SEQ ID NO: 39 or variant thereof; or
g) Mixtures thereof;

3. The *in vitro* screening assay according to claim 1 or claim 2, wherein the substrate of TCPase enzyme is selected from recombinant engineered telokin having at least the last 4 amino acids residues of the C-terminal sequence of an α-tubulin and a tyrosine (Y) as ultimate C-terminal amino acid residue, in particular comprising an amino acid sequence SEQ ID NO: 17 or variant thereof.

4. The *in vitro* screening assay according to anyone of claims 1 to 3, wherein the isolated or recombinant TCPase enzyme is selected from proteins having at least 30%, 40%, 50%, 60%, 70%, 80%, 90%, 95%, 99%, amino acid sequence identity with the amino acid sequence selected from SEQ ID NO: 1 (Ubiquitin carboxyl-terminal hydrolase 14), SEQ ID NO: 2 (Ubiquitin carboxyl-terminal hydrolase 5), SEQ ID NO: 3 (Methionine aminopeptidase 2), SEQ ID NO: 4 (Xaa-Pro aminopeptidase 1), SEQ ID NO: 5 (Tripeptidyl-peptidase 2), SEQ ID NO: 6 (Vasohibin-1), SEQ ID NO: 7 (Dihydropyrimidinase-related protein 1, CRMP1), SEQ ID NO: 8 (Dihydropyrimidinase-related protein 2), SEQ ID NO:9 (Dihydropyrimidinase-related protein 3), SEQ ID NO: 10 (Dihydropyrimidinase-related protein 4), SEQ ID NO: 11 (Dihydropyrimidinase-related protein 5), SEQ ID NO: 12 (Vasohibin-2), or SEQ ID NO: 14 (Uncharacterized protein / VASH of *Trypanosoma brucei*), preferably Vasohibin-1 (SEQ ID NO: 6) or Vasohibin-2 (SEQ ID NO: 12) in presence or absence of Small vasohibin-binding protein (SEQ ID NO: 13).

5. The *in vitro* screening assay according to claim 4, wherein a) the substrate of TCPase enzyme is selected from purified or recombinant α-tubulin from parasites, in particular comprising an amino acid sequence selected from SEQ ID NO: 25 and SEQ ID NO: 26 *(Trypanosoma brucei*) or variants thereof, and b) the isolated or recombinant TCPase enzyme is selected from parasites proteins, in particular having at least 30%, 40%, 50%, 60%, 70%, 80%, 90%, 95%, 99%, amino acid sequence identity with the amino acid sequence SEQ ID NO: 14 (Uncharacterized protein / VASH of *Trypanosoma brucei*).

6. The *in vitro* screening assay according to claim 4, wherein a) the substrate of TCPase enzyme is a recombinant engineered telokin, in particular comprising an amino acid sequence SEQ ID NO: 17 or variant thereof, and b) the isolated or recombinant TCPase enzyme is selected from proteins having at least 30%, 40%, 50%, 60%, 70%, 80%, 90%, 95%, 99%, amino acid sequence identity with the amino acid sequence Vasohibin-1 (SEQ ID NO: 6) or Vasohibin-2 (SEQ ID NO: 12) in presence or absence of Small vasohibin-binding protein (SEQ ID NO: 13).

7. The *in vitro* screening assay according to anyone of claims 1 to 6, wherein step (ii) comprises detection of the C-terminal free aromatic amino-acid via a colorimetric assay or using fluorescent properties of the aromatic residues, preferably detection of the C-terminal free-tyrosine via a colorimetric assay using a tyrosinase.

8. The *in vitro* screening assay according to anyone of claims 1 to 6, wherein the assay is an immuno-assay, in which the step (ii) comprises :
- adding an effective amount of specific labelled antibody raised against cleaved substrate, in particular detyrosinated alpha-tubulin (dTyr-Ab), under conditions that favor the formation of a complex antibody - cleaved substrate; and
- means for revealing the labelled signal;
or alternatively
- adding an effective amount of a primary specific antibody raised against cleaved substrate, in particular detyrosinated alpha-tubulin (dTyr-Ab), under conditions that favor the formation of a complex antibody - cleaved substrate;
- adding an effective amount of a secondary labelled antibody specific of the primary antibody, under conditions that favor the formation of a complex primary antibody- cleaved substrate- labelled secondary antibody, and
- means for revealing the labelled signal,
and wherein the reaction occurs in soluble (fluid) phase or solid phase.

9. The *in vitro* screening assay according to claim 8, wherein the antibody is labelled with a marker selected in the group consisting of an enzyme, a fluorescent compound or fluorophore, a (chemo)luminescent compound or a radioactive element, preferably an enzyme and more preferably a peroxidase.

10. The *in vitro* screening assay according to claim 8 or claim 9, wherein said immunoassay is an enzyme immunoassay, a fluoroimmunoassay, a luminescent immunoassay or a radioimmunoassay, preferably a dot-blot or an enzyme immunoassay (ELISA).

11. The *in vitro* screening immunoassay according to claims 8 to 10 wherein the reaction occurs in solid phase, and wherein:
- substrate of TCPase is coated on solid support, in particular on membrane or microplate, and the TCPase enzyme, the compound(s) to be tested and the antibodies raised against cleaved substrate, in particular detyrosinated α-tubulin (dTyr-Ab), optionally labelled or combined with labelled secondary antibodies raised against primary antibodies are added in the reaction solution, or
- alternatively, the antibodies raised against cleaved substrate, in particular detyrosinated α-tubulin (dTyr-Ab), are coated on solid support, in particular on membrane or microplate, and TCPase enzyme, substrate of TCPase, the compound(s) to be tested, and secondary labelled antibodies raised against cleaved substrate, are added in the reaction solution, or
- alternatively, the TCPase enzyme is coated on solid support, in particular on membrane or microplate, and the substrate of TCPase, the compound(s) to be tested, and the antibodies raised against cleaved substrate, in particular detyrosinated α-tubulin (dTyr-Ab), optionally labelled or combined with labelled secondary antibodies raised against primary antibodies, are added in the reaction solution.

12. The *in vitro* screening immuno-assay according to anyone of claims 8 to 11, wherein (a) the substrate of TCPase comprises dimers of alpha-tubulin extracted from SF9 cells (*Spodoptera frugiperda* origin), in particular comprising the amino acid sequence SEQ ID NO:27 or a variant thereof, (b) the TCPase enzyme is vasohibin-2 (VASH2) in the presence or absence of SVBP, and the step (ii) of detecting and measuring the level of detyrosinated-substrate comprises at least an antibody raised against detyrosinated α-tubulin SF9 (dTyr-Ab SF9) coupled to horseradish peroxidase (HRP).

13. The *in vitro* screening assay according to anyone of claims 1 to 12, which is a High Throughput Screening (HTS) assay, in particular a High Throughput Screening (HTS) assay based on ELISA assay.

14. A kit for performing the *in vitro* screening immuno-assay of anyone of claims 1-6 and 8-13, comprising :
(i) a TCPase enzyme substrate, preferably a recombinant engineered telokin as substrate of TCPase enzyme, in particular comprising an amino acid sequence SEQ ID NO: 17 or variant thereof,
(ii) a TCPase enzyme, preferably an isolated or recombinant TCPase enzyme selected from proteins having at least 30%, 40%, 50%, 60%, 70%, 80%, 90%, 95%, 99%, amino acid sequence identity with the amino acid sequence of Vasohibin-2 (SEQ ID NO: 12) in presence or absence of Small vasohibin-binding protein (SEQ ID NO: 13),
(iii) antibodies raised against cleaved substrate, preferably detyrosinated α-tubulin (dTyr-Ab), in particular raised against detyrosinated α-tubulin SF9 (dTyr-Ab SF9) and coupled to horseradish peroxidase (HRP), and optionally secondary antibodies raised against the cleaved substrate antibodies, preferably the detyrosinated α-tubulin antibodies,
(iv) a negative control, in particular comprising a non-tyrosinated isolated or recombinant telokin,
(v) a positive control, in particular comprising Epoxy-Y compound,
(vi) a fluid vessel or alternatively a solid support for coating or pre-coating either the substrate of TCPase either the TCPase enzyme, preferably microplates,
(vii) reagents for allowing contact of said substrate with TCPase enzyme in reaction conditions for substrate cleavage, preferably detyrosination,
(viii) reagents for detecting and measuring the level of substrate cleavage, preferably detyrosination ; and
(ix) optionally a notice of use.

15. A kit for performing the *in vitro* screening assay of claim 7 for detection of the free C-terminal aromatic amino-acid via a colorimetric assay or using fluorescent properties of the aromatic residues, comprising :
(i) a TCPase enzyme substrate, preferably a recombinant engineered telokin as substrate of TCPase enzyme, in particular comprising an amino acid sequence SEQ ID NO: 17 or variant thereof,
(ii) a TCPase enzyme, preferably an isolated or recombinant TCPase enzyme selected from proteins having at least 30%, 40%, 50%, 60%, 70%, 80%, 90%, 95%, 99%, amino acid sequence identity with the amino acid sequence of Vasohibin-2 (SEQ ID NO: 12) in presence or absence of Small vasohibin-binding protein (SEQ ID NO: 13),
(viii) optionally a tyrosinase for the detection of a C-terminal free-tyrosine via colorimetric assay,
(ix) a fluid vessel or alternatively a solid support for coating or pre-coating either the substrate of TCPase either the TCPase enzyme, preferably membranes or microplates,
(x) reagents for allowing contact of said substrate with TCPase enzyme in reaction conditions for liberation of a C-terminal free-aromatic amino acid residue, preferably a C-terminal free-tyrosine,
(xi) reagents for detecting and measuring the level of the C-terminal free-aromatic amino acid residue, preferably the C-terminal free-tyrosine,; and
(xii) optionally a notice of use.
